(19) Europäisches Patentamt
European Patent Office
Office européen des brevets

(11) EP 0 736 030 B1

(12) **EUROPEAN PATENT SPECIFICATION**

(45) Date of publication and mention of the grant of the patent:
**29.08.2001 Bulletin 2001/35**

(21) Application number: **95906620.0**

(22) Date of filing: **20.12.1994**

(51) Int Cl.[7]: **C07D 521/00**, C07F 9/6558, A61K 31/495, A61K 31/66

(86) International application number:
**PCT/US94/14236**

(87) International publication number:
**WO 95/17407 (29.06.1995 Gazette 1995/27)**

(54) **TETRAHYDROFURAN ANTIFUNGALS**
FUNGIZIDE TETRAHYDROFURANE
TETRAHYDROFURANES ANTIFONGIQUES

(84) Designated Contracting States:
**AT BE CH DE DK ES FR GB GR IE IT LI LU NL PT SE**

(30) Priority: **21.12.1993 US 171083**

(43) Date of publication of application:
**09.10.1996 Bulletin 1996/41**

(73) Proprietor: **SCHERING CORPORATION**
**Kenilworth New Jersey 07033 (US)**

(72) Inventors:
- **SAKSENA, Anil, K.**
  **Upper Montclair, NJ 07043 (US)**
- **GIRIJAVALLABHAN, Viyyoor, M.**
  **Parsippany, NJ 07054 (US)**
- **LOVEY, Raymond, G.**
  **West Caldwell, NJ 07006 (US)**
- **PIKE, Russell, E.**
  **Stanhope, NJ 07874 (US)**
- **WANG, Haiyan**
  **Dayton, NJ 08810 (US)**
- **LIU, Yi-Tsung**
  **Morris Township, NJ 07960 (US)**
- **GANGULY, Ashit, K.**
  **Upper Montclair, NJ 07043 (US)**
- **BENNETT, Frank**
  **Piscataway, NJ 08854 (US)**

(74) Representative: **Ritter, Stephen David et al**
**Mathys & Squire**
**100 Gray's Inn Road**
**London WC1X 8AL (GB)**

(56) References cited:
**EP-A- 0 539 938**
**WO-A-89/04829**
**US-A- 5 039 676**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 736 030 B1

## Description

This invention relates to tetrahydrofuran antifungals, (2R-cis)-4-[4-[4-[4-[[(5-(2,4-dihalophenyl)-tetrahydro-5-(1H-1,2,4-triazol-1-ylmethyl)-tetrahydrofuran-3-yl]methoxy]phenyl]-2,4-dihydro-2-[mono- or dihydroxy-substituted ($C_3$-$C_8$) alkyl]-3H-1,2,4-triazol-3-one substituted antifungals, pharmaceutically acceptable esters and salts thereof, pharmaceutical compositions containing them, useful for treating and/or preventing antifungal infections in hosts, including warm-blooded animals, especially humans with such tetrahydrofuran antifungals.

International Publication Number WO 89/04829, published 1 June 1990 and USP 5,039,676 (A.K. Saksena et al.) discloses (±) cis and (±) (trans antifungal compounds represented by the formula

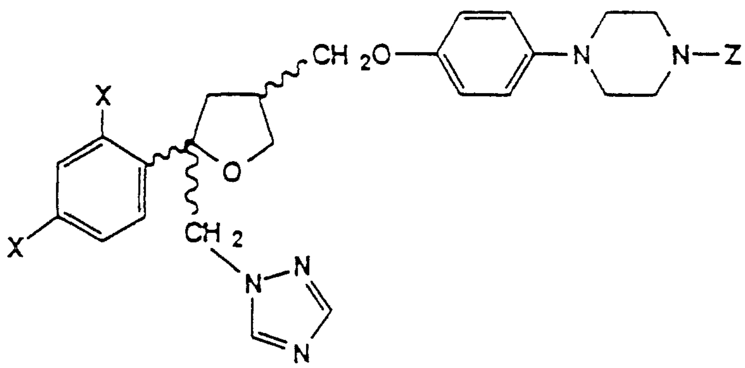

wherein X= F, Cl; Z=loweralkyl, (C2-C8) alkanoyl or phenyl substituted by 2-loweralkyl-3-oxo-1,2,4-triazol-4-yl, e.g., (±)-cis and (±)-trans-1-[4-[[2-(2,4-difluorophenyl)-2-[(1H-1,2,4-triazol-1-yl)methyl]tetrahydro-4-furanyl]methoxy] phenyl]-4-(1-methylethyl)piperazine. However, WO 89/04829 does not disclose the compounds of this invention.

Commonly-owned European Patent Publication No. 05399381, published 5 May 1993 discloses, for example, [(5R)-cis-4-[4-[4-[4-[[5-(2,4-dihalophenyl)-5-(1H-1,2,4-triazol-1-ylmethyl)tetrahydrofuran-3-7yl]methoxy]phenyl]-1-piperazinyl]pheynyl)-2,4-dihydro-2-($C_1$-$C_{10}$)alkyl)]-3H-1,2,4-triazol-3-one antifungals but does not disclose the compounds of this invention.

Janssen U. S. Patent 4,791,111 discloses, for example, (+)cis-4-[4-[4-[4-[[2-2,4-dichlorophenyl)-2-(1H-1,2,4-triazol-1-ylmethyl)-1,3-dioxolan-4-yl]methoxy]phenyl]-1-piperazinyl]-2,4dihydro-2-(2-hydroxy-1-methylpropyl)-3H-1,2,4-triazol-3-one useful as an antimicrobial agent and having increased solubility, but does not disclose the compounds of this invention.

There is a need for broad-spectrum antifungal agents having increased solubility and having favorable activity profile for treating systemic fungal infections, especially Aspergillus, Candida, Cyrptococcus and opportunistic infections.

## SUMMARY OF INVENTION

The present invention provides compounds represented by formula I

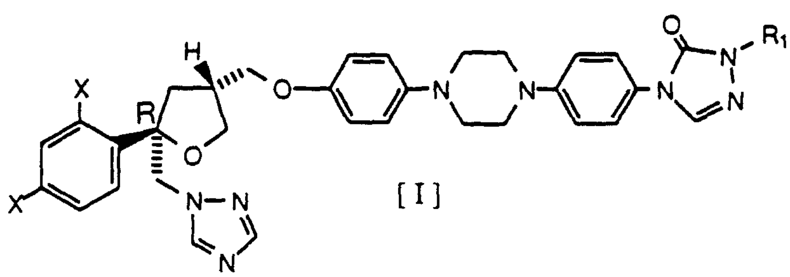

[ I ]

wherein

X is independently both F or both Cl or one X is independently F and the other is independently Cl;
$R_1$ is a straight or branched chain ($C_3$ to $C_8$) alkyl group substituted by one or two hydroxy moieties or stereoisomers

thereof or a pharmaceutically acceptable ester thereof, or a pharmaceutically acceptable salt thereof.

In a preferred aspect of the present invention, there is provided compounds represented by formula II

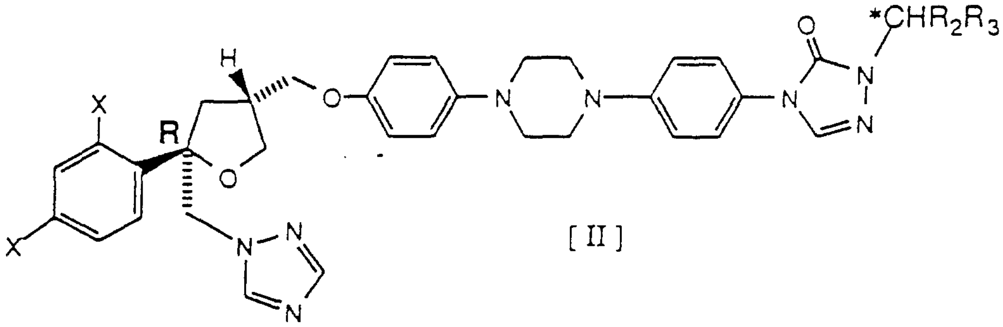

[ II ]

wherein X is independently both F or both Cl or one X is independently F and the other is independently Cl; wherein $R_2$ is H or $(C_1\text{-}C_3)$ alkyl and $R_3$ is $(C_1\text{-}C_3)$ alkyl substituted by one hydroxy moiety and the carbon with the asterisk (*) has the R or S absolute configuration; an ester thereof or a pharmaceutically acceptable salt thereof.

In another preferred aspect, the present invention provides a compound represented by formula III

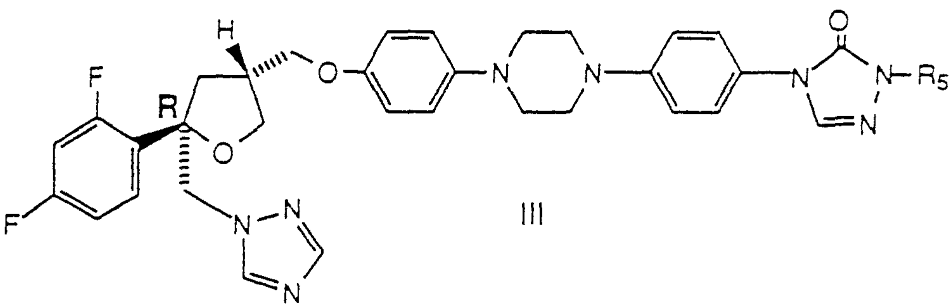

III

wherein $R_5$ is

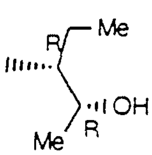 , 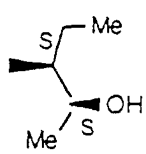 , 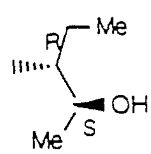 ,

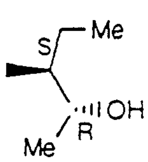 , 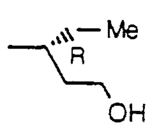 , 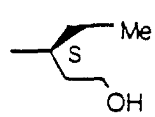 ,

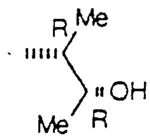 , 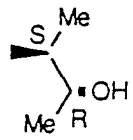 , 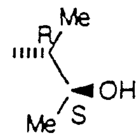 ,

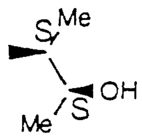 , 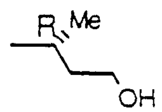 , 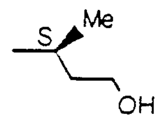 ,

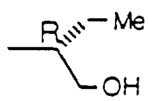 or 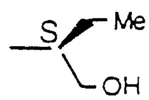

an ester thereof or a pharmaceutically acceptable salt thereof.

In another aspect of the present invention there is provided a compound represent by the formula IV

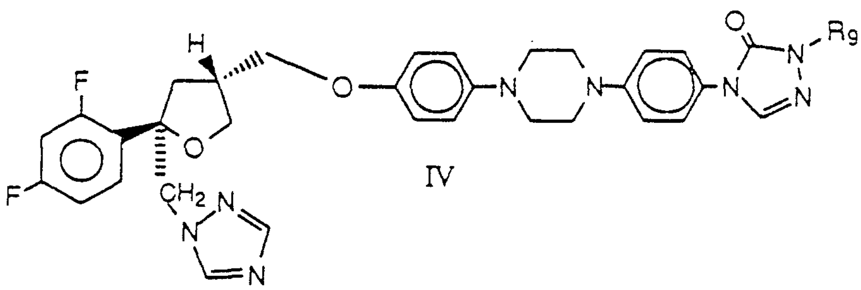

IV

wherein $R_9$ =

$$-\overset{*}{C}H(C_2H_5)CH(OR_6)CH_3 \text{ or } -\overset{*}{C}H(CH_3)CH(OR_6)CH_3$$

or wherein $R_6$ is H, a polyether ester, a phosphate ester; a sulfate ester; a heterocyclic ester; an alkanoate ester; an alkenoate ester; an amino acid ester; an acid ester or a pharmaceutically acceptable salt thereof.

DETAILED DESCRIPTION OF THE INVENTION AND OF THE PREFERRED EMBODIMENTS

The term "($C_3$-$C_8$) alkyl group substituted by one or two hydroxy moieties", as used herein means straight and branched chain alkyl groups of three to eight carbons including but not limited to methyl, ethyl, n- and isopropyl, n-, sec-, iso- and tert-butyl, n-, sec-, iso-, tert and neo-pentyl n-, sec-, iso-, tert- and neo-hexyl, n-, sec-, iso-, tert- and neo-heptyl, n, sec- iso, tert-and neo-octyl, substituted by one or two hydroxy moieties and includes R and S stereoisomers of such ($C_3$-$C_8$) alkyl groups.

The term "($C_1$-$C_3$) alkyl substituted by one hydroxy moiety" means

$$-CH_2OH,\ -\overset{*}{C}H(OH)CH_3,\ -CH_2CH_2OH,\ -\overset{*}{C}H(OH)C_2H_5,$$

$$-\overset{*}{C}H_2CH(OH)CH_3\ ,$$

and $-(CH_2)_3$-OH wherein the carbons with the asterisk(*) have the R or S absolute configuration.

The term "hydroxy-substituted $C_4$ or $C_5$ alkyl group" means

$$-\overset{*}{C}H(C_2H_5)\overset{*}{C}H(OH)CH_3\ ,\ -\overset{*}{C}H(C_2H_5)CH_2CH_2OH\ ,\ -(CH_2)_2-\overset{*}{C}H(OH)C_2H_5\ ,$$
$$-\overset{*}{C}H(CH_3)\overset{*}{C}H(OH)CH_3\ ,\ -\overset{*}{C}H(CH_3)\overset{*}{C}H(OH)CH_3 \text{ or } -\overset{*}{C}H(C_2H_5)CH_2OH$$

wherein each carbon with the asterisk (*) has the R or S absolute configuration.

The term "esters" means (a) polyether esters (b) phosphate esters (c) heterocyclic esters (d) alkanoate and alkenoate esters (e) amino-alkanoates and (f) acid esters and (g) sulfate esters.

The term "polyether esters" as used herein means those polyether esters represented by the formula

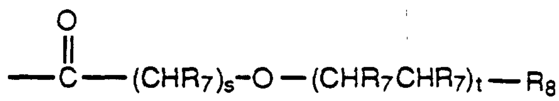

wherein

$R_7$ is as defined herein and s is an integer from 1 to 6, preferably s = 1 to 3 and more preferably s = 1; t is an integer from 1 to 6; preferably t is 1 to 3, more preferably t is 2 or 3.

$R^8$ is $R_7$ or $-(CHR_7)_s-CO_2R_7$; preferably $R_8$ is $CH_3$ or $C_2H_5$ or $-CH_2CO_2H$ or $-CH_2CO_2CH_3$ Typically suitable polyether esters include $-COCH_2O(CH_2CH_2O)_1CH_3$; $-COCH_2O(CH_2CH_2O)CH_3$ and, $-COCH_2O(CH_2CH_2O)_3CH_3$.

The term 'phosphate esters" as used herein means those phosphate acids esters represented by the formula

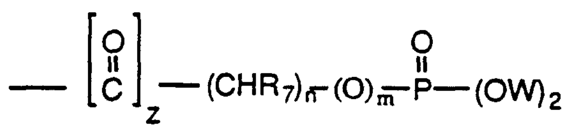

wherein z is 0 or 1; $R_7$ is as defined herein above and preferably is H; n is an integer from 0 to 6, m is 0 or 1 and W is H, $CH_2Ar$ or

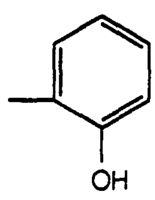

and wherein Ar is as defined herein above. Typically suitable phosphate acids and esters include

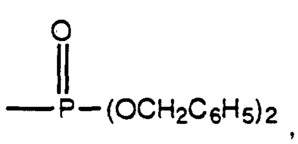

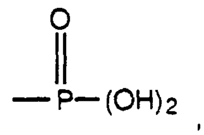,

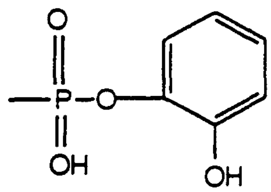 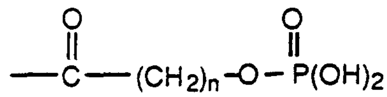

wherein m= n = 1 to 4; or

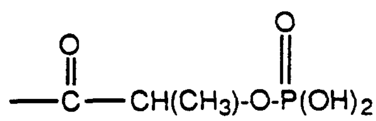

and pharmaceutically acceptable salts thereof.

The term "heterocyclic ester" as used herein means heterocyclic esters represented by the formula

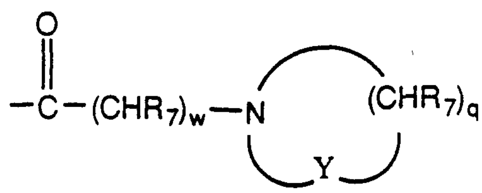

wherein $R_7$ is as defined herein above, W is an integer of from 1 to 5 preferably W is 1 to 3; q is = 3 or 4 and Y is $CHR_7$, -O-, NH, $NR_7$, S, SO or $SO_2$

Typically suitable heterocyclic esters include

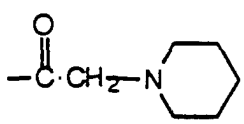, 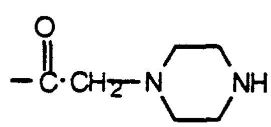 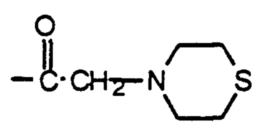, 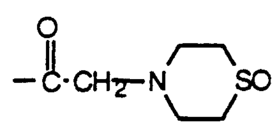

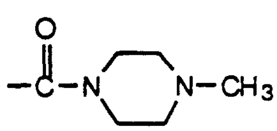, 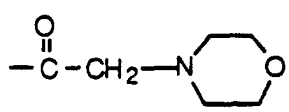, 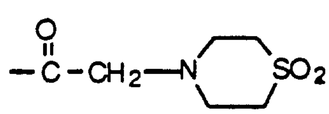

and 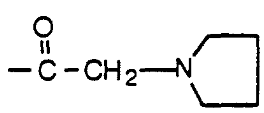,

The term "alkanoate and alkenoate esters" as used herein means straight or branched chain alkanoate or alkenoate groups optionally substituted by a hydroxy or ether moiety or mixtures of such alkanoates or alkenoates.

Preferred alkanoate esters include acetate to decanoate, especially acetate to butanoate. Preferred hydroxy substituted alkanoate ester include $C_1$ to $C_8$ alkanoate substituted one hydroxy moiety, especially

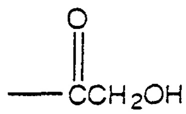 and 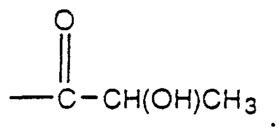

Preferred alkenoate esters are the $C_{10}$-$C_{20}$ alkenoates and include $C_{14}$ to $C_{18}$ alkenoates, such as cis-7-hexadecenoate.

The term "amino alkanoate" as used herein include the natural and unnatural amino acid residues preferably with amino groups protected by the conventional protecting groups well known to those in art such as phenyl acetate.

The term "acid ester" as used herein means those acid esters represented by the formula

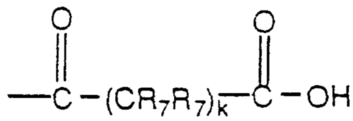

wherein $R_7$ is as defined herein above and k is an integer of from 1 to 8. Typically suitable acid esters include oxalic, malonic, succinic glutaric and adipic acids as well as branched chain diacids such as

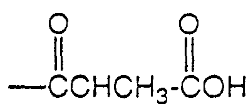

The compounds of the present invention as well as the esters thereof exhibit broad spectrum antifungal activity in various in vitro assays against Candida? other yeasts, dematophytes, Aspergillus and opportunistic fungi. The in vitro antifungal activity test were performed via conventional agar dilution methods in Sabouraud dextrose broth ("SDB") medium against a large number of fungi. Minimum Inhibitory Concentrations ("MICs") were measured after 24, 48 and 72 hour tests.

The term "opportunistic fungi" include Crytococcus, Histoplasma, Blastomyces, Coccidioides, Fusarium, Mucor, Paracoccidioides, Fonsecaea, Wangiella, Sporothrix, Pneumocystis, Tichosporon as shown by in vivo activity in an appropriate animal species e.g. mouse, rat or rabbit. The compounds of the inventions are expected to exhibit activity against many genera and species of bacteria, protozoa, gram negatives, gram positives, Anaerobes, Legionella Borrelia, Mycoplasma, Treponema, Gardneralla, Trichomononas and Trypanosoma

The preferred compounds of formula III wherein $R_5$=hydroxy-substituted $C_4$ and $C_5$ alkyl groups, exhibited the following in vitro antifungal activity in SDB against 37 species of Aspergillus niger, flavus, fumigatus and terreus: geometric mean MICs in the range of ≤0.05 to ≥1.53 (mcg/ml) and geometric mean MFCs in the range of 0.27 to ≥4.24 mcg/ml.

The preferred compounds of formula III wherein $R_5$ is a hydroxy-substituted $C_5$ alkyl group exhibited (1) superior antifungal activity as measured by geometric mean MICs and MFCs in various in vitro assays against C. albicans (N=26), C. krusei (N=26), C. glabrata (N=9), C. tropicalis (N=4), C. stellatoidea (N=1), C. neoformans (N=3), and the dermatophytes, T. rubrum, T. menta, and T. tonsurans (N=6) (after 48 or 78 hours) compared to fluconazole as well as (2) superior anti-fungal activity in the following in vivo models: an Aspergillus flavus and fumigatus (four strains) in a pulmonary immuno-compromised mouse model (PO-1XDX4D) compared to other azoles *e.g.* itraconazole, and in a Candida albicans (four species) systemic model with normal and compromised mice (PO-1XDX4D) compared to other azoles, *e.g.* fluconazole.

The in vivo oral antifungal activity of the compounds of the present invention were compared to azole antifungals, e.g., fluconazole in an Aspergillus pulmonary infection model in mice. The procedure of David Loebenberg *et al.* entitled "Sch 42427, The Active Enantiomer of Antifungal agent Sch 39304; *In vitro* Activity", Antimicrobial Agents and Chemotherapy, (1992), 36 498-501 was used. The Aspergillus flavus pulmonary model is also described in European Patent Application No. 0 539,938 Al published on 5 May 1993.

The preferred compounds of formula III exhibited superior antifungal in vitro activity in SDB against 37 species of Aspergillus with (a) geometric mean MICs of ≤ 0.05 to ≤ 0.81 compared to fluconazole (geometric mean MIC ≥ 32 and (b) with geometric mean MFCs of ≤ 0.89 to ≤ 3.78 compared to fluconazole (geometric mean MFC ≥ 32).

The Tables Q, R, and S hereinbelow display the superior in vitro antifungal activity of three preferred compounds of formula III compared to fluconazole. Table Q displays such antifungal acitivity such as the percentage of strains of various fungi with MICs ≤ 1 mcg/ml for the three preferred compounds of formula III compared to fluconazole. Table R displays the antifungal activity as the percentage of the same strains with MFCs ≤ 1 mcg/ml. Table S displays the in vitro MIC 90 values for the three preferred compounds of formula III agains the same organisms listed in Tables Q and R.

The most preferred compound of formula III where $R_5$ =

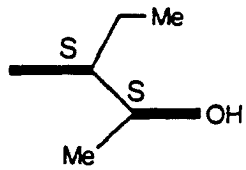

showed consistently higher serum levels in mice, rats, dogs and monkeys following oral dosing with a methyl cellulose formulation compared to azoles of similiar structure and also exhibited very long serum half life following O.D. dosing with good tissue distribution. The above listed most preferred compound of formula III are not inducers of various cytochrome P-450 liver drug metabolizing enzymes after oral administration in an in vivo rat model

TABLE Q

IN VITRO ANTIFUNGAL ACTIVITY FOR SELECTED COMPOUNDS OF FORMULA III[1]

PERCENTAGE OF STRAINS WITH MICs ≤1 MCG/ML

| | | (MCG/ML) | | | |
|---|---|---|---|---|---|
| | $R^5$ | 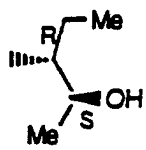 | 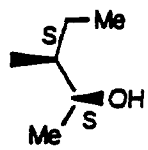 | 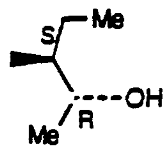 | |
| ORGANISM | STRAINS | | | | FLZ[2] |
| Aspergillus | 37 | 100 | 100 | 100 | 0 |
| Candida Albicans | 26 | 100 | 100 | 100 | 100 |
| Candida Kursei | 16 | 100 | 100 | 100 | 100 |
| Candida Tropicalis & Stellatoidea | 5 | 100 | 100 | 100 | 100 |
| Candida Glabrata | 9 | 22 | 22 | 33 | 0 |
| Cryptococcus Neoformans | 3 | 100 | 100 | 100 | 0 |
| Dermatophytes | 6 | 100 | 100 | 100 | 100 |

1.

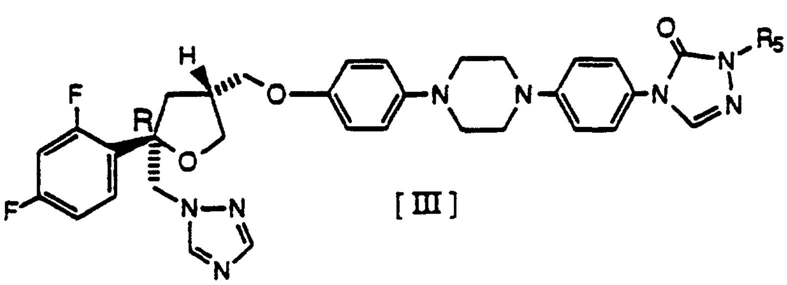

[III]

2. FLZ = fluconazole

## TABLE R

### IN VITRO ANTIFUNGAL ACTIVITY FOR SELECTED COMPOUNDS OF FORMULA III[1]

### PERCENTAGE OF STRAINS WITH MICs ≤1 MCG/ML

| | | (MCG/ML) | | | |
|---|---|---|---|---|---|
| ORGANISM | $R^5$ STRAINS | 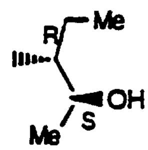 | 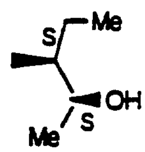 | 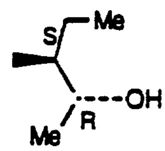 | FLZ[2] |
| Aspergillus | 37 | 50 | 62 | 89 | 0 |
| Candida Albicans | 26 | 100 | 100 | 100 | 100 |
| Candida Kursei | 1-16 | 88 | 94 | 100 | 0 |
| Candida Tropicalis & Stellatoidea | 5 | 100 | 100 | 100 | 100 |
| Candida Glabrata | 9 | 22 | 22 | 22 | 0 |
| Cryptococcus Neoformans | 3 | 100 | 100 | 100 | 0 |
| Dermatophytes | 6 | 67 | 83 | 100 | 0 |

1.

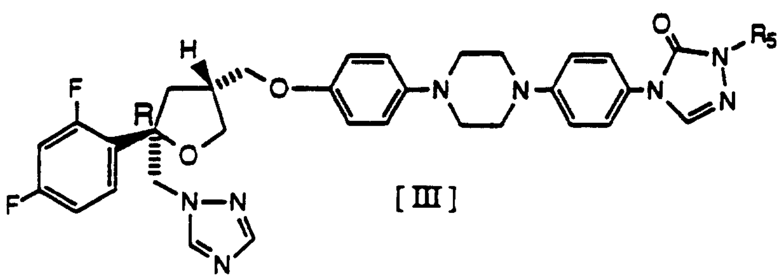

[III]

2. FLZ = fluconazole

## TABLE S

### IN VITRO ANTIFUNGAL ACTIVITY FOR SELECTED COMPOUNDS OF FORMULA III[1]

### PERCENTAGE OF STRAINS WITH MICs ≤1 MCG/ML

| | | (MCG/ML) | | | |
|---|---|---|---|---|---|
| | $R^5$ | 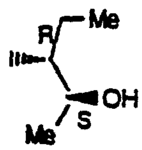 | 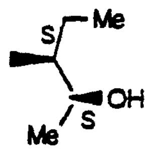 | 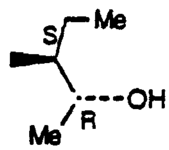 | |
| ORGANISM | STRAINS | | | | FLZ[2] |
| Aspergillus | 37 | .122 | .096 | .112 | 29.9 |
| Candida Albicans | 26 | .274 | .174 | .139 | .887 |
| Candida Kursei | 16 | .058 | .014 | .12 | 29.9 |
| Candida Tropicalis & Stellatoidea | 5 | .117 | .117 | .354 | .917 |
| Candida Glabrata | 9 | 28.8 | 17.1 | 28.8 | 29.3 |
| Cryptococcus Neoformans | 3 | .05 | .007 | .101 | 25.9 |
| Dermatophytes | 6 | .165 | .101 | .707 | 29.4 |

1.

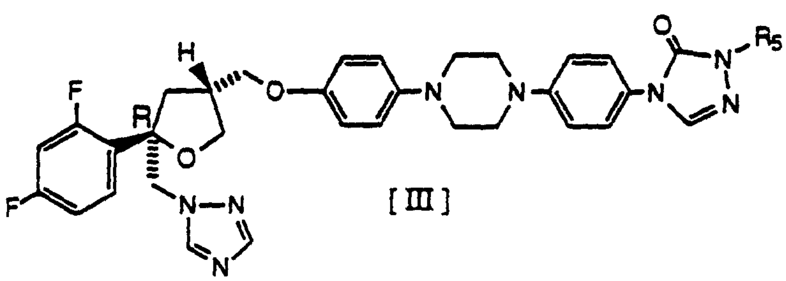

[III]

2. FLZ = fluconazole

The preferred esters of the compounds of the present invention of formula IV also exhibited superior in vivo antifungal activity against a broad range of fungi. After oral and parenteral *e.g.* IV administration in mice, rats, dogs and monkeys. The preferred esters of formula IV listed below wherein $R_9$ is :

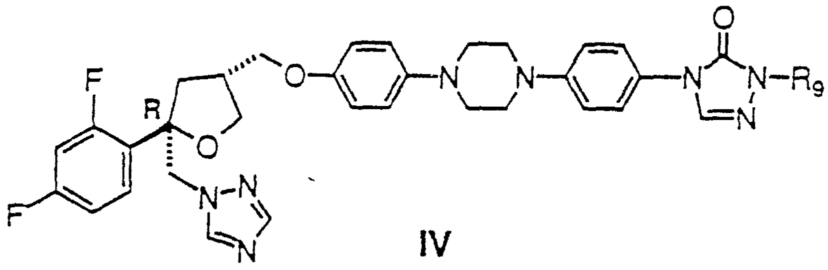

IV

| $R_9$ | $R_9$ |
|---|---|
| 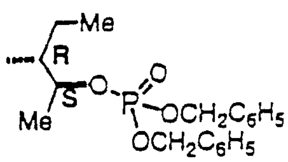 | 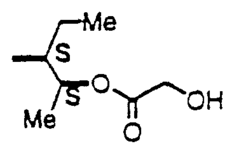 |
| $M^+$ 961.4 | $M^+$ 759.3 |

$R_9$

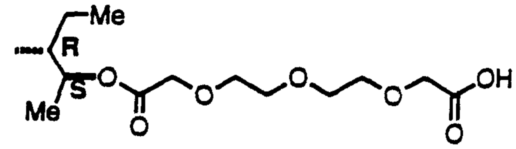

M+ 905

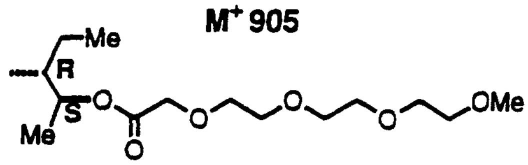

M+ 905

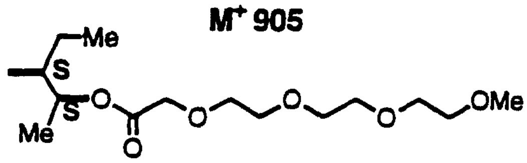

M+ 905

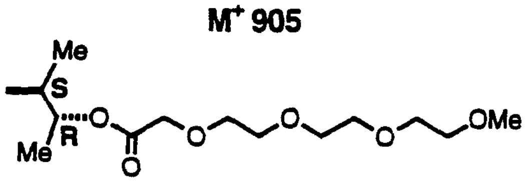

M+ 891

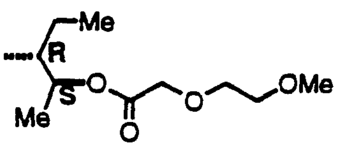

M+ 817

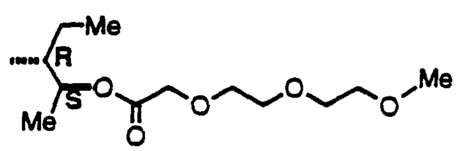

M+ 861

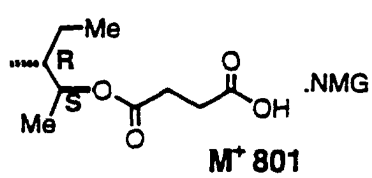

M+ 801

$R_9$

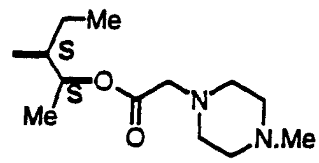

M+ 841

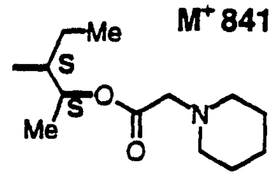

M+ 826

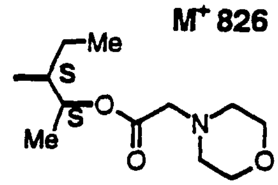

M+ 828

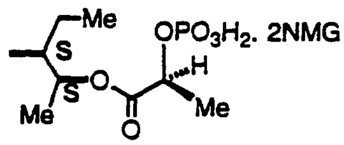

M+ 853.2

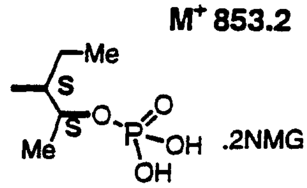

M+ 781.7

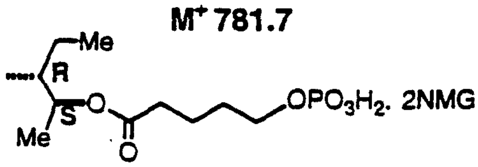

M+ 881.3

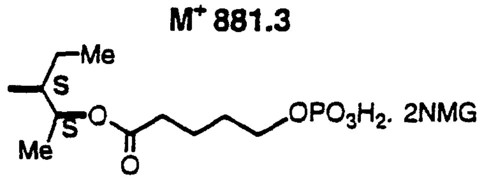

M+ 881.3

| $R_9$ | $R_9$ |
|---|---|
| 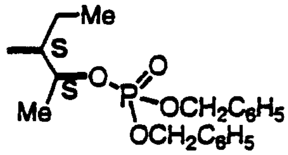 M+ 961.2 | 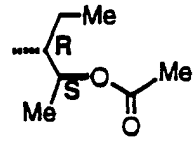 M+ 742.4 |
| 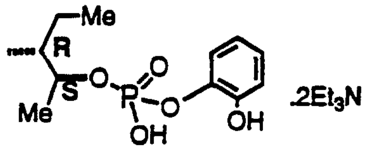 M+ 873.3 | 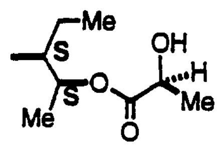 M+ 773.1 |
| 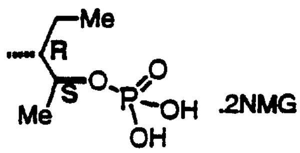 M+ 781.8 | 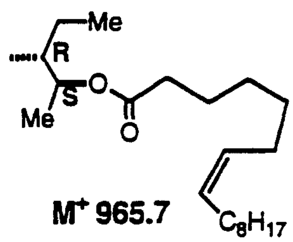 M+ 965.7 |
| 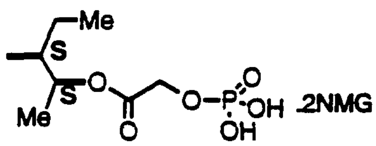 M+ 839 | 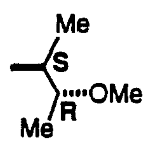 M+ 701.4 |
| 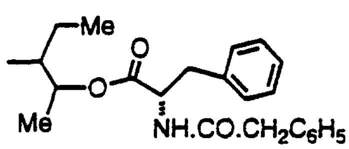 M+ 983 | 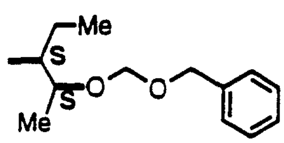 M+ 821.5 |

The more preferred esters listed hereinabove are readily metabolized in vivo to the corresponding alcohols *e.g.* $R_5$ is

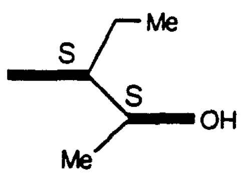

or

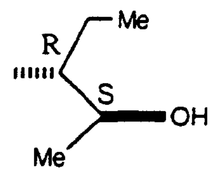

or

The most preferred metabolizable esters include those of compounds of formula IV wherein $R_9$ is

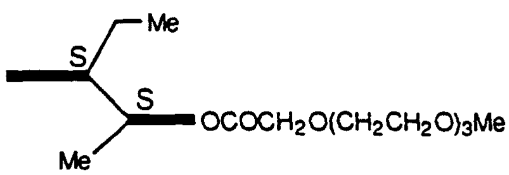

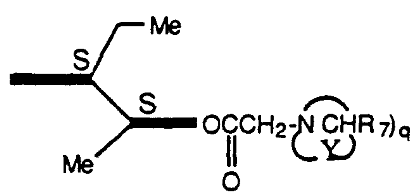

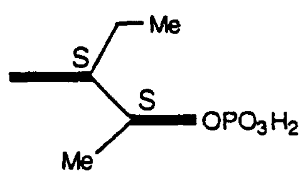

• 2NMG

wherein Y, $R_7$ and q are as defined herein above

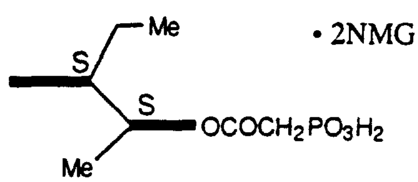

• 2NMG

and

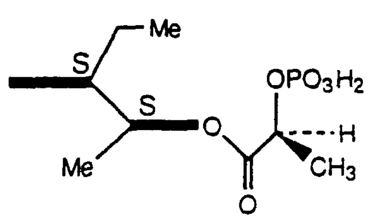

•2NMG

The antifungal compounds of this invention represented by formula I have the R absolute stereochemical configuration at the carbon in the tetrahydrofuran ring bearing the di-halophenyl and 1H, 1,2,4-triazol-1-ylmethyl moieties, and the $CH_2OY$ moiety has the "cis" stereochemical configuration relative to the 1H, 1,2,4-triazol-1-ylmethyl moiety. See the formula I hereinbelow.

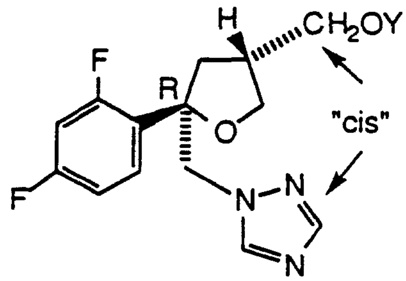

[I]

and

Y =

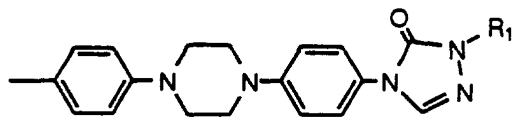

wherein $R_1$ is a straight or branched chain ($C_3$-$C_8$) alkyl group substituted by one or two hydroxy groups, which preferably exists as a single stereoisomer, but mixtures of stereoisomers are also contemplated as within the scope of this invention.

The compounds of formula I are generically but not specifically disclosed as the "cis" series, type ii, at col. 9 lines 59-68 of Saksena et al. USP 5,039,676 and Example 68 at Col. 5, line 16 to col. 52, line 44.

GENERAL SYNTHETIC PREPARATIONS

The compounds of this invention may be prepared by use of the sequence of steps illustrated in the following Schemes I-V. In Scheme I, compound 3 is readily prepared from commercially available compound 1 according to Examples 1a, 1b and 1c. Compound 4 is prepared by reaction of L(+) -diethyl tartarate ("L-DET") and molecular sieves in the presence of titanium tetra-isopropoxide $(i\text{-}PrO)_4Ti$ in an aprotic solvent, such as methylene chloride, at a temperature 0° to -35°C. See for Example, T. Katsuki, K.B. Sharpless, J. Am. Chem. Soc., 102, 5974 (1980); and 103, 464 (1981). An oxidizing agent, e.g. tert-butylhydroperoxide ("TBHP") is added to this reaction mixture (step d of Scheme I). Compound 3 is added and the compound of formula 4 (when L(+)-diethyl tartarate is used) is produced. Reaction of compound 4 with 1H-1,2,4-triazole in the presence of strong base, e.g., NaH in an aprotic solvent, such as DMF, at 0°-80°C provides the diol compound of formula 5. The primary hydroxy group in compound 5 is converted into a leaving group, e.g., mesylate or tosylate (compound 6) by reaction of 5 with, for example, mesyl chloride ("MsCl"), in an aprotic solvent, e.g., methylene chloride in the presence of base, e.g., triethylamine ("$Et_3N$"). Compound 6 is treated with strong base, e.g., sodium hydride (NaH) in an aprotic solvent, e.g., DMF at room temperature to give oxirane compound 7. Reaction of 7 with diethyl malonate in the presence of strong base, e.g., sodium hydride in an aprotic solvent, e.g., DMSO at 25°-75°C provides the lactone 8. Reduction of 8 with a metal hydride, e.g., lithium borohydride ($LiBH_4$) in an alcohol, e.g., ethanol (EtOH), provides the triol 9. Conversion of the two primary alcohols of 9 into leaving groups (mesylates or tosylates) by reaction of 9 with excess tosyl chloride in an aprotic solvent, e.g., THF, in the presence of base, e.g., $Et_3N$, provides ditosylate 10. Compound 10 is contacted with strong base, e.g., NaH, in an aprotic solvent such as toluene at elevated temperatures of 100°-120°C to provide a mixture of two tosylates (cis and trans) which are separated by chromatography to yield to the cis-tosylate 11. Reaction of compound 11 with alcohols HOY in the presence of strong base, such as NaH in an aprotic solvent, such as DMSO at a temperature of 25°-75°C provides compounds of formula I.

Scheme II provides an alternative reaction sequence to obtain compounds of the present invention. Reaction of compound 11 with the commercially available compound 12 in the presence of NaH gives compound 13. Hydrolysis of N-acetyl group in 13 is accomplished with a strong base such as NaOH in the presence of n-BuOH to provide compound 14. It should be made clear that instead of N-acetyl group in compound 12, any other base labile groups such as N-formyl, N-benzoyl, etc., can also be used to provide corresponding N-formyl and N-benzoyl derivatives of compound 13. Reaction of 13 with p-chloronitrobenzene in the presence of a hydrochloric acid scavenger such as $K_2CO_3$ provides the nitro compound 15. Catalytic reduction of 15 in the presence of a platinum or palladium catalyst yields the amine 16. Treatment of 16 with phenylchloroformate in the presence of pyridine gives the urethane intermediate 17. Reaction of 17 with hydrazine yields the semicarbazide 18 which is cyclized in the presence of formamidine acetate to furnish the key triazolone 19. Alkylation of 19 according to Examples 19 and 20 provides the compounds of structure 20 including compounds of formula I wherein $R_1$ is defined as hereinabove.

Scheme III provides a stereospecific access to the cis-alcohol 26 and cis-tosylate 11 by application of enzyme chemistry. For Example, reaction of the triol 9 with ethyl acetate in the presence of porcine pancreatic lipase gives a single monoacetate 21. The remaining primary hydroxy group in 21 is protected by an acid labile group such as tetrahydropyranyl group to give a compound such as 22. Hydrolysis of the acetoxy group in 22 is accomplished with a base such a KOH which provides 23. The remaining steps are: (i) tosylation of compound 23 to provide 24; (ii) cyclization of 24 in the presence of NaH to provide 25; (iii) deprotection of THP ether in 25 using an acid catalyst such as p-toluene sulfonic acid (to give 26) followed by tosylation of the resulting 26 to furnish the key intermediate 11.

A detailed description of a preferred preparation of key intermediate is disclosed in commonly owned U. S. Patent Application Serial No. 08/055,268, filed April 30, 1993, which is hereby incorporated by reference.

## Scheme I

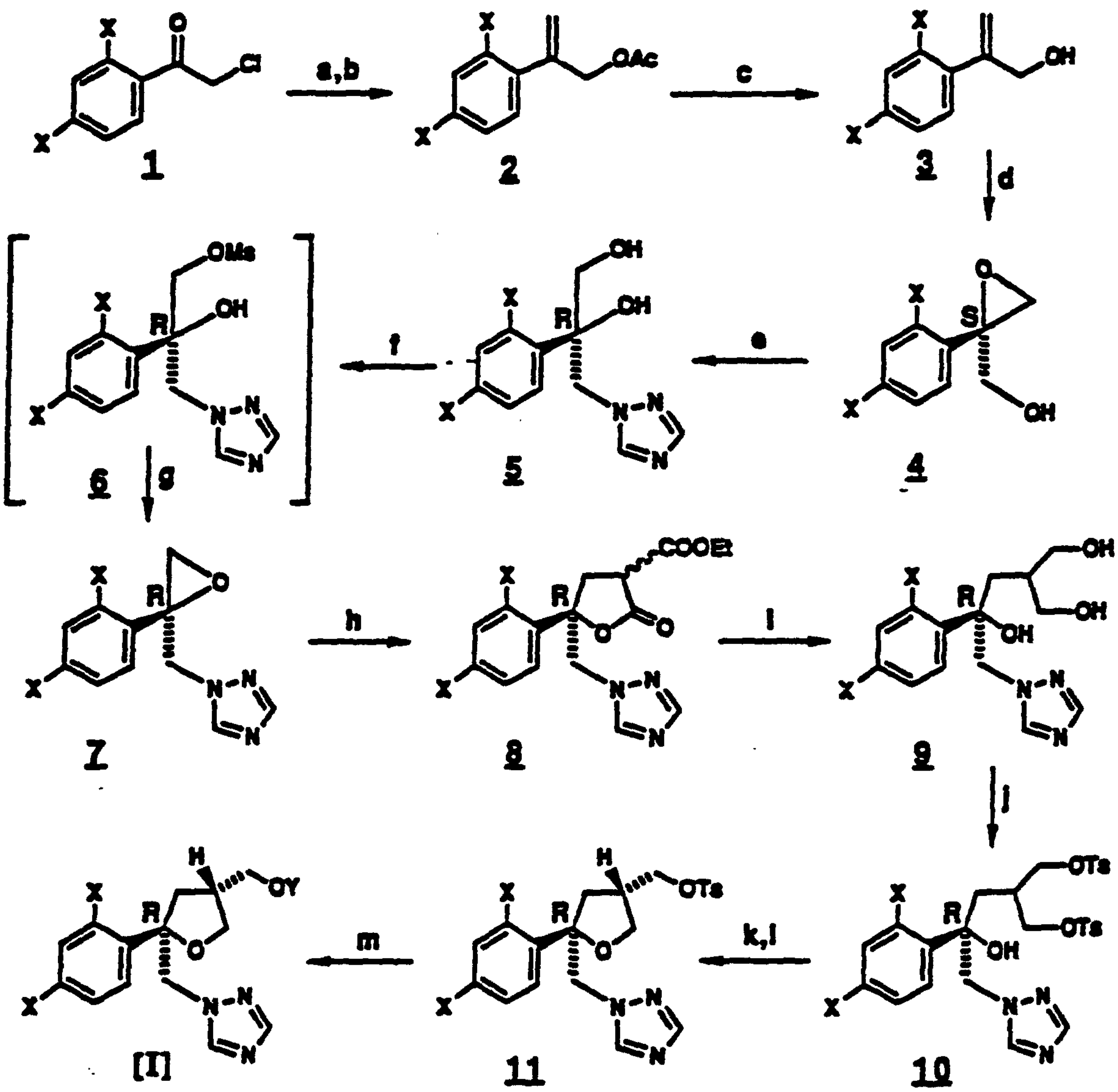


1 2 3 4 5 6 7 8 9 10 11 [I]

**Reagents:** (a) NaOAc; (b) Wittig Reaction; (c) KOH; (d) L-DET, TBHP, $(i\text{-}Pr)_4Ti$; (e) NaH, 1,2,4-triazole,DMF; (f) MsCl, $Et_3N$,$CH_2Cl_2$; (g) NaH, DMF; (h) NaH, $CH_2(COOEt)_2$, DMSO; (i) $LiBH_4$, EtOH; (j) TsCl, $Et_3N$, THF; (k) NaH, toluene, heat; (l) chromatography; (m) NaOY, DMSO

**X= F or Cl**

## Scheme II

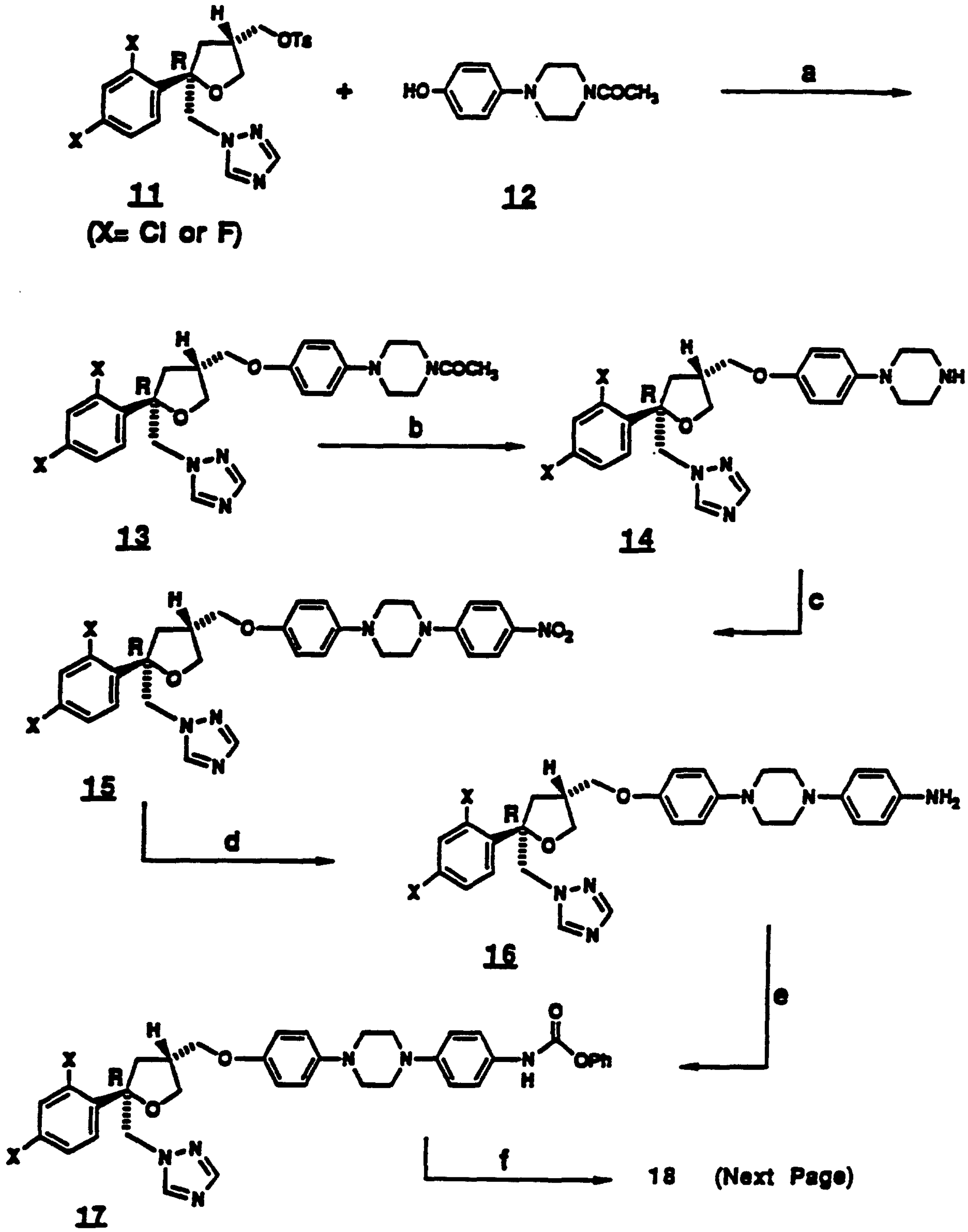


11 (X= Cl or F)

12

13

14

15

16

17

18 (Next Page)

## Scheme II (cont'd.)

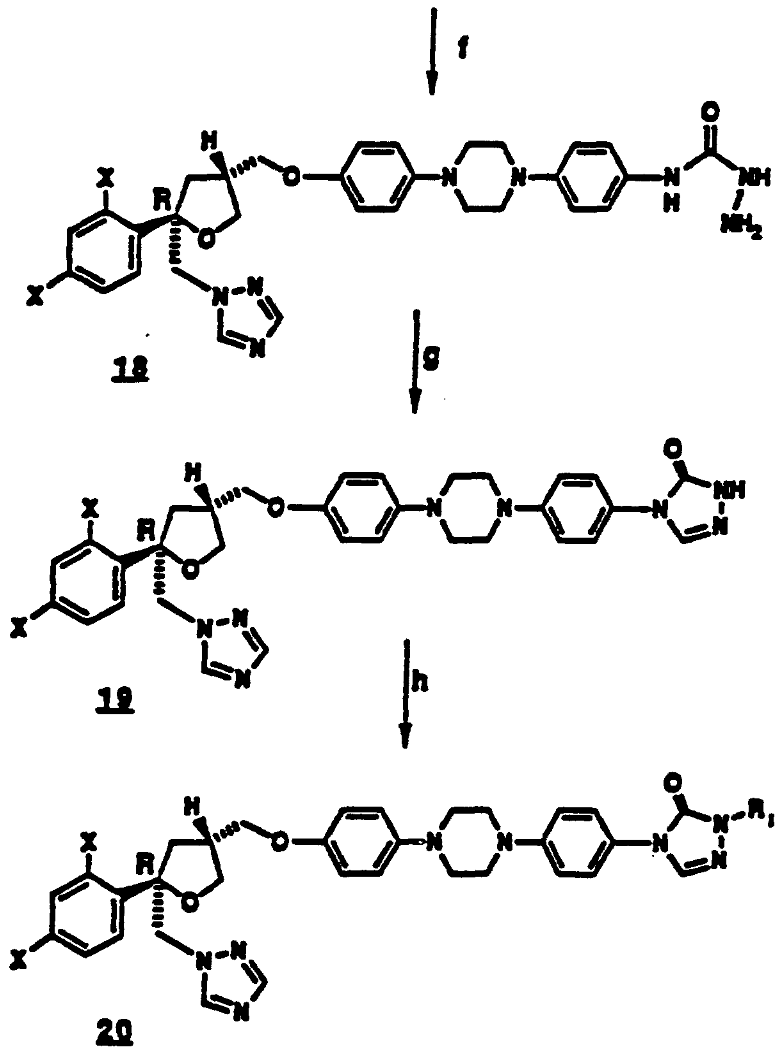


18

19

20

Reagents: (a) NaH; (b) NaOH/n-BuOH; (c) p-Cl-$C_6H_4NO_2$/ $K_2CO_3$/ DMSO; (d) $H_2$/ Pt/C; (e) $C_6H_5OCOCl$/ pyridine/ $CH_2Cl_2$; (f) $NH_2.NH_2$/ $H_2O$/ dioxane; (g) Formamidine acetate/ DMF/ heat; (h) according to Examples 19 and 20

## Scheme III

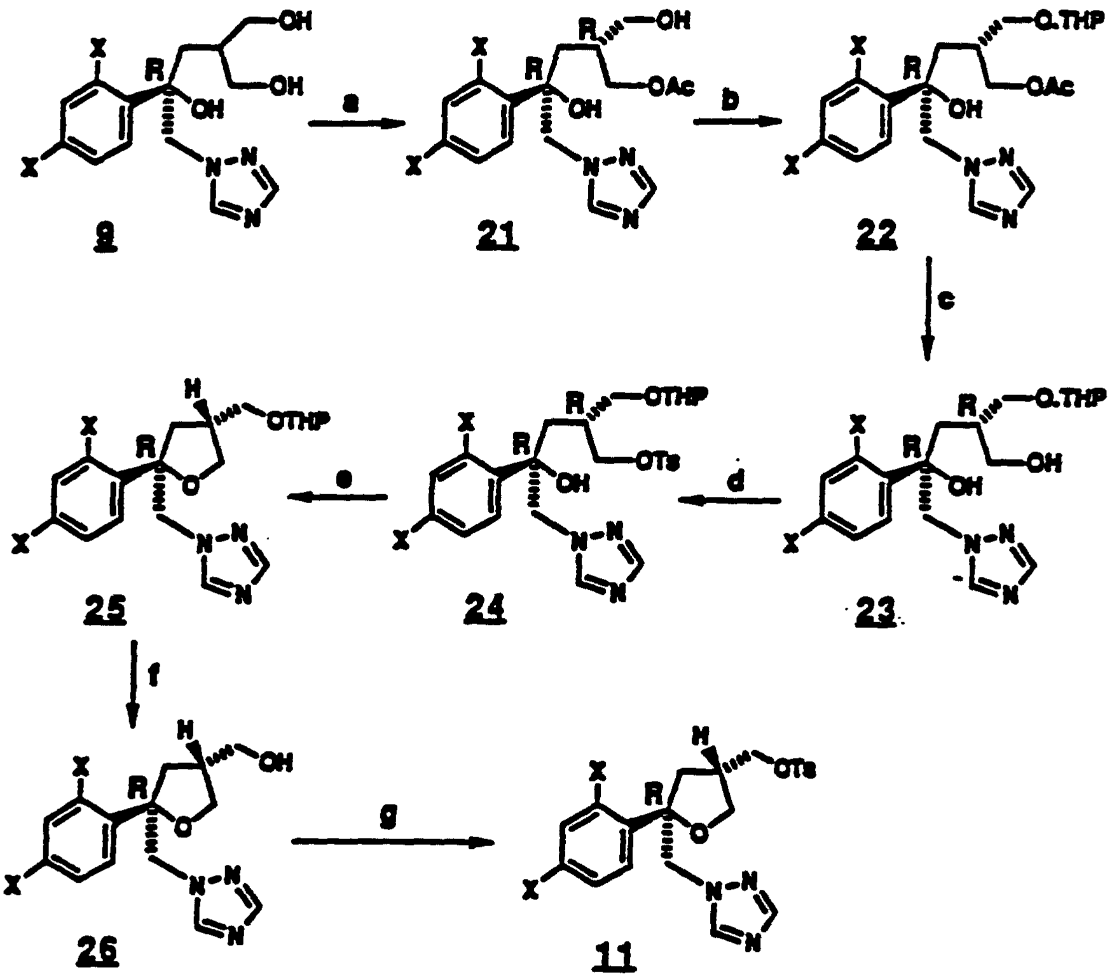


9 21 22 23 24 25 26 11

**Reagents:** (a) Porcine pancreatic lipase/ EtoAc; (b) dihydropyran/ $H^+$; (c) KOH; (d) Tosyl chloride/ pyridine; (e) NaH; (f) Methanol/ $H^+$; (g) Tosyl chloride/ pyridine.

## SCHEME IV

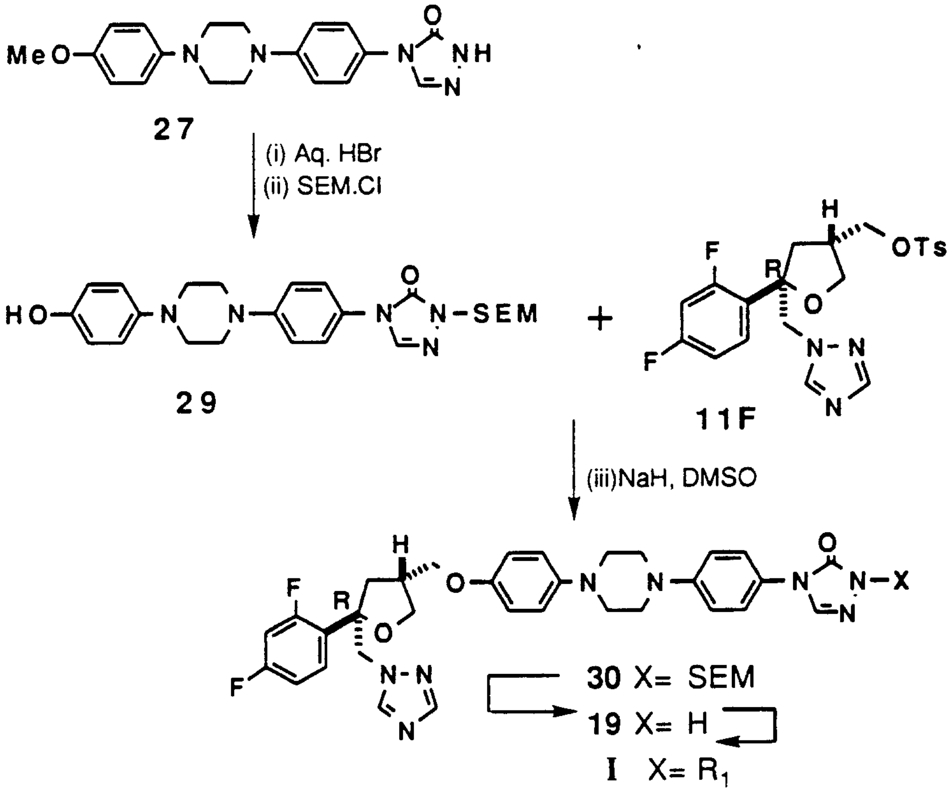

## SCHEME V

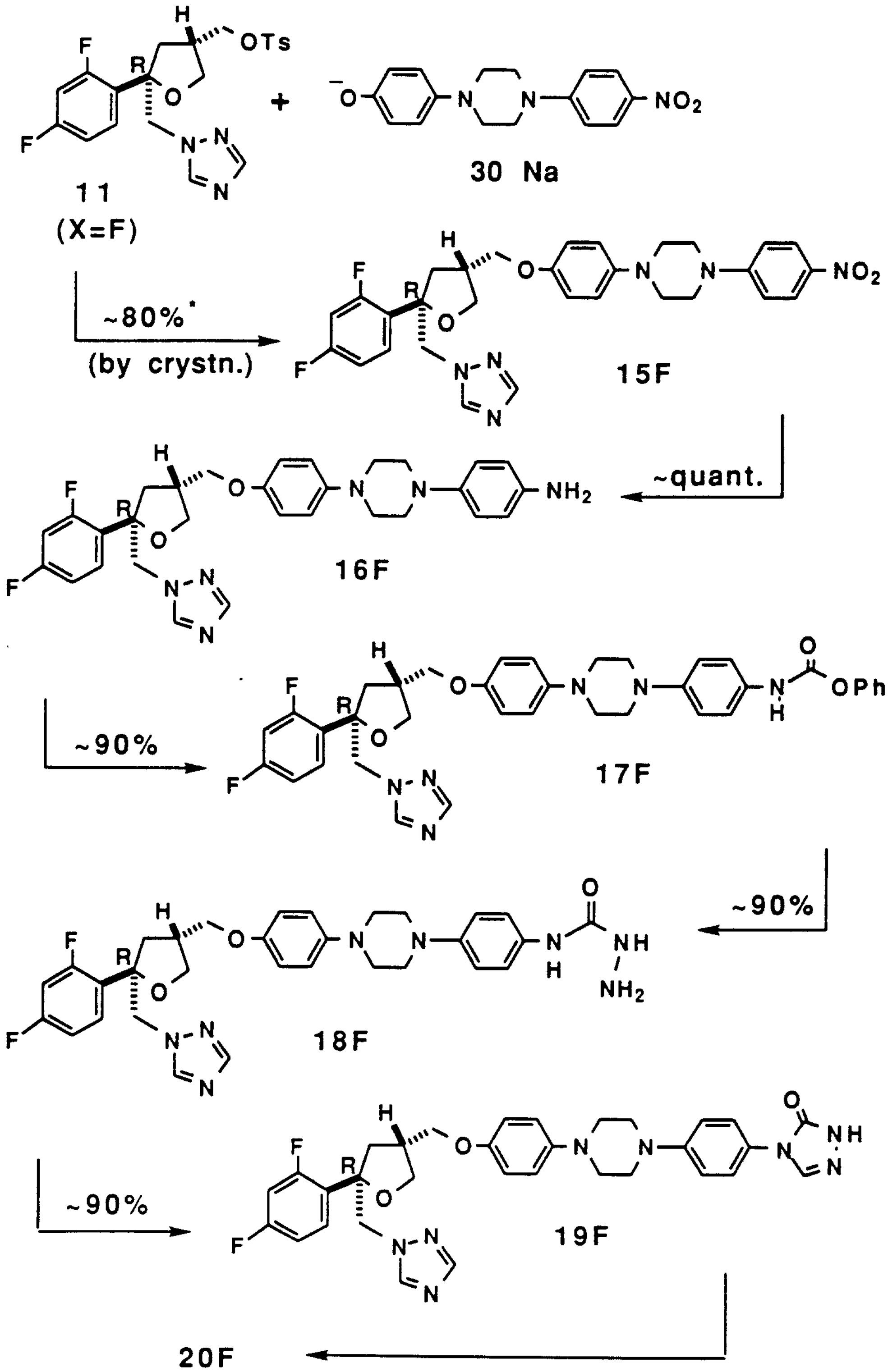

**SCHEME VI**

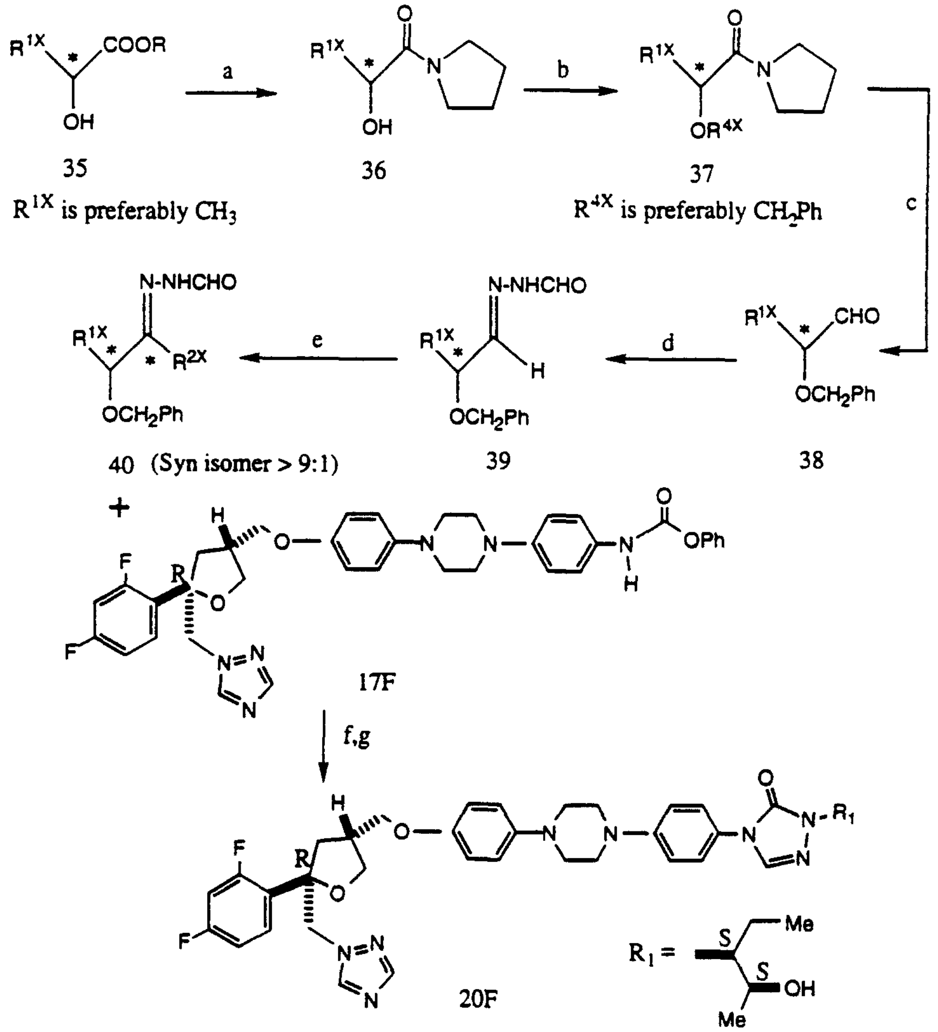


35, 36, 37

$R^{1X}$ is preferably $CH_3$

$R^{4X}$ is preferably $CH_2Ph$

40 (Syn isomer > 9:1), 39, 38

17F

20F

(a) pyrrolidine, r.t., 24 h; (b) $R^{4X}$-X, NaH, DMF; (c) RED-AL, toluene. -20°; (d) $H_2NNHCHO$, MeOH; (e) $R^{2X}MgBr$, $Et_2O$, -10°C to r.t., 24 h; (f) 17F of Scheme V and procedure of Example 32d; (g) $H_2$, Pd, HCOOH, 80°C.

## Scheme VII

## Preparation of Polyether Esters

$R_8(OCHR_7CHR_7)_tOH$ —$LG\text{-}(CHR_7)_sCO_2Na$(43), Base/THF→ $R_8(OCHR_7CHR_7)_t\text{-}O(CHR_7)_s\text{-}CO_2H$

42 44

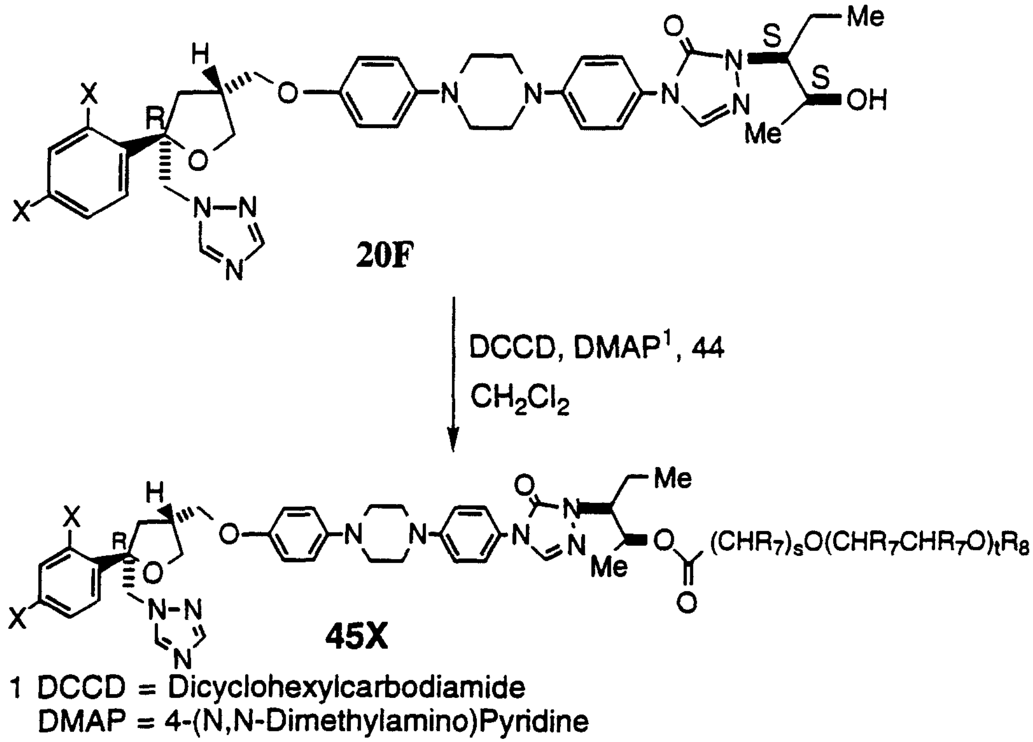


20F

45X

1 DCCD = Dicyclohexylcarbodiamide
DMAP = 4-(N,N-Dimethylamino)Pyridine

## Table for Scheme VII

$R^1$ = 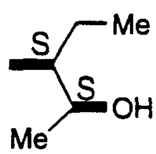

using 20F (X = F)

| 42 | 43 | 45 | M.S. |
|---|---|---|---|
| | | X | M+ |
| — | $PGOCH_2CO_2H$ PG = Protecting Group, e.g., $CH_2Ph$ | $COCH_2OH$ | 759.3 |
| $CH_3(OCH_2CH_2)_3OH$ | $ClCH_2CO_2H$ | $COCH_2O(CH_2CH_2O)_3Me$ | 905 |

## Table for Scheme VII

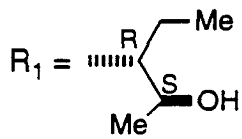

| **42** | **43** | **45** | **M.S.** |
|---|---|---|---|
| $CH_3(OCH_2CH_2)OH$ | $ClCH_2CO_2Na$ | $-COCH_2O(CH_2CH_2O)Me$ | 817 |
| $CH_3(OCH_2CH)_2OH$ | $ClCH_2CO_2Na$ | $-COCH_2O(CH_2CH_2O)_2Me$ | 861 |
| $CH_3(OCH_2CH_2)_3OH$ | $ClCH_2CO_2Na$ | $-COCH_2(CH_2CH_2O)_3Me$ | 905 |
| $HO_2C(OCH_2CH_2)_2OH$ | $ClCH_2CO_2Na$ | $-COCH_2O(CH_2CH_2O)_2COOH$ | 905 |

## Phosphate Esters

## Scheme VIIIA

20F

46$R_1$

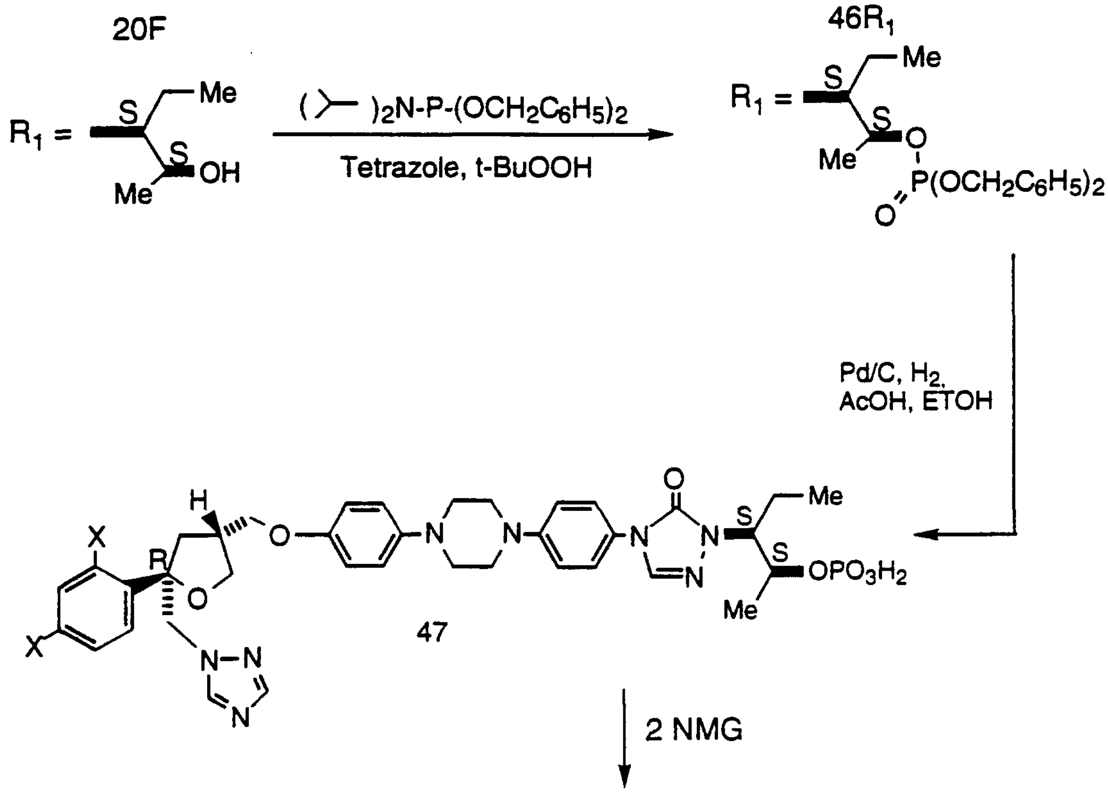


47

**47 • 2NMG**

## SCHEME VIIIB

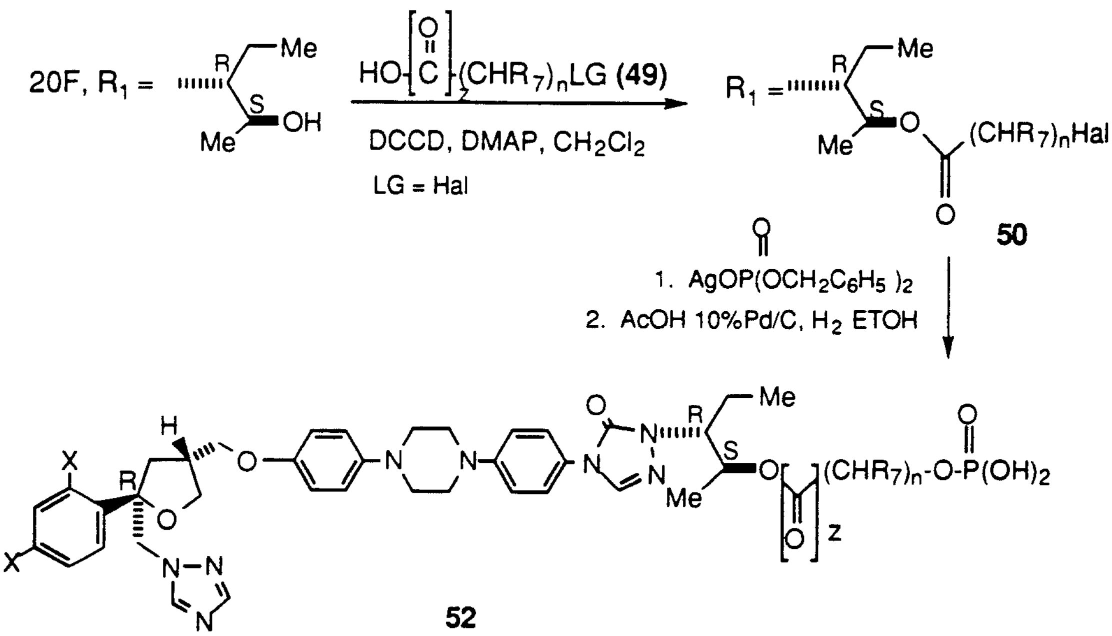


50

52

## Table for Scheme VIIIA

X = F

| 47 $R^1$ | | M.S. ($M^+$) |
|---|---|---|
| 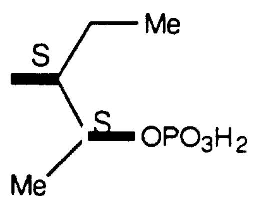 | .2NMG | 781.8 |
| 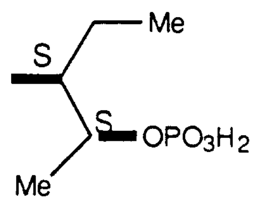 | .2NMG | 781.7 |
| 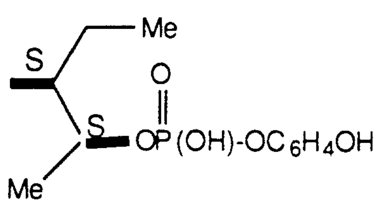 | | 873.3 |

| **47 $R^1$** | **M.S. ($M^+$)** |
|---|---|
| 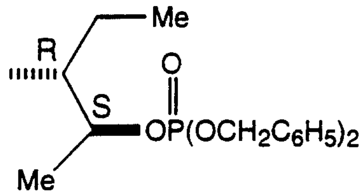 | 961.4 |
| 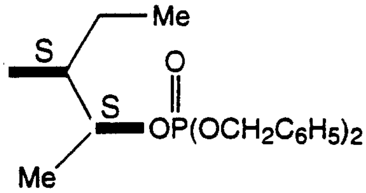 | 961.2 |

**Table for Scheme VIIIB**

| **49** | **$52R_1$** | **M.S. ($M^+$)** |
|---|---|---|
| $HOCOCH_2Cl$ | 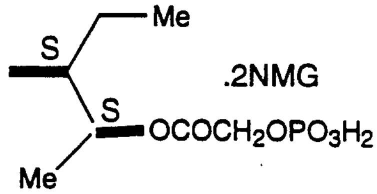 .2NMG | 839 |
| $HOCO(CH_2)_4OH$ | 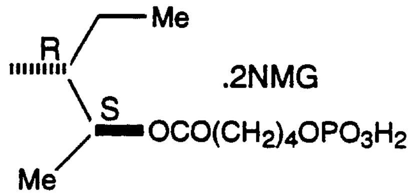 .2NMG | 881.3 |
| $HOCO(CH_2)_4OH$ | 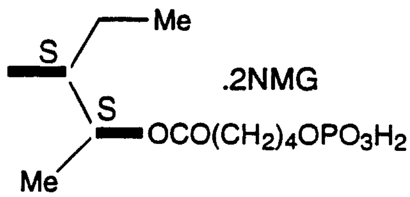 .2NMG | 881.3 |
| S-HOCOC*H(.OH)$CH_3$ | 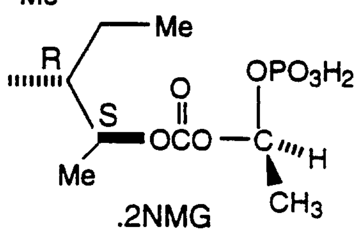 .2NMG | 853.2 |

## Scheme VIIIC

AR=
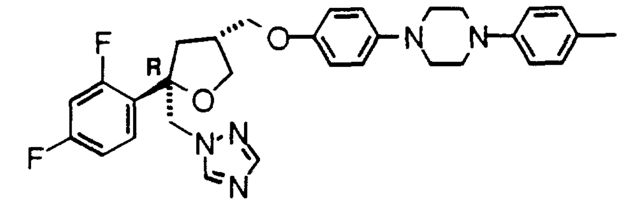

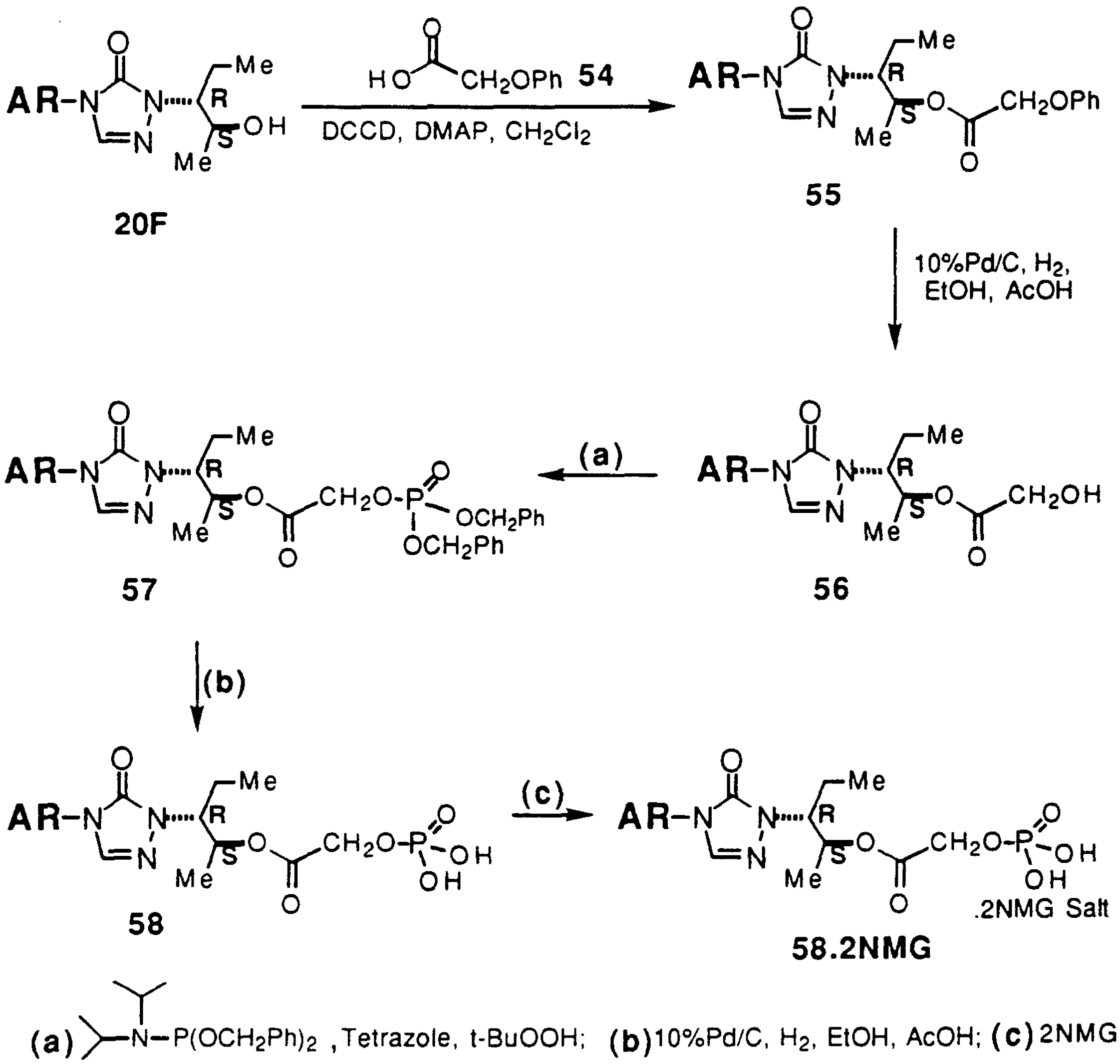


20F 55 56 57 58 58.2NMG

## Scheme IX
## Preparation of Heterocyclic Esters

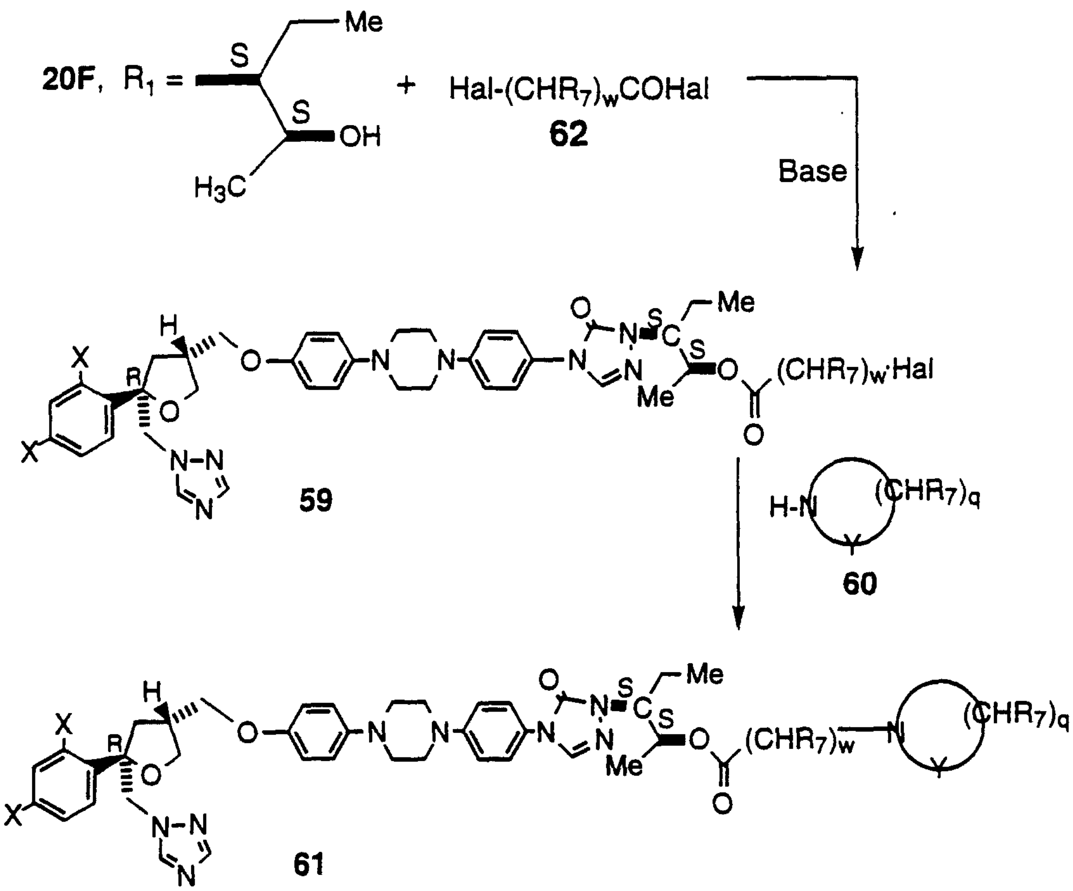

## Table for Scheme IX

| 61 $R_1$ | M.S. (M±) |
|---|---|
| 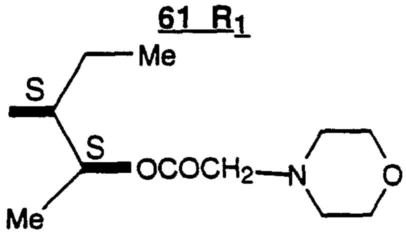 | 828 |
| 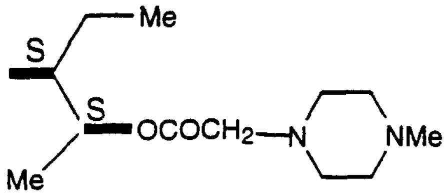 | 841 |
| 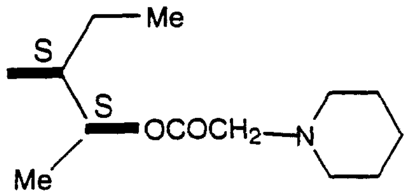 | 826 |
| 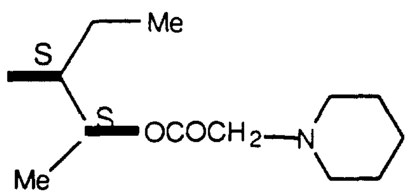 | 826 |

Scheme IV provides an additional reaction sequence to obtain the compounds of the present invention. Compound 27 is prepared from the methyl ether of compound 12 in Scheme II by subjecting the methyl ether of 12 to the reactions of steps a to g of Scheme II. Reaction of compound 27 with aqueous HBr or $BBr_3$ gives phenolic compound 28. Reaction of compound 28 with one equivalent of NaH and subsequent treatment with, for example, 2-(trimethyl)-silylethoxymethyl chloride ("SEM-Cl")and DMF at ambient temperatures produces SEM-nitrogen-protected compound 29. Deprotonation of compound 29 with NaH followed by reaction of the so-formed anion with tosylate 11 in DMF or DMSO at elevated temperatures produces compound 30. The nitrogen protecting group of 30, e.g., SEM is removed by treatment with, for example, 6NHCl in methanol at ambient temperatures for 3 hr to produce compound 19. Compound 19 is treated with NaH and DMSO at 20°C for 3/4 hr. and thereafter alkylated with $R_1X$ to produce compound I. In $R_1X$, $R_1$ is a $C_3$-$C_8$ alkyl group having at least one protected hydroxy moiety, e.g., O-SEM and X' is a leaving group, for example, brosylate. Removal of the hydroxy protecting group from compound 31, e.g., O-SEM is accomplished by, for example, 6NHCl in methanol to give compounds of this invention of formula I.

Scheme V provides a preferred route for preparation of the compounds of this invention set forth in Scheme II. The sodium salt of compound 31 prepared by reaction of (4-[4-(4-nitrophenyl)-1-piperazinyl]phenol with NaH in anhydrous DMSO at 50°-60° C for 30 minutes is reacted with the 2,4-diflurophenyl tosylate 11F (compound 11 in Scheme II wherein X=F) for 1 h. at 50°-70° C to provide, after flash silica chromatography or crystallization, compound 15F (compound 15 in Scheme II wherein X=F). Reduction of 15F by hydrogenation in the presence of 5% Pd/C in ethanol containing 1NHCl provided amino compound 16F (compound 16 in Scheme II wherein X=F). Reaction of 16F with phenylchloroformate in anhydrous pyridine at 0-5°C for 2h. provided phenylcarbamate 17F (compound 17 of Scheme II wherein X=F). Reaction of 17F with hydrazine hydrate in 1,2-dimethoxyethane at 80°C for 4h. provided the semicarbazide 18F (compound 18 of Scheme II wherein X=F). Reaction of 18F with formamidine acetate and $Et_3N$ in 2-methoxyethanol under dry argon in stirred reactor at 80°C overnight provided 3H-1,2,4-triazol-3-one 19F (compound 19 in Scheme II wherein X=F). Reaction of compound 19(f) with $R_1X$ in accordance with the procedure of Scheme IV produced compounds of formula I.

Scheme VI provides an alternative, stereoselective route for preparation of the preferred compounds of this invention. Compound 35 (*e.g.* S-lactic acid methylester) is contacted with excess pyrrolidine in methylene chloride for 24 hours at room temperature to give amide 36. Reaction of 36 and NaH with for example, benzyl halide in DMF gave 37. Selective reduction of amide 37 with a 3.4M solution of sodium bis(2-methoxyethoxy)aluminum hydride ("RED-Al") in toluene at -20°C gave aldehyde 38. Reaction of aldehyde 38 with $H_2NNHCHO$ in methanol gave 39 which was reacted with a Grignard reagent *e.g.* ethylmagnesium bromide in dry ether at a temperature of -10°C to room temperature for 24 hours to give 40 wherein the ratio of the S,S isomer: S,R isomer was 94:6. When the Grigand reaction was done in the presence of 1.2 equivalents of bis(trimethylsilyl)acetamide the SS to SR ratio was 99:1. Compound 40 was reacted with compound 17F of Scheme V in toluene in the presence of DBU (1,8-diazabicyclo[5.4.0]undec-7-ene) for six hours at 80°C. Cyclization was effected by raising the temperature to 100°-110°C and continuing to maintain this temperature overnight. After purification via TLC, 20F was obtained. Treatment of 20F with hydrogen and palladium black in methanol containing formic acid heated to 60°C gave the crude product which was isolated and purified (via TLC) to give compound 20F *i.e.* the compound of formula III wherein

$R_5$ =

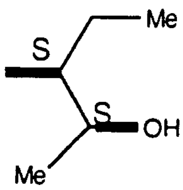

And X = F, Mt = 701•

The reaction of the Grignard reagent on the propanimine 39 produces 40 wherein the absolute stereochemistry induced at the new chiral center in 40 is substantially the same (i.e., S) as that at the chiral carbon in 39. By the term "substantially the same" as used herein is meant the ratio of S:S to S:R (in e.g., 40) is greater than 9:1, preferably is greater than 15:1 and most preferably is at least 99:1.

The mass spectral data presented herein as $M^+$ are parent ions which were determined by Fast Atom Bombardonment (FAB) technique and represent the ·M+1 peaks.

Scheme VII provides a general method for preparation of the polyether esters of alcohols of the present invention. The alcoholate of alcohol ether 42 e.g. $CH_3(OCH_2CH_2)_3OH$ i.e., 42 wherein $R_7$ = H and t = 3, was prepared by reaction, of 42 with excess strong base e.g. NaH in an anhydrous ether e.g. THF at ice bath temperatures. The so-formed reaction mixture was stirred for several hours i.e., 2 or more and the sodium salt of acid 43 e.g. sodium salt of chloroacetic acid (43 wherein LG=Cl, $R_7$=H and s=1) was added thereto. The so-formed reaction mixture was stirred at ice-bath temperatures and stirring was continued as temperature was allowed to warm to room temperature. Water was carefully added to the reaction mixture and the polyether acid 44 was separated and purified by conventional techniques.

To a solution of 44 in $CH_2Cl_2$ was added 1.3-1.5 equivalents of the base 4-(N,N-dimethylamino)pyridine ("DMAP") and 20F wherein

$R_1$ =

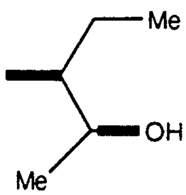

The temperature of the so formed reaction mixture was lowered by use of an ice bath and 1.3-1.5 equivalents of dicyclohexylcarbodiimide ("DCCP") was added thereto. The so-formed reaction mixture was continuously stirred as the temperature was allowed to warm to room temperature. The urea precipitate was removed and the crude product isolated by conventional techniques. The so formed residue was purified by chromatography on silica gel to provide the pure compound $[M + H]^+$ = 906 by FAB. By the appropriate substitution of different starting materials 42 and 43 the compounds 45 listed in Table for Scheme VII were prepared. The MS values for products listed under 45 in the Table for Scheme VII were measured by Fast Atom Bombardment ("FAB").

Schemes VIII A-C illustrate the generalised methods for preparing phosphate esters of the alcohols of this invention. Scheme VIIIA provides a method for preparation of phosphate esters of formula IV wherein $R_6$ is

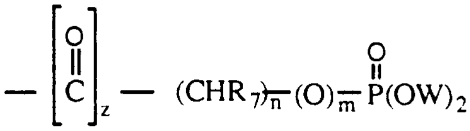

and z = m=n = 0. Compound 20F of Scheme II in methylene chloride at room temperature was reacted with 1.5 equivalents of N,N-diisopropyls-dibenzylphosphoramide, 1.5 equivalents of tert-butyl peroxide (3M in iso-octane) and a base such as tetrazole for several hours. The progress of the reaction was followed by TLC (5% methanol:EtoAc v:v) on silica gel. The crude product in EtoAc was washed with sodium thiosulfate and purified using standard techniques to provide the dibenzylphosphate ester 46. The dibenzyl ester groups of 46 were removed to give 47 by treatment of 46 dissolved in equal volumes of ethanol and glacial acetic acid in the presence of excess 10% Pd/C under a hydrogen atmosphere at room temperature in a stirred reactor overnight. The reaction was continued until no starting material was found by TLC (or NMR). The catatyst was removed by filtration and the crude phosphate ester 47 was purified by standard techniques. Treatment of 47 in methanol at room temperature with two equivalents of base e.g. NMG (or $Et_3N$) provided 47 • 2NMG. The compounds 46 and 47 prepared in accordance with Scheme VIIIA are listed in the Table for Scheme VIIA.

Scheme VIIIB illustrates preparation of phosphate esters of formula IV wherein

$R_6$ = 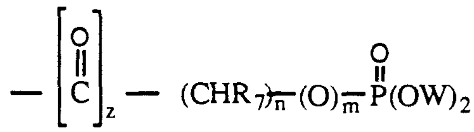

z =m=1 and n = o. Compound 2OF dissolved in methylene chloride was treated with 1.3 equivalents of DMAD 1.3 equivalents of DCCD and 1.3 equivalent of the acid 49 of the formula

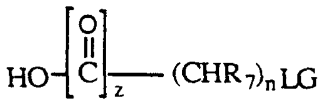

e.g., $HO_2C(CH_2)_4Br$, i.e., z = 1, n = 4, $R_7$ = H and the leaving group LG is Br. The reaction was stirred at room temperature until no starting material was found by TLC purification of the crude product gave bromide 50, a white solid wherein

$R_1$ = 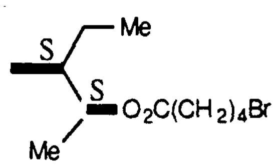

The bromide 50 in a benzene was heated at 80°C overnight with 1.5 equivalents of silver dibenzylphosphate (available from Sigma Chemical Co., St. Louis). The reaction mixture was cooled and washed with aqueous base, e.g., $K_2CO_3$. The crude product was separated and purified by silica gel column chromatography to give the dibenzyl phosphate ester 51. Treatment of 51 in ethanol/glacial acetic acid with excess 10% Pd/C under a hydrogen atmosphere overnight at room temperature gave phosphate ester 52. Treatment of 52 in methanol with two equivalents of base e.g. NMG (or $Et_3N$) gave 52 • NMG.

Scheme VIIIC provides an alternative procedure for preparation of phosphate esters of formula IV wherein $R_6$ is as defined above for Scheme VIIIB and z = 1 and n = 1. The benzyl ether of methyl acetate 53 in methanol-water and excess base e.g. $K_2CO_3$ were stirred overnight at room temperature to give the benzyl ether 54. Reaction of a solution of 20F and 54 in methylene chloride with a 1.3 - 1.5 equivalents of DCCD and DMAP at room temperature overnight gave ester 55. The benzyl ether group of 55 was removed by treatment with excess 10% Pd/C in ethanol-glacial acid under a hydrogen atmosphere at room temperature overnight. Purification of the crude product gave 56. Treatment of 56 with 1.5 equivalents of diisopropyldibenzylphosphate and 1.5 equivalents of tert-butyl peroxide and tetrazole in accordance with the procedure of Scheme VIIIB gave dibenzyl ester 57. Removal of the dibenzyl groups with 10% Pd/C in ethanol-glacial acetic acid under hydrogen atmosphere gave (as described hereinabove) phosphate ester 58. Treatment of 58 with two equivalents of base, e.g. NMG, gave 58 • 2NMG.

Scheme IX illustrates the preparation of heterocyclic esters of the present invention. Compound 20F, wherein

$R_1$ = 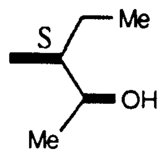

dissolved in methylene chloride is reacted with compound 62 in the (Hal=Br or Cl, w=1-5, e.g., Cl-$CH_2$-COCl) in presence of a base such as pyridine at a temperature of 0°-5°C for four hours. The reaction was placed in a refrigerator overnight. Additional compound 62 and base could be added, if necessary, and the reaction continued until no 20F is present by TLC. Purification of the crude product by column chromatography on silica gel gave pure 59 (w=1, Hal=Cl). Reaction of 59 with excess of the nitrogen heterocyclic compound 60 (e.g., Y=NH, $R_7$=H and q=4) at a temperature of 50°-60°C for 1 hour produced 61. Substitution of nitrogen heterocyclic compound 60 with a five and six membered compounds, e.g. morphiline, N-methylpiperdine provided the compounds listed in table below Scheme IX.

The alkanoate and alkenoate esters of 20F are conveniently prepared by standard synthetic techniques, (for example, by reaction of the anhydride or acid halide of the alkanoic acid or alkenoic acid in tghe presence of base e. g, pyridine) produced the alkanoate or alkenoates of the compounds of formula I.

The sulfate esters may be prepared by reaction of the alcohol compounds of formulas I to IV with sulfur trioxide in the presence of excess pryridine at temperatures of 70°-90°C for at least 2 hours in accordance with the procedure of R.M. Moriarty et. al. Tetrahedron Letters, Vol. 35, No. 44, p 8103-8106 (1994).

The compounds of formula I may also be prepared by reaction of compound 11 with alcohols of formula HOY in the presence of a strong base, e.g., NaH in an aprotic solvent, such as DMSO.

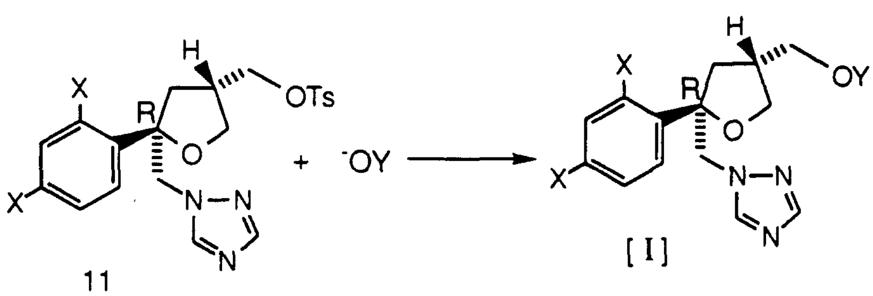

11 [I]

(R)-"Tosylate" Series

See Example 15

wherein X = F or Cl

Y = 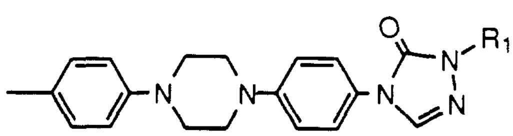

and $R_1$ = a ($C_3$-$C_8$) alkyl group substituted by one or two hydroxy moieties.

Compounds represented by formula I exhibit broad spectrum antifungal activity, in conventional antifungal screening tests, against human and animal pathogens, such as the following: *Aspergillus*, *Blastomyces*, *Candida,*

*Cryptococcus*, *Coccidioides*, *Epidermophyton*, *Fonsecaea*, *Fusarium, Mucor*, *Saccharomyces*, *Torulopsis*, *Trichophyton*, *Trichosporon*, *Sporothrix and Pneumocysitis.*

The preferred compounds of formula IV exhibit topical, oral and parenteral antifungal activity in in vivo tests in animals and such activity is unexpectedly better than that of existing antifungal agents *e.g.* itraconazole and fluconazole as well as that of the azole compounds specifically disclosed by Saksena et al. in USP 5,039,676 and International Publication No. WO 93/09114.

The antifungal compounds of formula I and pharmaceutical compositons of this invention are expected to exhibit anti-allergic, antiinflammatory and immunomodulating activities, broad spectrum antiinfective activity, e.g., antibacterial, anti-protozoal and antihelminthic activities.

The present invention also provides a composition for treating or preventing fungal infections comprising an antifungally effective amount of a compound represented by formula I or a pharmaceutically acceptable salt thereof and a pharmaceutically acceptable carrier or diluent therefor.

The pharmaceutical compositions of the present invention may also contain a fungicidally effective amount of other antifungal compounds such as cell wall active compound. The term "cell wall active compound", as used herein, means any compound that interferes with the fungal cell wall and includes, but is not limited to, compounds such as papulacandins, echinocandins, and aculeacins as well as fungal cell wall inhibitors such as nikkomycins, e.g, nikkomycin K and others which are described in USP 5,006,513.

The pharmaceutically acceptable salts of the compounds of the present invention include pharmaceutically acceptable acid and base addition salts.

The preferred pharmaceutically acceptable acid addition salts are nontoxic acid addition salts formed by adding to the compounds of the present invention about a calculated amount of a mineral acid, such as HCl, HBr, $H_2SO_4$, $HNO_3$ or $H_3PO_4$, or of an organic acid, such as an alkyl or arylsulfonic acid such as methanesulfonic, isithionic, paratoluenesulfonic, naphthylsulfonic and the like.

The pharmaceutically acceptable bases found suitable for use in the present invention are those which form pharmaceutically acceptable salts of the acidic pharmaceutically acceptable esters of the antifungal compounds of formulas I, II, III or IV and include suitable organic and inorganic bases. Suitable organic bases include primary, secondary and tertiary alkyl amines, alkanolamines, aromatic amines, alkylaromatic amines and cyclic amines. Exemplary organic amines include the pharmaceutically acceptable bases selected form chloroprocaine, procaine, piperazine, glucamine, N-methylglucamine, N-N-dimethyl glucamine ethylendediamine, diethanolamine, diisopropylamine, diethylamine, N-benzylenediamine, diethanolamine, diisopropylamine, diethylamine, N-benzyl-2-phenylethylamine, N-n'dibenzylethylenediamine, choline, clemizole, triethylamine ("$ET_3N$"), tris(hydroxymethyl)aminomethane, or D-glucosamine. The preferred organic bases include N-methyl glucamine ("NMG"), diethanolamine, and tris(hydroxymethyl) aminomethane ("TRIS"). Use of two equivalents of NMG in this invention is more preferred. The suitable inorganic bases also include alkali metal hydroxides such as sodium hydroxide.

The pharmaceutical compositions of the present invention may be adapted for any mode of administration e. g., for oral, parenteral, e.g., sc, im. IV and IP, topical or vaginal administration or by inhalation (orally or intranasally) Such compositions are formulated by combining the compound of formula I or an equivalent amount of a pharmaceutically acceptable salt of compound I with an suitable, inert, pharmaceutically acceptable carrier or diluent.

Examples of suitable compositions include solid or liquid compositions for oral administration such as tablets, capsules, pills, powders, granules, solutions, suppositories, troches, lozenges, suspensions or emulsions. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet, the active compound is mixed with carrier having the necessary binding properties in suitable proportions and compacted in the shape and size desired.

Topical dosage forms may be prepared according to procedures well known in the art, and may contain a variety of ingredients, excipients and additives. The formulations for topical use include ointments, creams, lotions, powders, aerosols, pessaries and sprays.

For preparing suppositories, a low melting wax such as a mixture of fatty acid glycerides or cocoa butter is first melted, and the active ingredients are dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection. Liquid preparations can also be formulated in solution with an appropriate amount of a hydroxypropyl $\alpha$- $\beta$ or -$\gamma$-cyclodextrin having 2 to 11 hydroxypropyl groups per molecule of cyclodextrin, polyethylene glycol, e.g., PEG-200 or propylene glycol, which solutions may also contain water. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the active component in finely divided form in water. A particularly preferred

aqueous pharmaceutical composition may be prepared from the compounds of formulas I to IV together with hydroxypropyl-β-cyclodextrin in water. The use of derivatives of α-, β- and γ-cyclodextrins, for example, hydroxpropyl-β-cyclodextrin are disclosed by N. Bodor USP 4,983,586, Pitha USP 4,727,064 and Janssen Pharmaceutical International Patent Application No. PCT/EP 84/00417.

The pharmaceutical compositions of the present invention may be prepared by admixing the pharmaceutically acceptable carrier, e.g., a hydroxypropyl-β-cyclodextrin in water, and adding thereto an antifungally effective amount of a drug of the present invention. The solution so formed is filtered, and optionally, the water may be removed by well known methods, e.g., rotatory evaporation or lyophilization. The formation of the solution may take place at a temperature of about 15° to 35°C. The water is normally sterilized water and may also contain pharmaceutically acceptable salts and buffers, e.g., phosphate or citrate as well as preservatives. The molar ratio of the antifungal compound of formula I to hydroxpropyl-β-cyclodextrin is about 1:1 to 1:80, preferably 1:1 to 1:2. Normally the hydroxypropyl-β-cyclodextrin is present in molar excess.

Also included are solid form preparations which are intended to be converted, shortly before use, into liquid form preparations for either oral or parenteral administration. The solid form preparations intended to be converted to liquid form may contain, in addition, to the active materials, such as compounds of this invention, and optionally a cell wall active compound, especially a fungal cell wall inhibitor, e.g., a nikkomycin, flavorants, colorants, stabilizers, buffers, artificial and natural sweeteners, dispersants, thickeners, solubilizing agents and the like. The solvent utilized for preparing the liquid form preparations may be water, isotonic water, ethanol, glycerin, polyethylene glycols, propylene glycol, and the like, as well as mixtures thereof.

Parenteral forms to be injected intravenously, intramuscularly, or subcutaneously are usually in the form of a sterile solution, and may contain salts or glucose to make the solution isotonic.

The topical dosage for humans for antifungal use in the form of a pharmaceutical formulation comprising a compound of formula I (usually in the concentration in the range from about 0.1% to about 20% preferably from about 0.5% to about 10% by weight) together with a non-toxic, pharmaceutically acceptable topical carrier, is applied daily to the affected skin until the condition has improved.

In general, the oral dosage for humans for antifungal use ranges from about 1 mg per kilogram of body weight to about 50 mg per kilogram of body weight per day, in single or divided doses, with about 2 mg per kilogram of body weight to about 20 mg per kilogram of body weight per day being preferred and the dose of about 5 mg per kilogram of body weight to about 10 mg per kilogram of body weight per day being most preferred.

In general, the parenteral dosage for humans for antifungal use ranges from about 0.5 mg per kilogram of body weight per day, to about 20 mg kilogram of body weight per day, in single or divided doses, with about 1 to about 10 mg per kilogram of body weight per day being preferred.

The exact amount, frequency and period of administration of the compounds of the present invention for antifungal use will vary, of course, depending upon the sex, age and medical condition of the patent as well as the severity of the infection as determined by the attending clinician.

GENERAL EXPERIMENTAL

The compounds of this invention are prepared in accordance with Schemes I-IX hereinabove and the following Examples using commercially available starting materials.

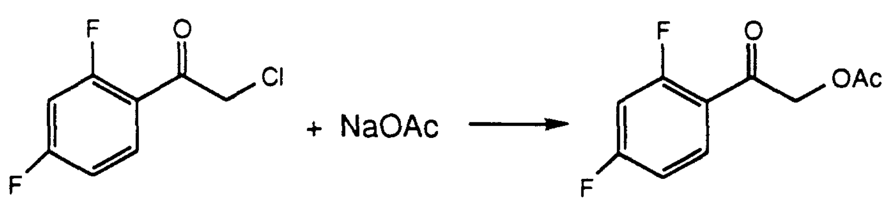

EXAMPLE 1a

2-Acetyloxy-1-(2,4-difluorophenyl)ethanone

Add 191 g of 2-chloro-2',4'-difluoroacetophenone (Aldrich Chemical Co.) to a mixture of 246 g of sodium acetate, 3 g of NaI, and 3 L of DMF. Stir the mixture at 20°C for 18 hr. then concentrate it to 1 L. Pour the residue into 6 L of cold dilute aqueous HCl and extract with EtOAc. Wash the extract with brine, dry it over anhydrous $Na_2SO_4$,

filter the so-formed mixture, and evaporate the filtrate to leave a residue. Chromatograph the residue on silica gel, eluting with $CH_2Cl$-2-hexane to obtain 198 g of the title compound.

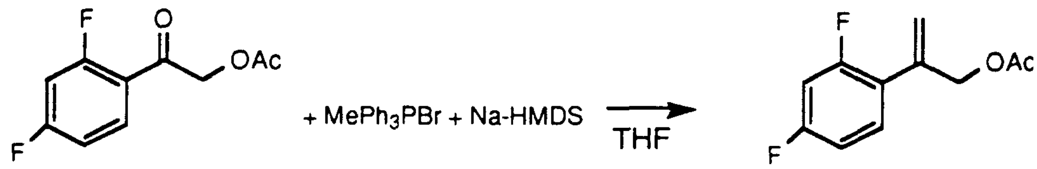


EXAMPLE 1b

1-[2-(2,4-Difluorophenyl)]-2-propenol acetate

Suspend 131 g of $MePh_3PBr$ in 270 mL of mechanically-stirred, dry THF at 20°C. Add 393 mL of 1M NaN $(Me_3Si)_2$ in THF, slowly at first, then rapidly over 5 min. while applying just enough ice cooling to maintain the temperature at < 23°C. Stir the so-formed mixture for 1 hr at 20°-24°C, cool it to ~-70°C, and stir it another 1/2 hr. Then add thereto a solution of 65.5 g of the product of Example 1a in 140 mL of dry THF, at a rate slow enough to keep the temperature below -70°C. Continue to stir the so-formed reaction mixture in the cold bath overnight during which the temperature rises to 20°C. Add 50 mL of EtOAc to the so-formed suspension, and then add 3 L of hexane. Allow the so-formed mixture to stand for ~ 15 min., and suction-filter to remove $Ph_3PO$. While the filter cake is still damp, transfer it to a beaker. Triturate the cake thoroughly with 1/2 L of hexane and suction-filter again to remove the remainder of product. Wash the combined hexane filtrates with 2 x 1 L of a 1:1 (v/v) MeOH-water, and then with brine. Dry the organic layer over $MgSO_4$, filter and evaporate the filtrate to leave a red oil. Add 1.5 L of hexane and suction-filter through a Celite pad to leave a clear yellow solution. Chromatograph the yellow oil on silica gel, eluting with 1/2 L of hexane, then 1L of 15:1 (v/v) hexane-EtOAc. Combine the homogeneous fractions to yield 38.6 g of the title compound as an oil.

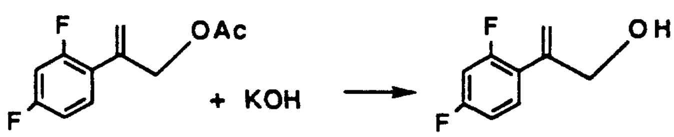


EXAMPLE 1c

2-(2,4-Difluorophenyl)-2-propenol.

Dissolve 40 g of the product of Example 1b in 400 mL of dioxane. Add a solution of 18 g of 85% KOH in 315 mL of water. Stir the so-formed mixture vigorously for 1 hr, and then pour the mixture into 1 L of $Et_2O$. Separate the aqueous layer and extract it with 250 mL of $Et_2O$. Combine the organic extracts, and wash them with water and then brine. Dry the organic extract over anhydrous $K_2CO_3$, and add 10 g of charcoal thereto. Filter, and evaporate the filtrate to leave 31.3 g of the title compound as a straw-colored oil.

EXAMPLE 1d

(S)-(-)-[2-[2-(2,4-Difluorophenyl)]oxiranyl]methanol

Add 33g of activated 3Å molecular sieve powder to a solution of 13g of L-(+)-diethyl tartarate in 2.3L of $CH_2Cl_2$, and cool the so-formed mixture to -5°C. Add a solution of 15.4 mL of titanium tetra-isopropoxide in 100 mL of $CH_2Cl_2$ over 2-3 minutes and then cool the so-formed mixture to -22°C. Add 109.5 mL of a 5.5 M solution of tert-butylhydroperoxide in 2,2,4-trimethylpentane over 4-6 minutes, and cool the so-formed mixture to -25°C. Stir the mixture at -25°C for 25 minutes and then add a solution of 40g of 2-(2,4-difluorophenyl)-3-propenol of Example 1c in 100 mL of $CH_2Cl_2$ over 3-4 minutes. Stir the so-formed mixture at -27°C for 4 1/2 hour. Add 102 mL of 30% aqueous sodium hydroxide saturated with NaCl and stir the so-formed mixture while warming to +10°C over a 1/2 hour period. Add thereto 100 g of anhydrous $MgSO_4$ and 33g of Celite, and stir 1/2 hour at +10°C. Suction-filter the mixture, wash the so-formed filter

cake with 1.2 L of diethyl ether ($Et_2O$) and then 1.5L of toluene, and dry the combined organic layers over anhydrous $MgSO_4$. Filter the organic layer, and evaporate the filtrate in vacuo to form a residue. Dissolve the residue in 1 L of $Et_2O$ and suction-filter the mixture to remove insolubles. Suction-filter the filtrate through 100g of silica gel, and wash the pad with 200 mL of fresh $Et_2O$. Evaporate the filtrate in vacuo to give 41g (94%) of the crude title compound as a yellowish oil, $[\alpha]_D^{25}$ - 36.7° (c=l, MeOH); PMR ($CDCl_3$) d 7.40(m,1H), 6.85(m, 2H), 3.95(m,2H), 3.31(d,1H), 2.84 (d,1H), 1.91(m,1H, deuterium exchangeable).

EXAMPLE 2

(R)-(+)-[2-[2-(2,4-Difluorophenyl)]oxiranyl]methanol

Follow the procedure of Example 1d, except substitute an equivalent amount of D-(-) diethyl tartarate in place of L-(+) diethyl tartarate to give the crude title compound, $[\alpha]_D^{25}$ + 33.9° (c=l, MeOH).

Purify a portion of the crude compound by silica gel chromatography to obtain a sample homogeneous by TLC, $[\alpha]_D^{25}$ + 40.0° (c=l, MeOH)

EXAMPLE 3

(R)-(-)-2-(2,4-Difluorophenyl)-3-(1,2,4-triazol-1-yl)-1,2-propanediol

Dissolve 8.91g of 1H-1,2,4-triazole in 150 mL of anhydrous DMF and cool so-formed mixture to 0-5°C. Add 2.81g of sodium hydride (60% oil dispersion) and stir the so-formed mixture 30 minutes at room temperature. Add thereto 10.9 g of the product of Example 1d. Stir the so-formed reaction mixture for 2 hours at 60-70°C. Cool the mixture to room temperature, add thereto 10 ml of $H_2O$ and evaporate it in vacuo to give a residue. Dissolve the residue in 100 mL of $H_2O$ and 900 ml of ethyl acetate (EtOAc). Extract the $H_2O$ layer with another 250 mL of EtOAc. Wash the combined EtOAc extracts with 100 mL of brine. Dry the EtOAc extracts over anhydrous $MgSO_4$ and evaporate. Triturate the so-formed oily residue with 10 mL of $CH_2Cl_2$ and add 100 mL of $Et_2O$. Stir the $CH_2Cl_2$-$Et_2O$ mixture for 1 hour at room temperature. Filter to give 11.2g (75%) of the title compound, $[\alpha]_D^{25}$- 70.7 (c=1.0, MeOH), mass spectrum (FAB): 256 $[M+H]^+$. Recrystallize 1.0g of the filtered product from 5 mL of $CH_3CN$ to give 0.83g of the title compound, m.p. 99-100°C; $[\alpha]_D^{25}$ - 71.5° (c=1.0, MeOH); elemental analysis: Calculated for $C_{11}H_{11}F_2N_3O_2 1/2CH_3CN$; 52.27C, 4.57H, 17.78N, 13.78F; Found: 52.26C, 4.58H, 17.54N, 13.78F; PMR(DMSO) d 8.25 (s,1), 7.66(s,1), 7.33, (m,1), 7.09(t,1), 6.90(t,1), 5.72(s,1), 5.05(t,1), 4.53(s,2), 3.61 (m,2).

EXAMPLE 4

(S)-(+)-2-(2,4-Difluorophenyl)-3-(1,2,4-triazol-1-yl)-1,2-propanediol

Follow the procedure of Example 3, except substitute an equivalent quantity of the product of Example 2 in place of the product of Example 1 to give the title compound; MP. 95-101°C; $[\alpha]_D^{25}$ + 70.0° (c=1.0, MeOH). The PMR and Mass spectra were consistent with the structure of the title compound.

EXAMPLE 5

(R)-2-(2,4-Difluorophenyl)-3-(1,2,4-triazol-1-yl)-1,2propanediol-1-methanesulfonate

Suspend 10.9 g of the powdered product of Example 3 in 150 mL of $CH_2Cl_2$. Add thereto 8.95 mL of triethylamine and cool to the so-formed mixture 0-5°C. Add 3.64 mL of methanesulfonyl chloride in 20 ml of $CH_2Cl_2$ over 10 min. Stir the so-formed mixture for 1 hour at room temperature. Cool it to 0-5°C, extract with 100 mL of cold (0-5°C) 5% $KH_2PO_4$, followed by 100 mL of cold (0-5°C) $H_2O$, followed by 50 mL of brine. Dry the separated organic layer over anhydrous $MgSO_4$ and evaporate to obtain 13.7 g (96%) of the title $[M+H+]^+$; PMR ($CDCl_3$) d 7.95 (s,1), 7.82 (s, 1), 7.53(m,1), 6.81(m,2), 4.84(d,1), 4.65(d,1), 4.46(m,2), 3.05(s,3).

EXAMPLE 6

(S)-2-(2,4-Difluorophenyl)-3-(1,2,4-triazol-1-yl)-1,2-propanediol-1-methanesulfonate

Follow the procedure of Example 5, except substitute an equivalent quantity of the product of Example 4 in place of the product of Example 3 to give the title compound . The PMR is consistent with the structure of the title

compound.

EXAMPLE 7

(R)-1-[2-[2-[2,4-Difluorophenyl)]oxiranylmethyl]-1,2,4-triazole

Dissolve 13.7g of the product of Example 5 in 200 mL of anhydrous DMF and cool the so-formed solution to 10-15°C. Add thereto 1.71g of sodium hydride (60% oil dispersion) and stir the so-formed reaction mixture at room temperature for 90 minutes. Concentrate in vacuo to 50 mL. Add thereto 200 mL of cold $H_2O$ (0-5°C) and extract with 3 x200 mL portions of EtOAc. Wash the combined EtOAc extracts with 100 mL of brine. Dry the EtOAc extracts over anhydrous $MgSO_4$ and evaporate it to give 10.8 g of a residue. Apply the residue in $CH_2Cl_2$ to a column of 400 g of MPLC grade silican gel previously prepared by slurry packing with $CH_2Cl_2$ containing 1 mL of $Et_3N$ per liter. Elute with 1 liter, each of 25, 50 and 75% EtOAc; $CH_2Cl_2$ (v/v) followed by 2 liters of EtOAc. Combine the fractions to give 6.92g (68%) of the title compound. Mass spectrum (FAB): 238 $[M+H]^+$; PMR ($CDCl_3$) d 7.97(s,1), 7.77(s,1), 7.07(m,1), 6.73 (m,2); 4.73(d,1), 4.41(d,1), 2.84(d,1), 2.78(d,1).

EXAMPLE 8

(S)-1-[2-[2-(2,4-difluorophenyl)]oxiranylmethyl]-1,2,4-triazole

Follow the procedure of Example 7, except substitute an equivalent amount of the product of Example 6 in place of the product of Example 5 to give the title compound. [PMR is consistent with the structure of the title compound].

EXAMPLE 9

Ethyl(5R-cis)-, and (5R-trans)-5-(2,4-Difluorophenyl)-2-oxo-5-[(1H-1,2,4-triazol-1-yl)methyl]tetrahydro-3-furancarboxylate

Dissolve 9.35 mL of diethyl malonate in 70 mL of anhydrous DMSO. Add 2.24g of sodium hydride (60% oil dispersion) in 2 portions and stir the so-formed reaction mixture at room temperature for 1 hour. Add 6.65 g of the product of Example 7 and stir 18 hours at 50-55°C. Cool to room temperature and pour the reaction mixture into a well-stirred mixture of 500 mL of $KH_2PO_4$, 500 mL of brine, and 1 liter of EtOAc. Separate and extract the $H_2O$ layer with another 300 mL of EtOAc. Wash the combined EtOAc extracts with 500 mL of brine, Dry the EtOAc extracts over anhydrous $MgSO_4$ and evaporate to give an oil. Apply the oil with $CH_2Cl_2$ to a column of 400 g MPLC grade silica gel prepared with hexane. Elute with 500 mL of hexane, followed by 2 liters of 50% EtOAc: hexane (v/v), followed by 2 liters of EtOAc. Combine fractions to give 8.66g (80%) of the title compound. Mass spectrum (FAB): 352$[M+H]^+$, PMR ($CDCl_3$) d 8.08(s,2), 7.91(s,1), 7.71(s,1), 7.42(m,1), 7.13(m,1), 7.85(m,2), 4.60(m,4), 4.10(m,4), 3.49(t,1), 3.14(t,1), 3.89(m,4), 1.18(m,6).

EXAMPLE 10

Ethyl(5S-cis), and (5S-trans)-5-(2,4-Difluorophenyl)-2-oxo-5-(1H-1,2,4-triazol-1-yl)methyl]tetrahydro-3-furancarboxylate

Follow the procedure of Example 9, except substitute an equivalent amount of the product of Example 8 in place of the product of Example 7 to give the title compound. [PMR and mass spectra are consistent with the structure of the title compound].

EXAMPLE 11

(R)-(-)-4-(2,4-Difluorophenyl)-2-hydroxymethyl-5-[1H-(1,2,4-triazol-1-yl)]-1,4-pentanediol

Dissolve 8.5 g of the product of Example 9 in 125 mL of EtOH and add 2.15 g of LiCl. Cool the stirred mixture to 0°C and add 1.92 g of $NaBH_4$ in portions. Stir the mixture for 18 hr without further cooling. Add 125 mL of MeOH and 10 mL of $H_2O$ to the mixture and stir for 4 hr. Evaporate the mixture to dryness and extract the precipitate with warm EtOH. Evaporate the extract to dryness, add 200 mL of THF to the residue, and sonicate the stirred mixture for 15 min. Filter the mixture and evaporate the filtrate. Chromatograph the residue on silica gel, eluting with $CH_2Cl_2$-MeOH-$NH_4OH$ (95:5:1) v/v/v) to obtain 3.9 g of the title compound. Mass spectrum (FAB): 314 (M+H+); PMR (DMSO)

d 8.25(s,1), 7.69(s,1), 7.35(m,1), 7.13(m,1), 6.94(m,1), 6.27(s,1), 5.16(t,1), 4.44(m,4), 3.39(m,1), 3.20(m,1), 3.05(t,2), 2.11(m,1), 1.52(m,1).

EXAMPLE 12

(S)-(+)-4-(2,4-Difluorophenyl)-2-hydroxymethyl-5-[1H-(1,2,4-triazolyl)]-1,4-pentanediol

Follow the procedure of Example 11, except substitute an equivalent amount of the product of Example 10 in place of the product of Example 9 to give the title compound. Chromatograph a portion of the crude product on silica gel eluting with $CH_2Cl_2$-MeOH-$NH_4OH$ to give a product homogeneous by TLC. Dissolve the material in $H_2O$ and filter, and lyophilize the filtrate to give the title compound. $[\alpha]^{25}_{D}$ +54.50 (c=1.0, MeOH)

EXAMPLE 13

(R)-(-)-4-(2,4-Difluorophenyl)-2-[[(4-methylphenyl)-sulfonyloxy]methyl]-5-[1H-(1,2,4-triazolyl)]-1,4-pentanediol-1-(4-methylbenzene)sulfonate

Dissolve 4.4g of the product of Example 11 in 50 mL of $CH_2Cl_2$-THF (1:1, v/v). Add 4.7 mL of $Et_3N$ and 180 mg of N,N-dimethylaminopyridine, and cool the solution to 0°C. Add thereto 5.9 g of p-toluenesulfonyl chloride in portions and stir the so-formed reaction mixture at 0°C for 1/2 hour, and then stir it at room temperature for 5 hours. Add 100 mL of EtOAc and suction-filter the mixture. Concentrate the filtrate; add thereto 150 mL of EtOAc, and wash with 5% aqueous $KH_2PO_4$. Wash the organic layer with cold aqueous 5% $NaHCO_3$, then with saturated brine, and then dry it over anhydrous $MgSO_4$. Filter the mixture, and evaporate the filtrate. Chromatograph the residue on silica gel, eluting with EtOAC-hexane to give 6.4 g (73%) of the title compound, PMR ($CDCl_3$) d 7.95(s,1), 7.67(m,5), 7.30(m,6) 6.70(t,2), 4.74(d,1), 4.53(d,1), 4.13(m,1), 3.97(m,1), 3.8(m,2), 2.43(s,6), 1.95(m,2), 1.77(m,1). Mass spectrum (FAB): $622[M+H]^+$.

EXAMPLE 14

(S)-(+)-4-(2,4-Difluorophenyl)-2-[[(4-methylphenyl)-sulfonyloxy]methyl]-5-[1H-(1,2,4-triazolyl)]-1,4-pentanediol-1 (4-methylbenzene)sulfonate

Follow the procedure of Example 13 except substitute an equivalent amount of the product of Example 12 in place of the product of Example 11 to obtain the title compound, $[\alpha]^{25}_{D}$ +14.2° (c=1, MeOH).

EXAMPLE 15

(-)-(5R-cis)-5-(2,4-Difluorophenyl)-5-[(1H-1,2,4-triazol-1-yl)methyl]-tetrahydro-3-furanmethanol,4-toluenesulphonate

Dissolve 6.3g of the product of Example 13 in 150 mL of toluene and heat the so-formed solution to 100°C. Add 2.4g of 60% NaH dispersion in oil portionwise, and then heat the so-formed reaction mixture at reflux until cyclization is complete (approx. 3-4 hours). Cool the mixture and decant the solution from excess NaH. Wash the solution with cold 5% aqueous $KH_2PO_4$. Evaporate the organic layer to form a residue and chromatograph the residue on silica gel, eluting with acetone-hexane to obtain 1.6g (35%) of the title compound as the less polar of the two products, $[\alpha]^{25}_{D}$ - 39.4°(c=1, $CHCl_3$); PMR ($CDCl_3$) d 8.09 (s,1), 7.88 (m,3), 7.31 (m,3), 6.81 (m,2), 4.52(ABq,2), 3.99(m,1), 3.85(m,1), 3.70(m,1), 3.59(m,1), 2.49(m,2), 2.47(s,3), 1.90(m,1). Mass spectrum (FAB): 450 $[M+H]^+$.

EXAMPLE 16

(+)-(5S-cis)-5-(2,4-Difluorophenyl)-5-[(1H-1,2,4-triazol-1-yl) methyl]-tetrahydro-3-furanmethanol,4-toluenesulphonate

Follow the procedure of Example 15, except substitute an equivalent amount of the product of Example 14 in place of the product of Example 13 to give the title compound, $[\alpha]^{25}_{D}$ + 40.3° (c=0.3, CHCl3), mp 96-98°C.

EXAMPLE 17

(-)-[(2R)-cis]-4-[4-[4-[4-[[5-(2,4-difluorphenyl)-tetrahydro-5-(1H-1,2,4-triazol-1-ylmethyl)furan-3-yl]methoxy]pheynyl-1-piperazinyl]phenyl-2,4-dihydro-3H-1,2,4-triazol-3-one.

The title compound is prepared starting with the tosylate of Example 15 and 4-[4-(4-nitrophenyl)-1-piperazinyl] phenol (Example 3a of USP 4,791,111) and using the synthetic scheme outlined in Scheme V and J. Heeres, et al., J. Med. Chem 1984, Vol 27, p894-900 at 898 and 900.

EXAMPLE 18

(-)-[(2R)-cis]-4-[4-[4-[4-[[5-(2,4-Difluorophenyl)-Tetrahydro-5-(1H-1,2,4-Triazol-1-ylmethyl)-3-Furanyl]Methoxy] Phenyl]-1-Piperazinyl]Phenyl]-2,4-Dihydro-2-[1(S)-Methyl-2(R)-Hydroxypropyl]-3H-1,2,4-Triazol-3-One.

a. 2-O-SEM Ether of (2R,3R)-2,3-Butanediol

To a stirred solution of 4.95g of (2R, 3R)-2,3-butanediol, (55 mmoles) and 9.3g of SEM-Cl (55.7 mmoles) in 55 ml of anhydrous DMF at O°C were added in four portions 2.34g of 60% oil-dispersed NaH (58.5 mmoles) over 10 min. The resulting mixture was stirred at 0°C for 4 hours and at ambient temperature overnight. The turbid reaction mixture was poured onto 0.5L of 5% $KH_2$ $PO_4$ solution and extracted with 2 x 300 ml of ether; the combined ethereal solution was washed once with distilled water, saturated brine, dried over $MgSO_4$ and evaporated to give a colorless liquid. Flash chromatography over 350g silica gel with 1 L of 7% ETOAC/Hexane, 2L of 10% ETOAC/Hexane and 1L of 15% ETOAC/Hexane gave 1.74g of the title compound (yield 14.4%)
MS:$(M+H)^+$=221.

b. Brosylation

A mixture of 0.7g of the 2-0-SEM ether of Example 18(a), (3.18 mmoles) and 0.97g of 4-bromobenzenesulfonyl chloride (3.82 mmoles) in 5ml of anhydrous pyridine was stirred under $N_2$ atmosphere at ambient temperature for 6 hours. The reddish slurry reaction mixture was diluted with 50ml of ice-cold water, extracted with 2 x 25ml of ether. The combined ethereal solution was washed with 2 x 25ml of 1-% $CuSO_4$ solution, distilled water, saturated brine, dried over $MgSO_4$ and evaporated to give a reddish oily residue. Flash chromatography over 50g silica gel with 1L of 10% ETOAC/Hexane gave 1.02g of the brosylate as a colorless liquid (yield 72.9%)
$[\alpha]_D^{23}$= -3.69° ($CHCl_3$ ; c=1)

c. Alkylation Reaction

A mixture of 0.98g of the brosylate of Example 18(b) (2.23 mmoles), 0.69g of the 3H-1,2,4-triazol-3-one of Example 17(1.12 mmoles) and 0.37g of cesium carbonate (1.12 mmoles) in 20 ml of anhydrous DMF was stirred at 80°C under $N_2$ overnight (~20 hours). The reaction mixture was diluted with 100ml of ice-cold water, extracted with 2 x 50 ml of ethyl acetate. The combined organic solution was washed once with distilled water, saturated brine, dried over $MgSO_4$ and evaporated to give a brown solid residue. Flash chromatography of the residue over 125g silica gel with 1.2L of 80% ETOAC/Hexane gave 0.327g of the product as a tan solid (yield 35.7%)
MS=$(M+H)^+$=81.7.

d. Acidic Hydrolysis of 18(c) to the title product

A mixture of 0.32g of the SEM-ether of Example 18(c) and 6ml of 6N HCl solution in 6ml of methanol was stirred at ambient temperature for 4 hours and was evaporated under reduced pressure. The residue was diluted with 5ml of ice water, carefully basified with 10% $Na_2CO_3$ solution until pH=8-9 was obtained. Extraction of the so-formed reaction mixture with 2 x25ml of $CH_2$ $Cl_2$ followed by washing with saturated brine, drying over $MgSO_4$ and evaporation gave a tan solid. Filtration of the tan solid through a 50g silica gel column and elution with 0.75L of 4% MeOH/$CH_2Cl_2$ gave 0.26g of title product as a tan solid, yield 96.6%. MS=$(M+H)^+$=687;
$[\alpha]_D^{23}$= -23.65° ($CHCl_3$ ; c=1)

Example 19

(-)-[(2R)-cis]-4-[4-[4-[4-[[(5-(2,4-Difluorophenyl)-Tetrahydro-5-(1H-1,2,4-Triazol-1-ylmethyl)-3-Furanyl]Methoxy] Phenyl]-1-Piperazinyl]Phenyl]-2,4-Dihydro-2-1[1(R)-Methyl-2(R)-Hydroxypropyl]-3H-1,2,4-Triazol-3-one.

a. Mitsunobu Reaction

To a stirred solution of 0.72g of the 2-O-SEM ether of Example 18(a) (3.27 mmoles), 2.1g of triphenyl phosphine (8.06g) and 1.2g of p-nitrobenzoic acid (7.17 mmoles) in 30ml of dry benzene at 0°C were added, dropwise, 1.25ml (8.06 mmoles) of diethyl azodicarboxylate ("DEAD"). The so-formed clear yellow solution became turbid and the mixture was stirred at ambient temperature for 2 hours, and mixture loaded on a 100g silica gel column. Elution of the column with 15% ETOAC/Hexane gave 1.5g of the 3-β-nitrobenzoate having the S absolute configuration (95% yield) MS: 219 ($M^+$- 150), 252 ($M^+$ - 117).

b. Basic Hydrolysis of the p-Nitrobenzoate

A solution of 1.12g of of the p-nitrobenzoate of Example 19(a) (3 mmoles) and 3.5ml of 1 N NaOH solution in 20ml of methanol was stirred at ambient temperature for 3 hours. Solvents were evaporated and the residue was diluted with 10ml of distilled water, and extracted with 2 x 20ml of ether. The combined ethereal solution was washed once with saturated brine, dried over $MgSO_4$ and evaporated to give 0.67g of the corresponding alcohol as a colorless liquid (~100%), which was used directly for the next reaction without further purification.

c. Brosylation, Akylation and Acidic Hydrolysis

Following the procedures of Example 18(c) and (d), the title compound was prepared in 32% overall yield in 3 steps from the products of Example 19(b) and of Example 17. MS: $[M+H]^+$=687;
$[\alpha]_D^{23}$= -23.65° ($CHCl_3$ ; c=1)

Example 20

(-)-[(2R)-cis]-4-[4-[4-[4-[[(5-(2,4-Difluorophenyl)-Tetrahydro-5-(1H-1,2,4-Triazol-1-ylmethyl)-3-Furanyl]Methoxy] Phenyl]-1-Piperazinyl]Phenyl]-2,4-Dihydro-2-[(S)-1-Methyl-3-Hydroxypropyl]-3H-1,2,4-Triazol-3-one.

a. Formation of TBDPS Ether

To a solution of 0.9g or (R)-(-)-1,3-butanediol (10 mmoles), 1.5g of imidazole (22 mmoles) in 10 ml of anhydrous DMF at 0°C were added 3ml of t-butylchlorodiphenylsilane ("TBDPS") (11 mmoles) over 3 minutes. The reaction mixture was stirred at 0°C for 4 hours, diluted with 50ml of ice-cold water and extracted with 2 x 30ml of ether. The aqueous phase was back extracted with 50ml of ether and the combined ethereal solution was washed once with saturated brine, dried over $MgSO_4$ and evaporated to give a colorless residue. Flash chromatography over 150g silica gel with 1.5L of 5% EtOAC/Hexane and 1L of 10% EtOAC/Hexane gave 2.87g of the TBDPS ether (87.5%) MS: $[M+H]^+$: 329;
$[\alpha]_D^{23}$ = +0.64° ($CHCl_3$ ; c=1)

b. Brosylation

To a solution of 0.984g of TBDPS ether of Example 20(a) (3 mmoles) in 7ml of anhydrous pyridine were added 0.845g of 4-bromobenzenesulfonyl chloride (3.3 mmoles). The reaction was run and worked-up and purified in accordance with the procedure of Example 18(b) and 1.02g of the brosylate was obtained in 61.1% yield; MS: $[M+23]^+$ = 569/571;
$[\alpha]_D^{23}$ = +2.45° ($CHCl_3$ ; c=1)

c., Alkylation

The brosylate of Example 20(b), 0.95g (1.74 mmoles) was reacted with the compound of Example 17 according to the procedure of Example 18(c) to provide 0.49g of corresponding alkylated product, yield 60.3% MS: $(M+H)^+$ 925 $[\alpha]_D^{23}$ = -32.27° ($CHCl_3$; c=1)

d. Acidic Hydrolysis

The compound of Example 20(c), 0.32g, (0.35 mmoels) was hydrolyzed by 6N HCI solution in accordance with the procedure of Example 18(d) to give 0.22g of the title compound (yield 92.4%); MS: $M^+$ = 686; $[M+Na]^+$ = 709; $[\alpha]_D^{23}$ = -38.52° ($CHCl_3$ ; c=1)

Alternatively a solution of 0.19g of the compound of Example 20(c) and 60mg of tetrabutylammonium fluoride (0.23 mmoles) in 5ml of THF was stirred at ambient temperature for 24 hours. The brown solution was concentrated to a syrup. Flash chromatography of the syrup over 50g silica gel with 0.5L each of 2% and 4% $MeOH/CH_2Cl_2$. gave 0.11g of the title compound (yield 88.7%).

Example 21

(-)-[(2R)-cis]-4-[4-[4-[4-[[5-(2,4-Difluorophenyl)-Tetrahydro-5-(1H-1,2,4-Triazol-1-Ylmethyl)-3-Furanyl]Methoxy] Phenyl]-1-Piperazinyl]Phenyl]-2,4-Dihydro-2-[(R)-1-Methyl-3-Hydroxypropyl]-3H-1,2,4-Triazol-3-one.

The procedures of Example 20 were followed except an equivalent amount of S-(+)-1,3-butanediol was substituted for the corresponding R enantiomer. An overall 31.8% yield of the title compound was obtained in four steps; MS=$[M+H]^+$ = 687.

Example 22

(-)-[(2R)-cis]-4-[4-[4-[4-[[5-(2,4-Difluorophenyl)-Tetrahydro-5-(1H-1,2,4-Triazol-1-ylmethyl)-3-Furanyl]methoxy] Phenyl]-1-Piperazinyl]Phenyl]-2,4-Dihydro-2-[1(S)-Methyl-2-Hydroxypropyl]-3H-1,2,4-Triazol-3-one.

a. Benzylation

To a solution of 10g of (2R, 3R)-(-)-2,3-butanediol (111 mmoles) in 40ml of anhydrous $CH_2Cl_2$ and 80ml of cyclohexane at 0°C were added 1 ml of trifluoromethanesulfonic acid (TfOH), followed by dropwise addition of 21ml of benzyl trichloroacetimidate (113 mmoles). The resulting slurry was stirred at ambient temperature overnight, diluted with 125ml of hexane and filtered. The combined filtrate was concentrated to a yellow syrup. Flash chromatography of the yellow syrup over 250g silica gel with 1.5L of 7% ETOAC/Hexane, 2L of 15% ETOAC/Hexane and 2L of 25% ETOAC/Hexane, 1.5L of 10% $MeOH/CH_2Cl_2$ gave 11.88g of the 2-monobenzyl ether of the starting material (74.5% yield) and 2.03g of unreacted starting material MS: $[M+H]^+$: 181.

b. Mitsunobu Reaction

The 2-monobenzyl ether of Example 22(a), 5.4g, was converted into 6.6g of the 3- benzoate ester (yield 66.9%) by Mitsunobu reaction in accordance with the procedure of Example 19(a); MS: $[M+H]^+$= 330.

c. Alkaline Hydrolysis

The 5.3g of the product of Example 22(b) was subjected to alkaline hydrolysis according to the procedure of Example 19(b) to give 2.33g of the 2-monobenzyl ether of (2R,3S)-2,3-butanediol (yield 80.3%) $(M+H)^+$ = 181; $[\alpha]_D^{23}$ = -23.75° ($CHCl_3$ ; c=1)

d. Formation of the SEM Ether

To a stirred solution of 3.14g of the product of Example 22(c) (17.44 mmoles) and 3,8ml of di-isopropylethylamine (2.82g, 21.8 mmoles) in 30ml of anhydrous $CH_2Cl_2$ at ambient temperature were added 3.8ml of SEM-Cl (3.64g, 21.8 mmoles) in one portion. Fuming formed and the resulting yellow solution was stirred for 20 hours. The orange-colored reaction mixture was evaporated under reduced pressure and the solid residues were partitioned between ether and water. The ethereal solution was washed once with distilled water, saturated brine, dried over mg 504 and concentrated to give the crude product. Flash chromatography of the crude product over 200g silica gel with 2L of 3% ETOAC/Hexane gave 5.3g of the 3-O-SEM ether of the product of Example 22(c) (98% yield) as a colorless liquid; MS: $[M+H]^+$ = 311.

e. Hydrogenolysis

A mixture of 5.25g of the product of Example 22(d) (16.94 mmoles) and 0.5g of 10% Pd/C in 150ml of methanol was hydrogenated under atmospheric pressure for 6 hours. Catalysts were filtered and washed with additional methanol. The combined filtrate was concentrated to give a colorless liquid. Flash chromatography of the liquid over 100g silica gel with 2L of 10% ETOAC/hexane 3.53g of the free alcohol (yield 95%) as a colorless liquid; MS: 174, 103.

f. Brosylation

The product of Example 22(e) 1g was converted into 1.52g of the corresponding brosylate in 76.2%yield in accordance with the procedure of 18(b);
$[\alpha]_D^{23}$ = -1.53° ($CHCl_3$ ; c=1)

g. Alkylation Reaciton

The brosylate of Example 22(f), 1.48g of was reacted with the product of Example 17 to give 0.75g of the 2-alkylated triazol-3-one (yield 54.3%); $[\alpha]_D^{23}$ = -32.69° ($CHCl_3$ ; c=1)

h. Acidic Hydrolysis

Hydrolysis of 0.7g of the product of Example 22(g) in accorcdance with the procedure of Example 18(d) gave 0.51g of the title compound as a cream solid (yield 86.7%);
$[\alpha]_D^{23}$ = -32.69° ($CHCl_3$ ; c=1)

Example 23

(-)-[(2R)-cis]-4-[4-[4-[4-[[5-(2,4-Difluorophenyl)-Tetrahydro-5-1H-1,2,4-Triazol-1-ylmethyl)-3-Furanyl]Methoxy] Phenyl]-1-Piperazinyl]Phenyl]-2,4-Dihydro-2-[1(S)-Methyl-2(S)-Hydroxypropyl]-3H-1,2,4-Triazol-3-One.

a. Mitsunobu Reaction

The product of step e of Example 22 (1.99g, 9.05 mmoles) was reacted with p-nitrobenzoic acid in accordance with the procedure Example 19(a) to give 3.3g of product (yield 98.8%); MS = $[M+H]^+$ = 221.

b. Alkaline Hydrolysis

The product of step (a) of this Example (2.36g, 6.4 mmoles) was hydrolyzed by 7ml of 1N NaOAc to give 1.18g of the 3-O-SEM ether of (2S,3S)-2,3-butanediol (yield 83.7%). MS: $[M+H]^+$ = 221
$[\alpha]_D^{23}$ = +55.15° ($CHCl_3$ ; C=1)

c. Brosylate Formation

The product of step (b) of this Example (1.15g were converted into the brosylate in accordance with the procedure of Example 18(b) to give 3.47g of the brosylate (yield 97.7%).

d. Alkylation and Acidic Hydrolysis

The procedures of Example 18(c) and (d) were followed except the product of Example 23(c) was substituted for that of 18(b) to give the title compound.

Example 24

(2R-cis)-4-[4-[4-[4-[[-5-(2,4-difluorophenyl)-tetrahydro-5-(1H-1,2,4-triazol-1-ylmethyl)furan-3-yl]methoxy]phenyl]-1-piperazinyl]phenyl]2-4-dihydro-2-[(S)-1-ethyl-2(S)-hydroxypropyl]-3H-1,2,4-Triazol-3-One.

a. The methyl ester of (S)-lactic acid was converted into the corresponding benzyloxymethyl ether in accordance with the procedure of W. C. Still, et al. Tetrahedron Letters, 21, 1035-1038 (1980).

b. Reduction to the Aldehyde

DIBAL-H, 37.7ml of a 1M solution, was added dropwise to a stirred solution of 7.67g of the ester of step (a) of this Example in toluene at -78°C (dry ice/acetone bath) under an atmosphere of nitrogen. After 6 min. methanol (10ml) followed by an aqueous aolution of Rochelles salt were added. After warming to room temperature the moisture was partitioned between ETOAc and water. The organic phase was separated, washed with water, dried ($MgSO_4$) and concentrated to produce the crude aldehyde which was used in the next step without purification.

b. Grignard Step

The THF solution of 80ml of 1 molar solution of the ethyl magnesium bromide Grignard reagent was added dropwise to a stirred THF solution of the crude aldehyde obtained from step (b) of this Example at -78°C (dry ice/ acetone bath) under an atmosphere of nitrogen. After the addition was complete, the resulting mixture was allowed to warm slowly to room temperature overnight and stirred for a further period of 48 h. An aqueous solution of Rochelles salt was added and then the resulting mixture was partitioned between acetone and water. The organic phase was separated, washed with water, dried ($MgSO_4$) and concentrated. The residue was purified by column chromatography on silica gel using ETOAC/Hexane (1:10) as eluant to give

(i) non-polar alcohol (2S,3S) 2.31g;31%, as a colorless oil.
(ii) a mixture of both alcohols, 1.23g; 41% and
(iii) polar alcohol (2S,3R) 1.23g; 16%, as a colorless oil.

c. Brosylation of polar alcohol

4-Bromobenzenesulphonyl chloride (1.035g, 4.1 mmoles) was added to a stirred solution of (0.605g, 2.7 mmoles) the polar (2S, 3R) alcohol of step (b) of this Example and 2.20g (5.9 mmoles) of DMAP in $CH_2Cl_2$ at room temperature under an atmosphere of nitrogen. The resulting mixture was stirred for 12 h. and then partitioned between ETOAC and water. The organic phase was separated, washed with water, dried and concentrated. The residue was purified by column chromatography on silica gel using ETOAC/Hexane (1:10) as eluant to give the desired brosylate (85%) as a colorles oil.

d. Alkylation and acidic hydrolysis

The procedures of Example 18(c) and (d) were followed except the (2S, 3R) bosylate of step (c) of this Example was substituted for that used in Example 18(c). The acidic hydrolysis produced the title compound as a white solid, mp 170-172°C.

Example 25

(2R-cis)-4-[4-[4-[4-[[-5-(2,4-difluorphenyl)-tetrahydro-5-(1H-1,2,4-triazol-1-ylmethyl)furan-3-yl]methoxy]phenyl]-1-piperazinyl]phenyl]-2,4-dihydro-2-[(R)-1-ethyl-2(S)-hydroxypropyl]-3H-1,2,4-triazol-3-one.

The procedures of Example 24 were followed except the non-polar (2S,3S) alcohol from step (b) of Example 24 was converted into the (2S,3S)-3-brosylate. Alkylation of the brosylate followed by acidic hydrolysis of the SEM protecting group in accordance with the procedures of Example 24(d) provided the title compound.

Example 26

(2R-cis)-4-[4-[4-[4-[[-5-(2,4-difluorophenyl)-tetrahydro-5-(1H-1,2,4-triazol-1-ylmethyl)furan-3-yl]methoxy]phenyl] 1-piperazinyl]phenyl]2-4-dihydro-2-[(R)-1-ethyl-2(R-hydroxypropyl]-3H-1,2,4-triazol-3-One.

The procedures of Example 24 were followed except the methyl ester of (R) lactic ester was substituted for the methyl ester of (S)-lactic acid in step (a) of Example 24. The (2R, 3S) alcohol was used in steps (c) and (d) to provide the title compound.

Example 27

(2R-cis)-4-[4-[4-[4-[[-5-(2,4-difluorophenyl)-tetrahydro-5-(1H-1,2,4-triazol-1-ylmethyl)furan-3-yl]methoxy]phenyl] 1-piperazinyl]phenyl]2-4-dihydro-2-[(S)-1-ethyl-2(R)-hydroxypropyl]-3H-1,24-triazol-3-One.

The procedures of Example 26 were followed except the (2R, 3R) alcohol was used in steps (c) and (d) to provide the title compound.

Example 28

(2R-cis)4-[4-[4-[4-[[-5-(2,4-difluorophenyl)-tetrahydro-5-(1H-1,2,4-triazol-1-ylmethyl)furan-3-yl]methoxy]phenyl] 1-piperazinyl]phenyl]2-4-dihydro-2-[(R)-1-ethyl-3-hydroxyoropyl]-3H-1,2,4-triazol-3-One.

a. Reduction

To methyl (3R)-hydroxyvalerate (5.289, 40.0 mmoles) dissolved in 100ml of anhydrous THF at 0-5°C was added dropwise 60ml of a 1 M THF solution of $LiAlH_4$ (60 mmoles). The solution was allowed to warm to ambient temperature and to the so-formed mixture was added sequentially, 2.5 mL of water, dropwise, 2.5mL of 15% NaOH and 7.5mL of water. The so-formed reaction mixture was stirred at ambient temperature for 4 h. The inorganic solids were removed by filtration and the filtrate was evaporated to give 4.31g of (3R)-1,3-pentanediol.

b. 1-O-SEM ether formation

The procedure of Example 18(a) was followed except an equivalent quantity of the product of step (a) of this Example was substituted for the (2R, 3R)-2,3-butanediol to provde the title compound.

c. Mitsunobu Reaction

The procedure of Example 19(a) was followed except that an equivalent quantity of the product of step (b) of this Example was substituted for the 2-SEM ether of (2R,3R)-2,3-butanediol to give 3.34g of the corresponding p-nitrobenzoate.

d. Basic Hydrolysis

The procedure of Example 19(b) was followed except that an equivalent quantity of the p-nitrobenzoate ester of step (c) of this Example was used to provide 1.88g of the 1-O-SEM ether of (3S)-1,3-pentanediol.

e. Brosylation, Alkylation and Acid Hydrolysis

The procedures of Example 18 (b), (c), and (d) were followed except that an equivalent quantity of the product of step (d) of this Example was substituted for the corresponding 1-0-SEM ether of (2R, 3R) 2,3-butanediol used in Example 19(b) to produce 1.04g of the title compound of this Example $[\alpha]_D^{23}$ = -8.42° ($CHCl_3$ ; c=1)

Example 29

(2R-cis)-4-[4-[4-[4-[[-5-(2,4-difluorophenyl)-tetrahydro-5-(1H-1,2,4-triazol-1-ylmethyl)furan-3-yl]methoxy]phenyl] 1-piperazinyl]phenyl]2-4-dihydro-2-[(S)-1-ethyl-3-hydroxypropyl]-3H-1,2,4-triazol-3-One.

The procedures (a) and (b) of Example 28 were followed to produce the 1-O-SEM-(3R)-1,3-pentanediol which was converted directly into the 3R brosylate by following the procedures of Example 18(b). The 3R brosylate was used to alkylate the product of Example 17 in accordance with the procedures of Example 18(c). The so-formed product was subjected to acidic hydrolysis in accordance with the procedures of Example 18(d) to provide 368mg (90% yield) of the title compound;
$[\alpha]_D^{23}$ = -47.11° ($CHCl_3$ ; c=1)

Example 30

(2R-cis)-4-[4-[4-[4-[[-5-(2,4-difluorophenyl)-tetrahydro-5-(1H-1,2,4-triazol-1-ylmethyl)-3-furanyl]methoxy]phenyl] 1-piperazinyl]phenyl]2-4-dihydro-2-[1-hydroxy-(2R)-butyl]-3H-1,2,4-triazol-3-One.

a. Preparation of (2S)-1,2,-butanediol

A solution of (2S)-3-butene-1,2-diol which was purchased from Eastman Kodak, (3g, 0.034mmoles) in 40mL of ethanol was hydrogenated in the presence of 300mg of 10% Pd/C overnight. The so-formed reaction mixture was filtered through celite. The so-formed filter cake was washed with ethanol and the combined filtrates were evaporated to provide 2.08g (68% yield) of the title compound.

b. 1-O-SEM ether formation, brosylaton, alkylation and acidic hydrolysis

The procedures of Example 18(a) - (d) were followed except that an equivalent amount of the product of step (a) of this Example was substituted for the (2R, 3R) 2,3-butanediol of Example 18 to provide the title compound $[\alpha]_D^{23}$ = -24.3° ($CHCl_3$ ; c=1)

Example 31

(2R-cis)-4-[4-[4-[4-[[-5-(2,4-difluorophenyl)-tetrahydro-5-(1H-1,2,4-triazol-1-ylmethyl)-3-furanyl]methoxy]phenyl] 1-piperazinyl]phenyl]2-4-dihydro-2-[1-hydroxy-(2S)-butyl)-3H-1,2,4-triazol-3-one.

The procedures of Example 30 were followed except that an equivalent quantity of (2R)-3-butene-1,2-diol (available from Eastmand Kodak) was substituted for (2S)-3-butene-1,2-diol in step (a) of Example 30. The procedures of Example 30(b) were there after followed to produce the title compound
$[\alpha]_D^{23}$ = -29.4° ($CHCl_3$ ; c=1)

Example 32

(-)(2R-cis)-4-[4-[4[-5-(2,4-difluorophenyl)tetrahydro-5-(1H-1,2,4,-triazol-1-ylmethyl)furan-3-yl]methoxy]phenyl]-1-piperazinyl]pheny]-2,4-dihydro-2-[(S)-1-ethyl-2(S)-hydroxypropyl]-3H-1,2,4-triazol-3-one

a. (S)-2-(benzyloxy) propionaldehyde by selective reduction of (S)-(O-benzyl) lactic acid pyrrolidine amide: To a solution of the S-(O-benzyl)lactic acid pyrrolidene amide prepared in accordance with the procedure of Tetrahedron, 1989, vol. 45, pages 57-67 (5g, 0.0214 mol.) dissolved in 20 ml of toluene cooled to in a ice methanol bath was added slowly with stirring 4.25 ml or RED-AL (3.4M solution of sodium bis(2-methoxyethoxy) aluminum hydride) in toluene available from Aldrich Chemical Catalogue #19, 619-3). The solution was stirred fro 5 hrs., quenched with 2.5 ml of acetone and thereafter with 35 ml of 2NHCI. The so-formed mixture was extracted with EtoAc. The organic extracts were washed with water, $NaHCO_3$ and brine, dired over $Na_2SO_4$ and evaporated to give the titled product.

b. (S)-2-(Benzyloxy)-N-(Formylamino) propanimine. The propionaldehyde of step (a) (1g, 16.09 mml) was added dropwise to a solution of formic hydrazine (0.73g, 12.18 mmol) dissolved in 5 ml of methanol. The so-formed reaction mixture was stirred overnight. The solvent was removed by evaporation and the so-formed residue was stirred with ethyl ether. The undissolved excess formic hydrazine was removed by filtration and the ether was removed to provide a residue which was chromatographed on silica gel(/) using 20% EtoAc: hexane (v:v) to give 805 mg of the title product as a light yellow waxy solid having strong UV activity; ms $[M + H]^+$ = 207.

c. 2-[3-(2S, 3S)-2-(Benzyloxy)pentyl]formic acid hydrazide

Ethylmagnesium bromide (1.3 ml, 3.9 mmol, 3.0 molar in ethyl ether) was added to a stirred solution of 200 mg, 0.97 mmol of the propanimine of step (b) in 10 ml of ethyl ether at 0°C. The so-formed reaction mixture was stirred overnight at room temperature and quenched with water. The organic layer was separated and the solvent removed to provide a residue which was chromatographed on silica gel using 30 to 50% of EtoAc:hexane (v:v) to provide 113 mg; (50 % yield) of the title compound as an oil. The ratio of S,S isomer: S,R isomer in the product was 94:6. When the reaction was repeated in the presence of 1.2 equivalent of bis(trimethylsilyl) acetamide the S,S:S,R ratio improved to 99:1 MS: $[M + H]^+$ = 237

d. Cyclization Reaction

A solution of 156.3 mg, 0.66 mmol of the product of step (c) and 400 mg 0.60 mmol of 17F of Scheme V and 1 mole of DBU (1,8-diaza bicyclo [5.4.0]undec-7-ere) in volume was stirred at 80°C for six hours; the temperature was raised to 100° to 110°C and stirring was continued at this temperature overnight. The reaction mixture was allowed to cool to room temperature and the stirring was continued over the weekend. The solvent was removed by evaporation and the crude product was purified on preparative TLC (80% EtoAc) hexane, v:v) to provide 200 mg of the benzyl ether of the title product of this example as a foamy solid; MS:$[M + H]^+ = 792$ This cyclization reaction is the invention of Mergelsberg, Gala et. al. which is disclosed in commonly-owned U.S. Patent Application S.N. (attorney's Docket #CD0475)

e. Hydrogenolysis

To the solution of the benzyl ether (190 mgs , 0.24 mmol) of step d dissolved in 10 ml of methanol was added 40 mg of Pd black on carbon and 4 ml of formic acid. The reaction flask was sealed with a ballon and heated at 60°C for four hours. The catalyst was removed by filtration through a celite cake and the filtrate was poured into cold water. The pH of the so-formed solution was adjusted to a value of 4 to 5 with amonia. The so-formed mixture was extracted with EtoAc. The organic layer was separted and dried over $Na_2SO_4$. The solvent was removed to provide a crude product which was purified on preparative TLC (5% methanol: $CH_2CL_2$, v:v) to give 95 mg of the title compound of this example. (57% yield) as a tan solid. MS : $[M+ H]^+ = 701$. $[\alpha] = -28.4$ (c, = 1.0, $CHCl_3$)

**Claims**

1. A compound represented by the formula I

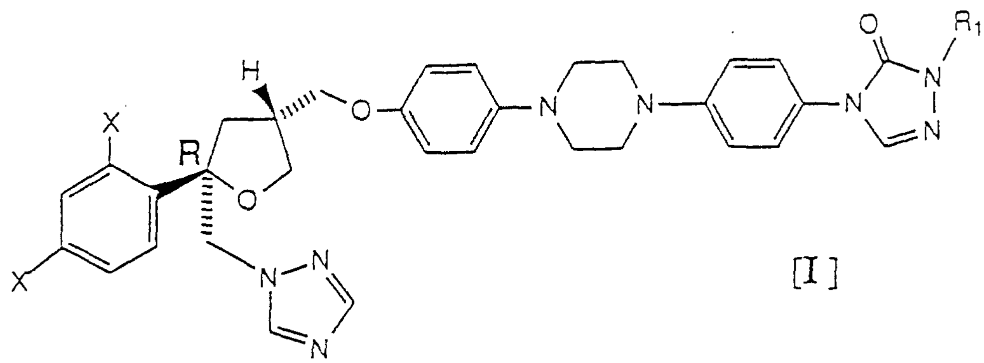

[I]

wherein

X is independently both F or both Cl or one X is independently F and the other is independently Cl;

$R_1$ is a straight or branched chain ($C_4$ to $C_5$) alkyl group substituted by one hydroxy moiety.

2. A compound of Claim 1 wherein $R_1$ is a hydroxy-substituted $C_4$- or $C_5$-alkyl group selected from:

$-\overset{*}{C}H(C_2H_5)\overset{*}{C}H(OH)CH_3$, $-\overset{*}{C}H(C_2H_5)CH_2CH_2OH$,
$-(CH_2)_2\overset{*}{C}H(OH)C_2H_5$, $-\overset{*}{C}H(CH_3)\overset{*}{C}H(OH)CH_3$,
$-\overset{*}{C}H(C_2H_5)CH_2OH$, $-\overset{*}{C}H(CH_3)CH_2CH_2OH$

wherein the carbons with the asterisk(*) have the R or S absolute configuration.

**3.** A compound represented by formula III

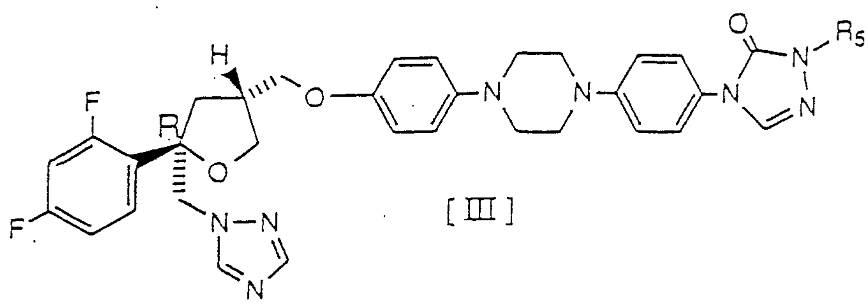

[III]

wherein $R_5$ is

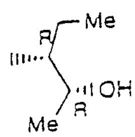 , 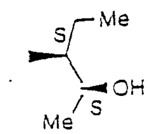 , 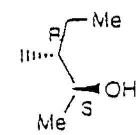 ,

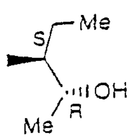 , 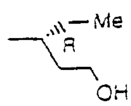 , 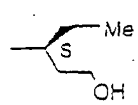 ,

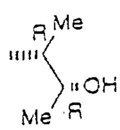 , 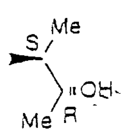 , 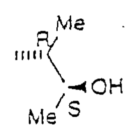 ,

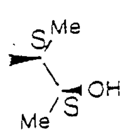 , 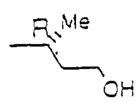 , 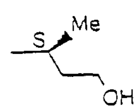 ,

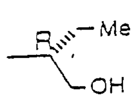 or 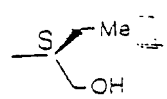

**4.** A compound represented by the formula IV

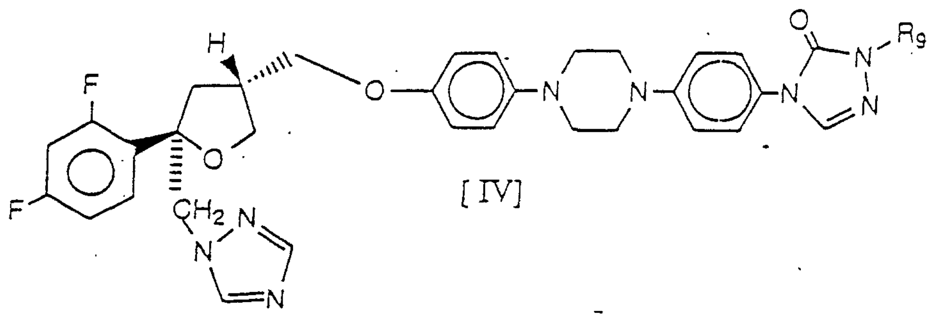

[IV]

wherein $R_9$ =

-$\overset{*}{C}H(C_2H_5)\overset{*}{C}H(OH)CH_3$ or -$\overset{*}{C}H(CH_3)\overset{*}{C}H(OH)CH_3$.

**5.** A compound of Claim 3 wherein $R_5$ is

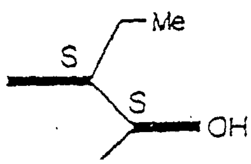

or

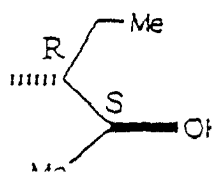

or

**6.** A compound of the formula

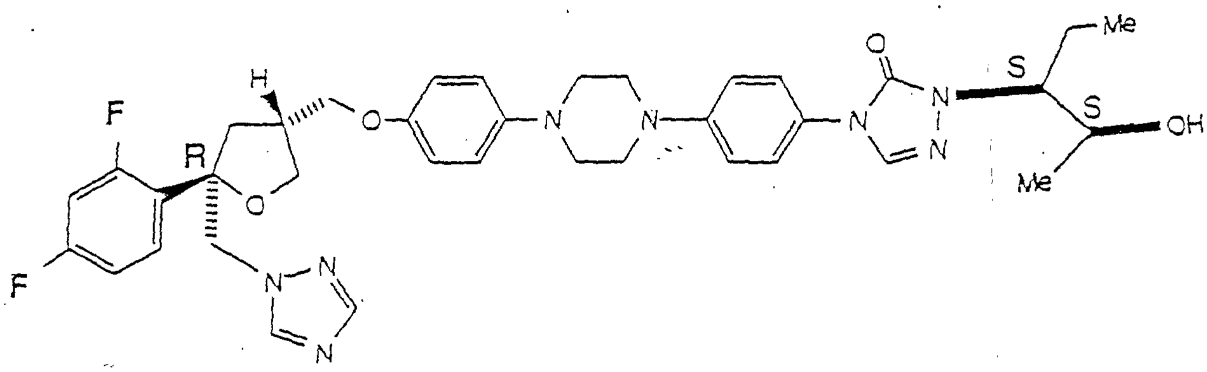

**7.** A pharmaceutically acceptable ester of a compound as claimed in any preceding claim.

**8.** A pharmaceutically acceptable ester of a compound as claimed in any of Claims 1 to 6 wherein the hydrogen of said one hydroxy moiety is replaced by a group of the formula

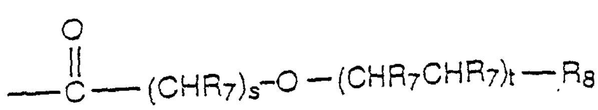

wherein $R_7$ is H or ($C_1$-$C_6$) straight or branched chain alkyl group; and s is an integer from 1 to 6; t is an integer from 1 to 6; $R^8$ is $R_7$ or $-(CHR_7)_S-CO_2R_7$.

**9.** A pharmaceutically acceptable ester of a compound as claimed in any of Claims 1 to 6 wherein the hydrogen of said one hydroxy moiety is replaced by a group of the formula

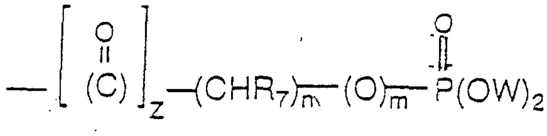

wherein z is 0 or 1; m is 0 or 1; $R_7$ is H or a ($C_1$-$C_6$) straight or branched chain alkyl group and preferably is H; n is an integer from 0 to 6 and W is H, $CH_2Ar$ or

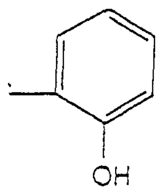

and wherein Ar is phenyl or phenyl substituted by halo, cyano, nitro or trihalomethyl.

**10.** A pharmaceutically acceptable ester of a compound as claimed in any preceding claim wherein the hydrogen of said one hydroxy moiety is replaced by $SO(OH)_2$.

**11.** A pharmaceutically acceptable ester of a compound as claimed in any of Claims 1 to 6 wherein the hydrogen of said one hydroxy moiety is replaced by a group of the formula

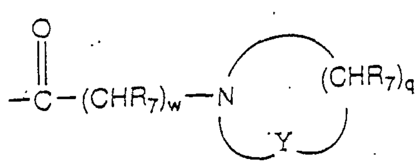

wherein $R_7$ is as defined herein above, W is an integer of from 1 to 5 preferably W is 1 to 3; q is = 3 or 4 and Y is $CHR_7$, -O-, NH, $NR_7$, S, SO or $SO_2$.

**12.** A pharmaceutically acceptable ester of a compound as claimed in any of Claims 1 to 6 wherein the hydrogen of said one hydroxy moiety is replaced by a straight or branched chain alkanoyl or alkanoyl group which is optionally substituted by a hydroxy or ether moiety.

**13.** A pharmaceutically acceptable ester of a compound as claimed in any of Claims 1 to 6 wherein said one hydroxy group is esterified with a natural amino acid residue, wherein amino groups are optionally protected by conventional protecting groups.

**14.** A pharmaceutically acceptable ester of a compound as claimed in any of Claims 1 to 6 wherein the hydrogen of said one hydroxy moiety is replaced by a group of the formula

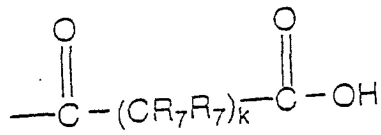

wherein $R_7$ is as defined herein above and k is an integer of from 1 to 8,

**15.** A pharmaceutically acceptable ester of a compound as claimed in any of Claims 1 to 6 wherein the hydrogen of said one hydroxy moiety is replaced by a group of the formula

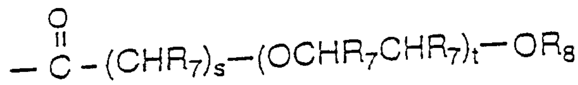

wherein $R_7$ is H or ($C_1$-$C_6$) straight or branched chain alkyl group, $R_8$ is $R_7$ or

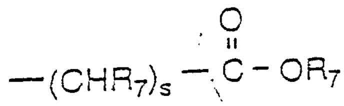

and s is 1 to 6 and t is 1 to 6.

**16.** A pharmaceutically acceptable ester of a compound as claimed in any of Claims 1 to 6 wherein the hydrogen of said one hydroxy moiety is replaced by a group of the formula

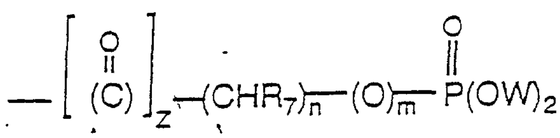

wherein z is 0 or 1, m is 0 or 1 n is 0 to 6, and $R_7$ is H or ($C_1$-$C_6$) straight or branched chain alkyl group and W is H or $CH_2Ar$ or

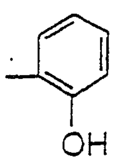

wherein Ar is phenyl or phenyl substituted by halo, cyano, nitro or trihalomethyl.

**17.** A pharmaceutically acceptable ester of a compound according to Claim 5 or Claim 6 wherein the hydrogen of the hydroxy moiety is replaced by

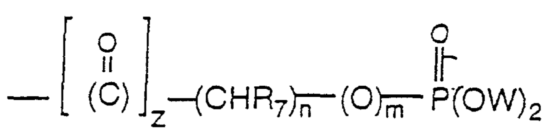

wherein z is 0 or 1; $R_7$ is H; m is 0 or 1, n is an integer from 0 to 6 and W is H.

**18.** A pharmaceutically acceptable ester of a compound according to Claim 5 or Claim 6 wherein the hydrogen of the hydroxy moiety is replaced by

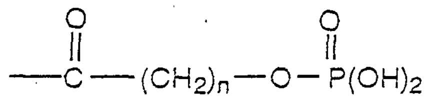

**19.** A compound of the formula

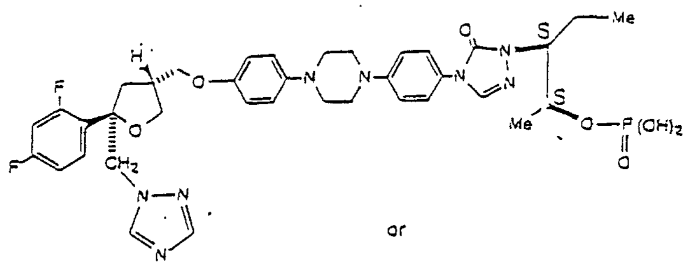

or

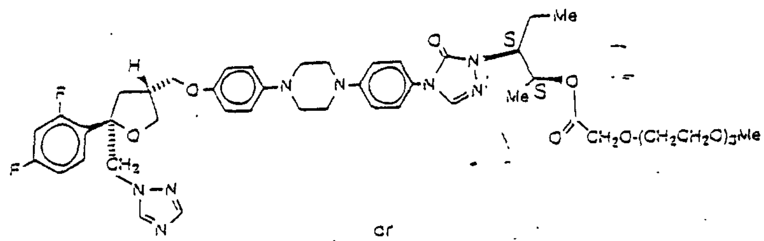

or

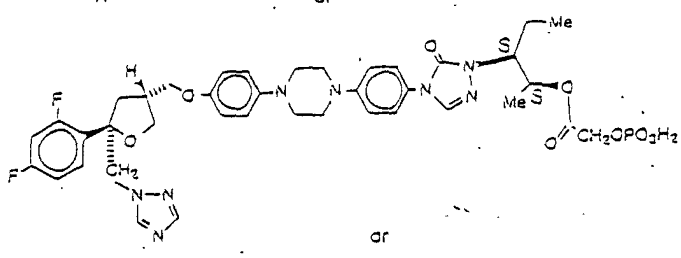

or

**20.** A pharmaceutically acceptable salt of a compound according to any preceding claim.

**21.** A pharmaceutical composition for treating or preventing fungal infection comprising an antifungally effective amount of a compound of any preceding claim together with a pharmaceutically acceptable carrier therefor.

**22.** The pharmaceutical composition of Claim 21 wherein the mode of administration is oral or parenteral.

**23.** A method of making compounds of the formula III of Claim 3 wherein $R_4$ is

$$\overset{*}{C}H(C_2H_5)\overset{*}{C}H(OH)CH_3$$

wherein the absolute stereochemistry at each asterisk carbon (*) is same i.e., S,S or R,R substantially free of S, R or R,S and wherein S or R-lactic acid ester is converted into the corresponding amide, which is selectively reduced to the corresponding aldehyde and then converted into the corresponding N-formyl amino propanimine which comprises reacting the N-formyl amino propanimine of the formula

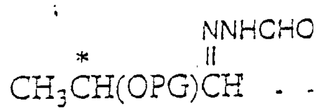

with ethyl magnesium bromide under Grignard reaction conditions sufficient to produce a compound of the formula

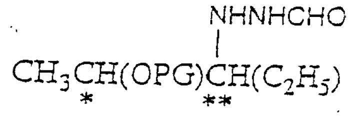

wherein the absolute stereochemistry induced at the doubt asterisk carbon (**) is substantially the same as that at the single asterisk carbon and wherein PG is a conventional hydroxy protecting group such as benzyl.

**24.** A method of Claim 23 wherein S-lactic acid methyl ester is used as the starting material and the Grignard reaction is conducted in the presence of more than one equivalent of bis(trimethylsilyl)acetamide.

**Patentansprüche**

**1.** Verbindung der Formel I

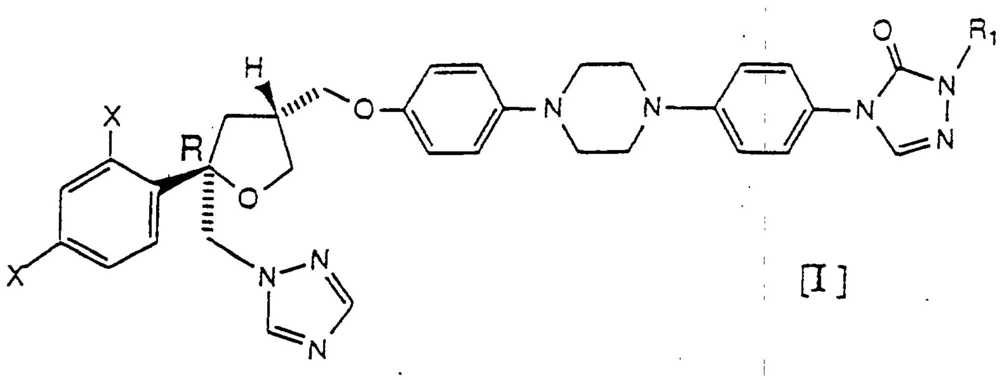

[I]

worin beide X unabhängig voneinander sowohl F als auch Cl sein können oder ein X unabhängig F und das andere unabhängig Cl ist;

$R_1$ eine mit einer Hydroxy-Komponente substituierte geradkettige oder verzweigte ($C_4$ bis $C_5$) Alkylgruppe ist.

**2.** Verbindung gemäß Anspruch 1, worin $R_1$ eine Hydroxy-substituierte $C_4$- oder $C_5$-Alkylgruppe ist, die aus:

$-\overset{*}{C}H(C_2H_5)\overset{*}{C}H(OH)CH_3$ , $-\overset{*}{C}H(C_2H_5)CH_2CH_2OH$ ,

$-(CH_2)_2\overset{*}{C}H(OH)C_2H_5$ , $-\overset{*}{C}H(CH_3)\overset{*}{C}H(OH)CH_3$ ,

$-\overset{*}{C}H(C_2H_5)CH_2OH$, $-\overset{*}{C}H(CH_3)CH_2CH_2OH$

ausgewählt wurde, worin die mit einem Sternchen (*) markierten Kohlenstoffe die absolute R oder S Konfiguration aufweisen.

**3.** Verbindung der Formel III

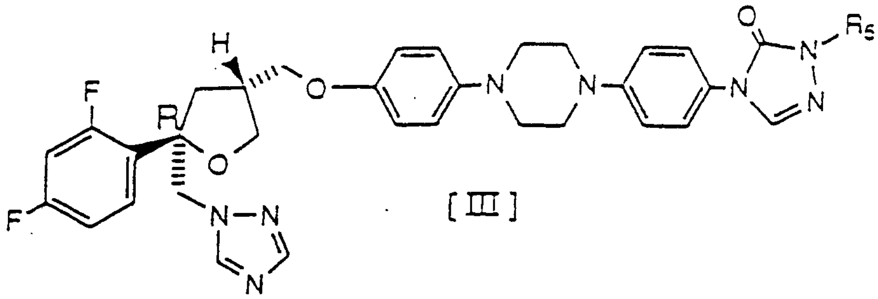

[III]

worin $R_5$

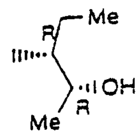 , 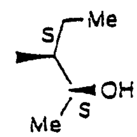 , 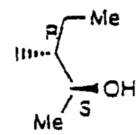 ,

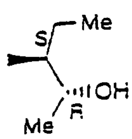 , 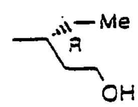 , 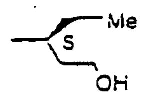 ,

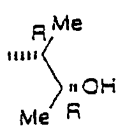 , 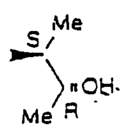 , 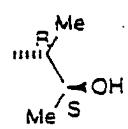 ,

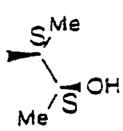 , 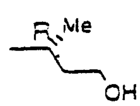 , 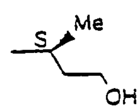 ,

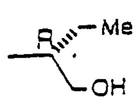 oder 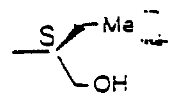

ist.

**4.** Verbindung der Formel IV

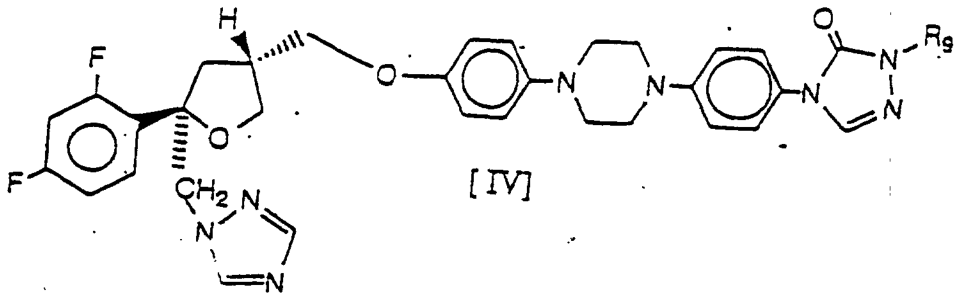

[IV]

worin $R_9$ $-\overset{*}{C}H(C_2H_5)\overset{*}{C}H(OH)CH_3$

oder $-\overset{*}{C}H(CH_3)\overset{*}{C}H(OH)CH_3$.

oder ist.

**5.** Verbindung gemäß Anspruch 3, worin $R_5$

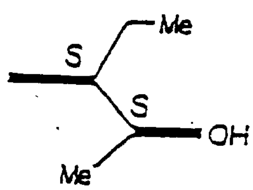

oder

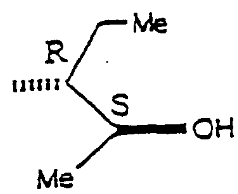

oder ist.

**6.** Verbindung der Formel

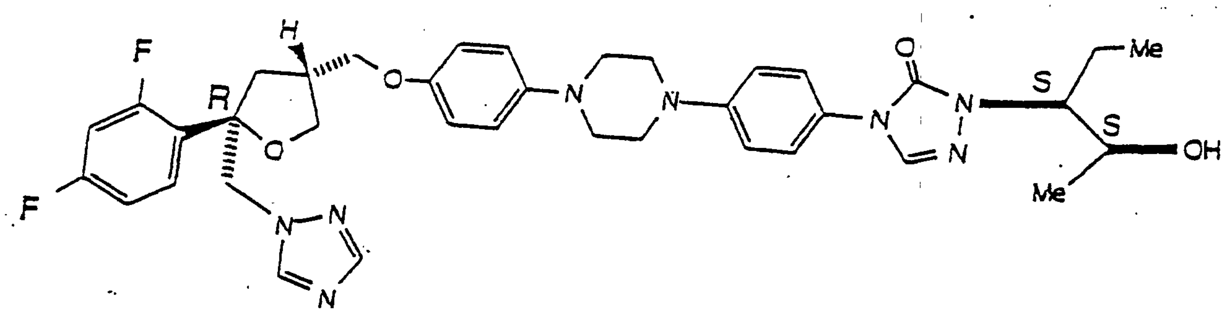

**7.** Pharmazeutisch verträglicher Ester einer Verbindung gemäß irgendeinem der vorstehenden Ansprüche.

**8.** Pharmazeutisch verträglicher Ester einer Verbindung gemäß irgendeinem der Ansprüche 1 bis 6, worin der Wasserstoff der Hydroxy-Komponente durch eine Gruppe der Formel

$$-\overset{\overset{O}{\|}}{C}-(CHR_7)_s-O-(CHR_7CHR_7)_t-R_8$$

ersetzt wurde, worin $R_7$ H oder eine geradkettige oder verzweigte ($C_1$-$C_6$) Alkylgruppe ist; und s eine ganze Zahl von 1 bis 6 ist; t eine ganze Zahl von 1 bis 6 ist; $R^8$ $R_7$ oder-$(CHR_7)_s$-$CO_2R_7$ ist.

**9.** Pharmazeutisch verträglicher Ester einer Verbindung gemäß irgendeinem der Ansprüche 1 bis 6, worin der Wasserstoff der Hydroxy-Komponente durch eine Gruppe der Formel

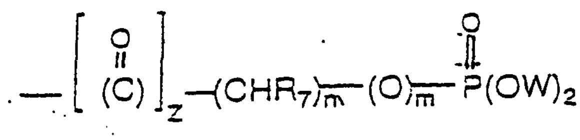

ersetzt wurde, worin z 0 oder 1 ist; m 0 oder 1 ist; $R_7$ H oder eine geradkettige oder verzweigte ($C_1$-$C_6$) Alkylgruppe ist, jedoch vorzugsweise H ist; n eine ganze Zahl von 0 bis 6 ist und W H, $CH_2Ar$ oder

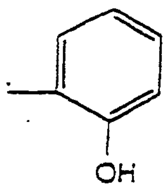

ist, und worin Ar Phenyl oder mit Halogen-, Cyano-, Nitro-oder Trihalogenmethyl-substituiertes Phenyl ist.

**10.** Pharmazeutisch verträglicher Ester einer Verbindung gemäß irgendeinem der vorstehenden Ansprüche, in dem der Wasserstoff der einen Hydroxy-Komponente durch $SO(OH)_2$ ersetzt wurde.

**11.** Pharmazeutisch verträglicher Ester einer Verbindung gemäß irgendeinem der Ansprüche 1 bis 6, in dem der Wasserstoff der Hydroxy-Komponente durch eine Gruppe der Formel

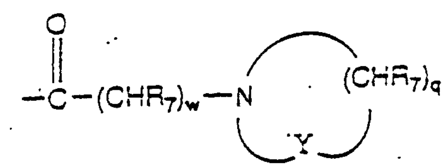

ersetzt wurde, worin $R_7$ wie oben definiert ist, W eine ganze Zahl von 1 bis 5 ist, W vorzugsweise von 1 bis 3 ist; q 3 oder 4 ist und Y $CHR_7$, -O-, NH, $NR_7$, S, SO oder $SO_2$ ist.

**12.** Pharmazeutisch verträglicher Ester einer Verbiindung gemäß irgendeinem der Ansprüche 1 bis 6, in dem der Wasserstoff der Hydroxy-Komponente durch eine geradkettige oder verzweigte Alkanoyl-Kette oder durch eine Alkanoyl-Gruppe ersetzt wurde, welche gegebenenfalls durch eine Hydroxy- oder Ether-Komponente substituiert wurde.

**13.** Pharmazeutisch verträglicher Ester einer Verbindung gemäß irgendeinem der Ansprüche 1 bis 6, in dem die Hydroxy-Komponente mit einem natürlichen Aminosäurerest verestert wurde, wobei die Aminogruppen gegebenenfalls durch übliche Schutzgruppen geschützt wurden.

**14.** Pharmazeutisch verträglicher Ester einer Verbindung gemäß irgendeinem der Ansprüche 1 bis 6, in dem das Wasserstoff der Hydroxy-Komponente durch eine Gruppe der Formel

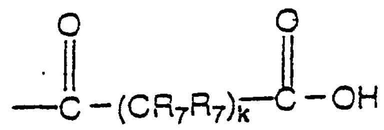

ersetzt wurde, worin $R_7$ wie oben definiert ist, und k eine ganze Zahl von 1 bis 8 ist.

**15.** Pharmazeutisch veträglicher Ester einer Verbindung gemäß irgendeinem der Ansprüche 1 bis 6, in dem das Wasserstoff der Hydroxy-Komponente durch eine Gruppe der Formel

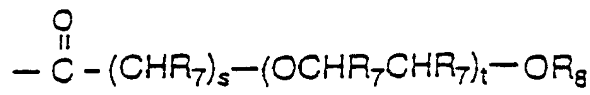

ersetzt wurde, worin $R_7$ H oder eine geradkettige oder verzweigte ($C_1$-$C_6$) Alkylgruppe ist; $R_8$ $R_7$ oder

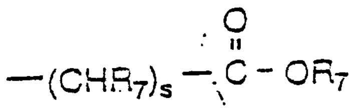

ist und s 1 bis 6 und t 1 bis 6 ist.

**16.** Pharmazeutisch verträglicher Ester einer Verbindung gemäß irgendeinem der Ansprüche 1 bis 6, in dem der Wasserstoff der Hydroxy-Komponente durch eine Gruppe der Formel

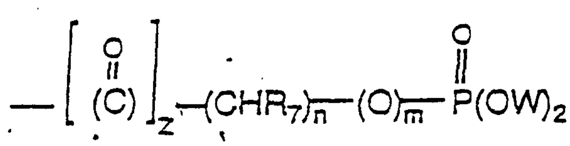

ersetzt wurde, worin z 0 oder 1 ist, m 0 oder 1 ist, n 0 bis 6 ist und $R_7$ H oder eine geradkettige oder verzweigte ($C_1$-$C_6$) Alkylgruppe ist und W H oder $CH_2Ar$ oder

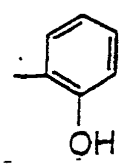

ist, worin Ar Phenyl oder Halogen-, Cyano- Nitro-, oder Trihalogenmethyl-substituiertes Phenyl ist.

**17.** Pharmazeutisch verträglicher Ester einer Verbindung gemäß Anspruch 5 oder 6, in dem der Wasserstoff der Hydroxy-Komponente durch

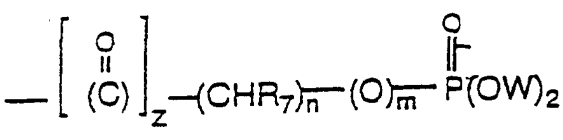

ersetzt wurde, worin z 0 oder 1 ist; $R_7$ H ist, m 0 oder 1 ist, n eine ganze Zahl von 0 bis 6 ist und W H ist.

**18.** Pharmazeutisch verträglicher Ester einer Verbindung gemäß Anspruch 5 oder 6, in dem der Wasserstoff der Hydroxy-Komponente durch

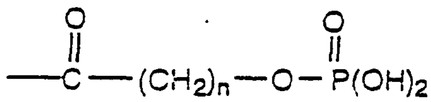

ersetzt wurde.

**19.** Verbindung der Formel

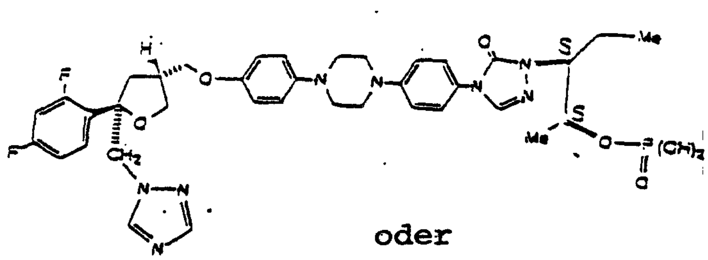

oder

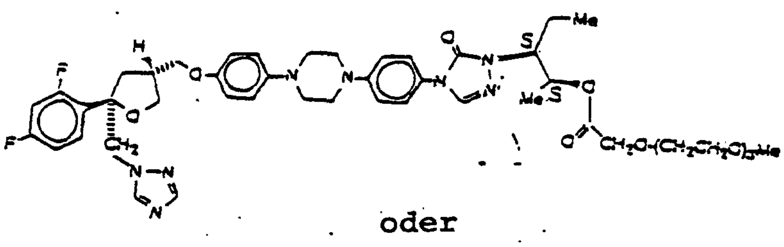

oder

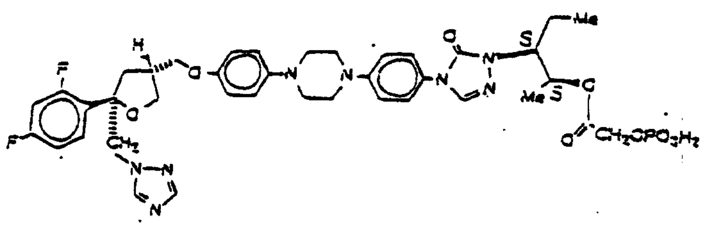

**20.** Pharmazeutisch verträgliches Salz einer Verbindung gemäß irgendeinem der vorstehenden Ansprüche.

**21.** Pharmazeutische Zusammensetzung zur Behandlung oder Vermeidung einer Pilz-Infektion, umfassend eine ausreichend Pilzbefall-verhütende Menge einer Verbindung gemäß irgendeinem der vorstehenden Ansprüche und einen pharmazeutisch verträglichen Träger.

**22.** Pharmazeutische Zusammensetzung gemäß Anspruch 21, bei der die Verabreichungsform oral oder parenteral ist.

**23.** Verfahren zur Herstellung von Verbindungen der Formel III des Anspruchs 3, worin $R_4$

$$\overset{*}{C}H(C_2H_5)\overset{*}{C}H(OH)CH_3$$

ist, worin die absolute stereochemische Konfiguration jedes mit einem Sternchen (*) markierten Kohlenstoffs dieselbe ist, d.h. S,S oder R,R, im wesentlichen frei von S,R oder R,S, und bei dem man S oder R-Milchsäureester

in das entsprechende Amid überführt, welches selektiv zu dem entsprechenden Aldehyd reduziert wird und anschließend in das entsprechende N-Formylaminopropanimin überführt wird, was die Reaktion des N-Formylaminopropanimin mit einer Verbindung der Formel

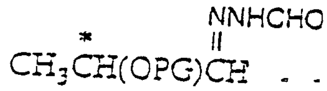

und Ethylmagnesiumbromid unter ausreichenden Grignard-Reaktionsbedingungen umfaßt, um eine Verbindung der Formel

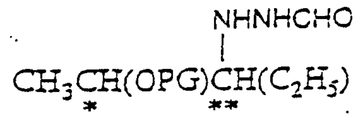

zu erzeugen, worin die absolute Stereochemie, die bei dem mit einem doppeltem Sternchen (**) markierten Kohlenstoff im wesentlichen der des mit einem einzelnen Sternchen markierten Kohlenstoff entspricht, und worin PG eine übliche Hydroxy-Schutzgruppe, wie beispielsweise Benzyl, ist.

**24.** Verfahren gemäß Anspruch 23, bei dem man S-Milchsäuremethylester als Ausgangsmaterial verwendet und die Grignard-Reaktion in Gegenwart von mehr als einem Äquivalent an bis(Trimethylsilyl)azetamid durchführt.

**Revendications**

**1.** Composé représenté par la formule I

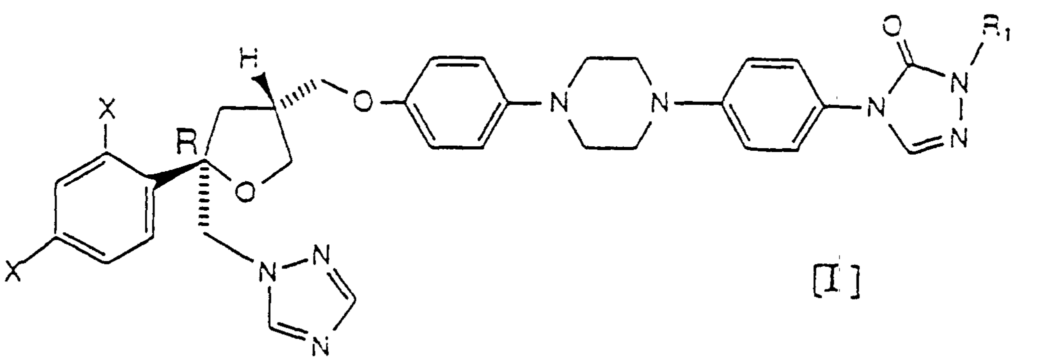

[I]

où X est indépendamment tous deux F ou tous deux Cl ou bien un X est indépendamment F et l'autre est indépendamment Cl;

$R_1$ est un groupe alkyle ($C_4$ à $C_5$) à chaîne droite ou ramifiée substitué par une fraction hydroxy.

**2.** Composé de la revendication 1 où $R_1$ est un groupe alkyle $C_4$- ou $C_5$ hydroxy substitué, sélectionné parmi:

$-\overset{*}{C}H(C_2H_5)\overset{*}{C}H(OH)CH_3$, $-\overset{*}{C}H(C_2H_5)CH_2CH_2OH$,
$-(CH_2)_2\overset{*}{C}H(OH)C_2H_5$, $-\overset{*}{C}H(CH_3)\overset{*}{C}H(OH)CH_3$,
$-\overset{*}{C}H(C_2H_5)CH_2OH$, $-\overset{*}{C}H(CH_3)CH_2CH_2OH$

où les carbones avec l'astérisque (*) ont la configuration absolue R ou S.

**3.** Composé représenté par la formule III

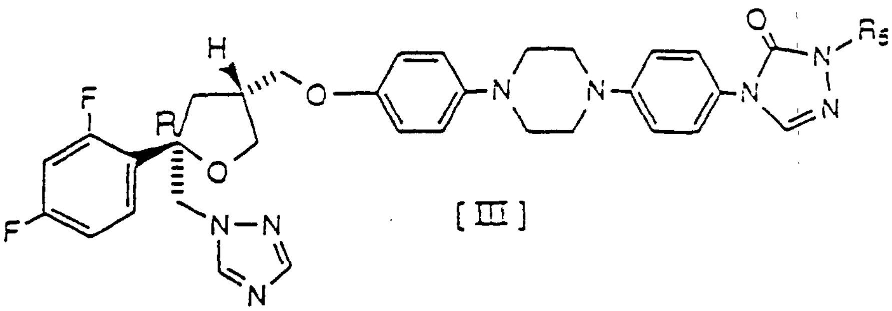

[III]

où $R_5$ est

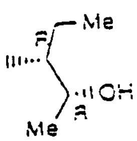 , 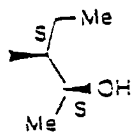 , 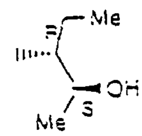 ,

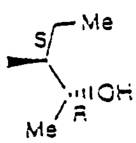 , 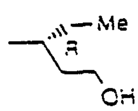 , 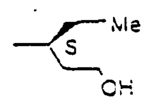 ,

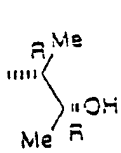 , 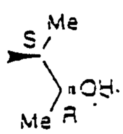 , 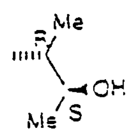 ,

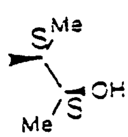 , 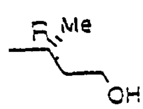 , 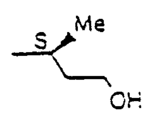 ,

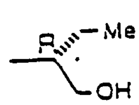 ou 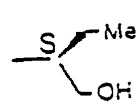

**4.** Composé représenté par la formule IV

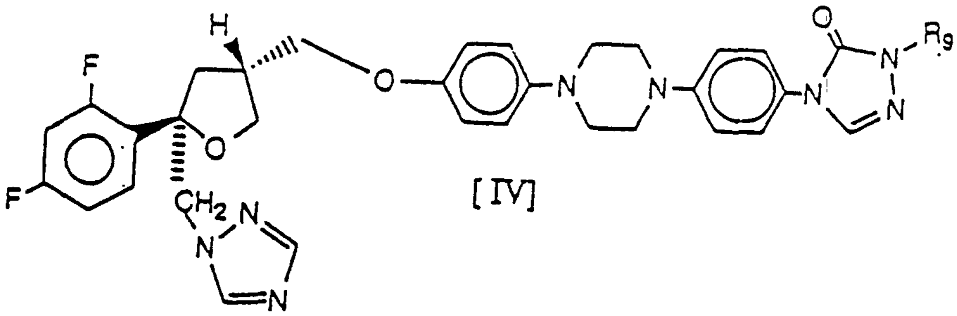

[IV]

où $R_9$ =

$$-\overset{*}{C}H(C_2H_5)\overset{*}{C}H(OH)CH_3 \text{ ou } -\overset{*}{C}H(CH_3)\overset{*}{C}H(OH)CH_3.$$

**5.** Composé de la revendication 3 où $R_5$ est

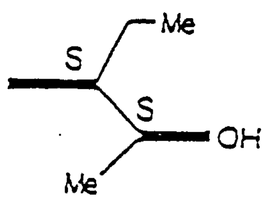

ou

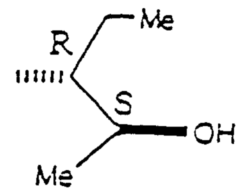

ou

**6.** Composé de la formule

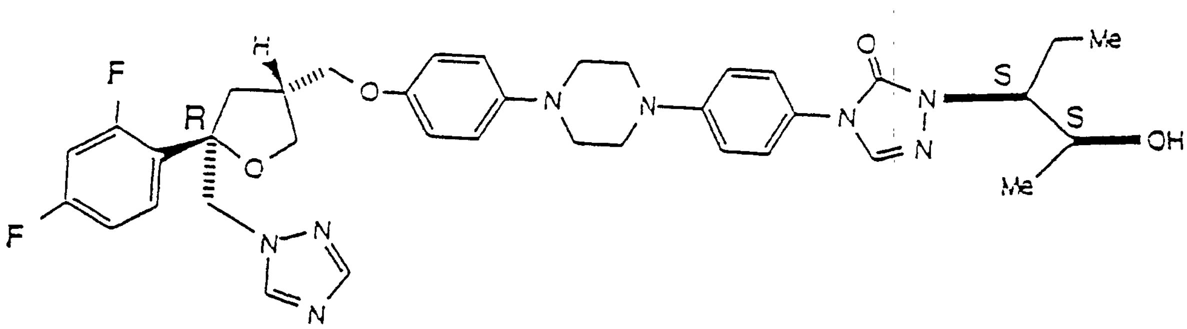

**7.** Ester pharmaceutiquement acceptable d'un composé selon l'une quelconque des revendications précédentes.

**8.** Ester pharmaceutiquement acceptable d'un composé selon l'une quelconque des revendications 1 à 6 où l'hydrogène de ladite fraction hydroxy est remplacé par un groupe de la formule

$$-\overset{O}{\overset{\|}{C}}-(CHR_7)_s-O-(CHR_7CHR_7)_t-R_8$$

où $R_7$ est H ou un groupe alkyle ($C_1$-$C_6$) à chaîne droite ou ramifiée; et s est un entier de 1 à 6; t est un entier de 1 à 6; $R^8$ et $R^7$ ou —$(CHR_7)_S$-$CO_2R_7$.

**9.** Ester pharmaceutiquement acceptable d'un composé selon l'une quelconque des revendications 1 à 6 où l'hydrogène de ladite fraction hydroxy est remplacé par un groupe de la formule

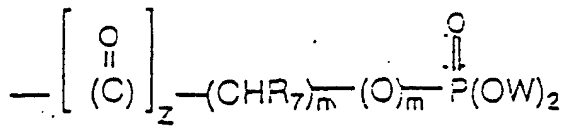

où z est O ou 1; m est 0 ou 1; $R_7$ est H ou un groupe alkyle ($C_1$-$C_6$) à chaîne droite ou ramifiée et de préférence est H; n est un entier de 0 à 6 et W est H, $CH_2$Ar ou

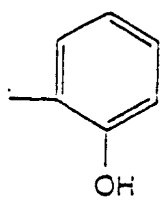

et où Ar est phényle ou phényle substitué par halo, cyano, nitro ou trihalométhyle.

**10.** Ester pharmaceutiquement acceptable d'un composé selon l'une quelconque des revendications précédentes où l'hydrogène de ladite fraction hydroxy est remplacé par $SO(OH)_2$.

**11.** Ester pharmaceutiquement acceptable d'un composé selon l'une quelconque des revendications 1 à 6 où l'hydrogène de ladite fraction hydroxy est remplacé par un groupe de la formule

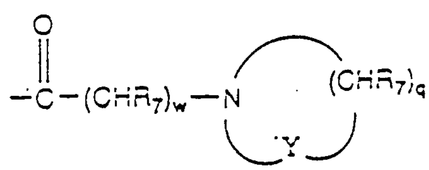

où $R_7$ est tel que défini ci-dessus, W est un entier de 1 à 5, de préférence W est 1 à 3; q est = 3 ou 4 et Y est $CHR_7$, -O-, NH, $NR_7$, S, SO ou $SO_2$.

**12.** Ester pharmaceutiquement acceptable d'un composé selon l'une quelconque des revendications 1 à 6 où l'hydrogène de ladite fraction hydroxy est remplacé par un groupe alcanoyle à chaîne droite ou ramifiée ou alcanoyle qui est facultativement substitué par une fraction hydroxy ou éther.

**13.** Ester pharmaceutiquement acceptable d'un composé selon l'une quelconque des revendications 1 à 6 où ledit groupe hydroxy est estérifié par un résidu d'acide aminé naturel, où les groupes aminés sont facultativement protégés par des groupes protecteurs conventionnels.

**14.** Ester pharmaceutiquement acceptable d'un composé selon l'une quelconque des revendications 1 à 6 où l'hydrogène de ladite fraction hydroxy est remplacé par un groupe de la formule

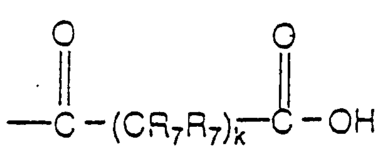

où $R_7$ est tel que défini ici et k est un entier de 1 à 8.

**15.** Ester pharmaceutiquement acceptable d'un composé selon l'une quelconque des revendications 1 à 6 où l'hydrogène de ladite fraction hydroxy est remplacé par un groupe de la formule

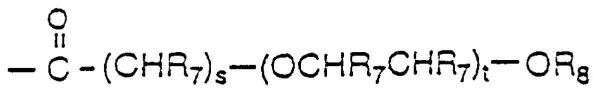

où $R_7$ est H ou un groupe alkyle ($C_1$-$C_6$) à chaîne droite ou ramifiée, $R_8$ est $R_7$ ou

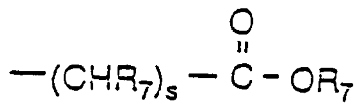

et s est 1 à 6 et t est 1 à 6.

**16.** Ester pharmaceutiquement acceptable d'un composé selon l'une quelconque des revendications 1 à 6 où l'hydrogène de ladite fraction hydroxy est remplacé par un groupe de la formule

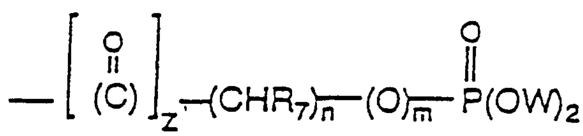

où z est 0 ou 1, m est 0 ou 1, n est 0 à 6, et $R_7$ est H ou un groupe alkyle ($C_1$-$C_6$) à chaîne droite ou ramifiée et W est H ou $CH_2Ar$ ou

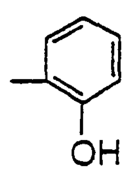

où Ar est phényle ou phényle substitué par halo, cyano, nitro ou trihalométhyle.

**17.** Ester pharmaceutiquement acceptable d'un composé selon la revendication 5 ou la revendication 6 où l'hydrogène de la fraction hydroxy est remplacé par

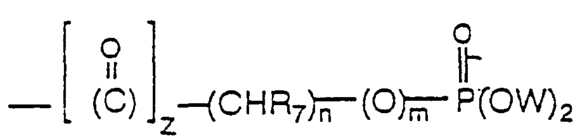

où z est 0 ou 1; $R_7$ est H; m est 0 ou 1, n est un entier de 0 à 6 et W est H.

**18.** Ester pharmaceutiquement acceptable d'un composé selon la revendication 5 ou 6 où l'hydrogène de la fraction hydroxy est remplacé par

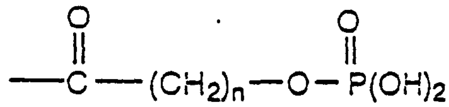

**19.** Composé de la formule

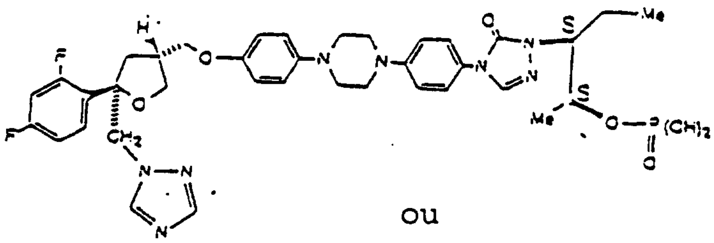

ou

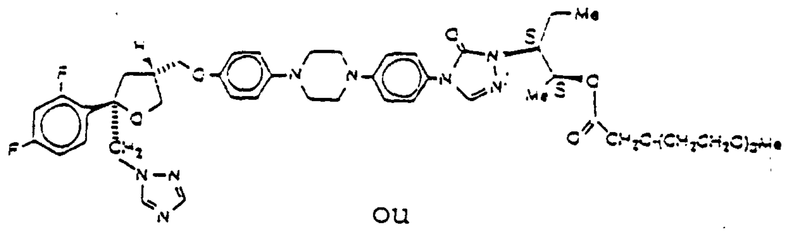

ou

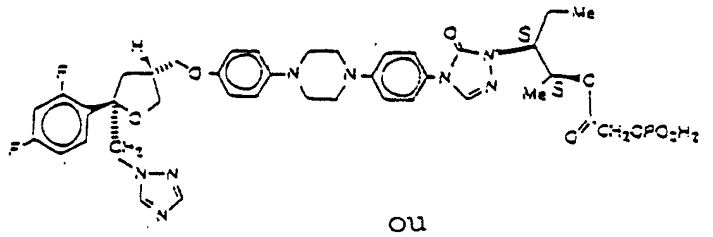

ou

**20.** Sel pharmaceutiquement acceptable d'un composé selon l'une quelconque des revendications précédentes.

**21.** Composition pharmaceutique pour le traitement ou la prévention d'une infection fongique comprenant une quantité fongicidement efficace d'un composé selon toute revendication précédente avec un support pharmaceutiquement acceptable pour celui-ci.

**22.** Composition pharmaceutique de la revendication 21 où le mode d'administration est par voie orale ou parentérale.

**23.** Méthode de production des composés de la formule III de la revendication 3 où $R_4$ est

$$\overset{*}{C}H(C_2H_5)\overset{*}{C}H(OH)CH_3$$

où la stéréochimie absolue à chaque carbone avec astérisque (*) est la même, i.e., S,S ou R,R sensiblement sans S,R ou R,S et où l'ester de l'acide S ou R lactique est converti en amide correspondant, qui est sélectivement réduit en aldéhyde correspondant puis est converti en N-formyl amino propanimine correspondante qui comprend la réaction de la N-formyl amino propanimine de la formule

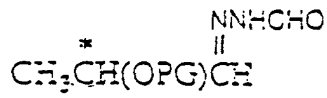

avec du bromure d'éthyl magnésium en conditions de réaction de Grignard suffisantes pour produire un composé de la formule

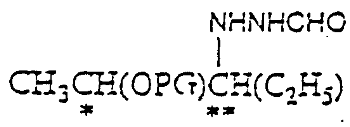

où la stéréochimie absolue induite au carbone du double astérisque (**) est sensiblement la même que celle au carbone du simple astérisque et où PG est un groupe hydroxy protecteur conventionnel tel que benzyle.

**24.** Méthode de la revendication 23 où l'ester méthylique de l'acide S-lactique est utilisé en tant que matière première et la réaction de Grignard est entreprise en présence de plus d'un équivalent de bis(triméthylsilyl)acétamide.